(19) Europäisches Patentamt
European Patent Office
Office européen des brevets

(11) EP 2 289 542 A1

(12) # EUROPEAN PATENT APPLICATION

(43) Date of publication:
**02.03.2011 Bulletin 2011/09**

(51) Int Cl.:
***A61K 38/49*** *(2006.01)* ***A61K 39/395*** *(2006.01)*
***C07K 14/705*** *(2006.01)* ***A61P 25/00*** *(2006.01)*

(21) Application number: **09011149.3**

(22) Date of filing: **31.08.2009**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(71) Applicants:
- **PAION Deutschland GmbH**
  **52062 Aachen (DE)**
- **Inserm**
  **75013 Paris (FR)**

(72) Inventors:
- **Petersen, Karl-Uwe**
  **52072 Aachen (DE)**
- **Vivien, Denis**
  **Boulevard Henri Becquerel**
  **14073 CAEN (FR)**
- **Ali, Carine**
  **Boulevard Henri Becquerel**
  **14073 CAEN (FR)**
- **Macrez, Richard**
  **Boulevard Henri Becquerel**
  **14073 CAEN (FR)**

(74) Representative: **Von Renesse, Dorothea et al**
**König Szynka Tilmann von Renesse**
**Patentanwälte Partnerschaft**
**Lohengrinstrasse 11**
**40549 Düsseldorf (DE)**

(54) **Treatment of neurological or neurodegenerative disorders**

(57) The Invention relates to a protein or peptide consisting of a kringle portein or peptide and its use for the treatment of neurological or neurodegenerative disorders, in particular stroke. The invention relates also to an isolated antibody of fragment thereof which binds to the N-terminal domain of the NMDA receptor subunit NR1 (anti-NR1 antibody), whereas binding of the antibody or the fragment thereof prevents the cleavage of the extracellular domain of the NR1 subunit, or the fragment and its use for the treatment of neurological or neurodegenerative disorders, in particular stroke. The invention relates further to a pharmaceutical composition containing said kringle protein or peptide or anti-NR1 antibody.

EP 2 289 542 A1

## Description

This invention relates to a method for the treatment of neurological or neurodegenerative disorders. More particularly, this invention relates to the protection of neurons and/or the blood brain barrier which are adversely affected as result of a neurological or neurodegenerative disorder.

Stroke is a leading cause of adult death and disability with approximately 6 million deaths per year, with an alarming projected incidence over the next decade due to the increase In the elderly population. Despite the significant advances that have been made in understanding the cellular and molecular pathophysiology of cerebral ischaemia, recombinant tissue-type plasminogen activator (rt-PA) remains the only approved acute treatment for ischaemic stroke. Hundreds of compounds have been tested so far in clinical trials for ischaemic stroke, but aside from rt-PA, none has proven effective.

The use of rt-PA is limited by a short therapeutic window (4.5 hours post-onset) and by promotion of both intracerebral haemorrhage and neurotoxicity. Thus, there is a critical need for safer and more effective treatments, in order to improve the global benefit of rt-PA induced thrombolysis and to provide treatment for stroke patients who are not eligible for thrombolysis (*i.e.* more than 80% of stroke patients).

T-PA is a serine protease with two faces which displays key roles in the intravascular space, at the interface between blood and brain and in the brain parenchyma (for review: Yepes et al., 2008). In the intravascular compartment, t-PA's main substrate is the inactive zymogen plasminogen and its main role is to promote fibrinolysis. Blood-derived t-PA can cross both the intact and the injured blood-brain barrier (BBB) (Benchenane et al. 2005a, Benchenane et al., 2005b) and thus can, together with endogenously produced t-PA, interact in the brain parenchyma with a variety of substrates, thus extending its functions above t-PAlplasmin(ogen)-driven extracellular matrix degradation.

There is a growing body of evidence indicating that the interaction between t-PA and the N-methyl-D-aspartate receptor (NMDAR), the low density lipoprotein receptor-related protein (LRP), annexin-II in glial cells and/or neurons activate cell signaling processes results in a deleterious outcome including cerebral edema, hemorrhagic transformation and cell death. Based on these multiple pathophysiological effects of t-PA, the participation of endogenous t-PA (supported by the side effects of exogenously applied rt-PA) is discussed beyond the established role in ischaemic disorders for several neurological or neurodegenerative disorders such as epilepsy, Alzheimer's disease, multiple sclerosis or meningitis.

According to the complex behaviour of t-PA several molecular strategies for inhibiting the deleterious effects of t-PA can be envisaged. However a strategy that can be transferred into the clinical situation is still missing.

Thus, it is the objective of the present invention to provide novel means for the treatment of neurological or neurodegenerative disorders, in particular of stroke.

This objective is solved by a method according to claim 1. Further embodiments of the invention are subject matter of additional independent or dependent claims.

The inventors have found that proteins comprising or consisting of a kringle domain or antibodies specifically binding to the amino-terminal domain of the NR1 subunit of the NMDA receptor (anti-ATD-NR1 antibody) are active at two t-PA relevant target sites: the blood brain barrier and the NMDA receptor and as a result exhibit vasoprotective and neuroprotective activity.

Accordingly the antl-ATD-NR1 antibody (or an antigen binding fragment thereof) and the kringle domain-containing proteins or peptides (hereinafter "kringle proteins" and/or "kringle peptide") are together referred to as "t-PA inhibitor".

As will be shown below the results from the animal model reveal that the t-PA inhibitors of the invention are not only effective when given together with t-PA but also when administered alone. Furthermore the t-PA inhibitors exhibit beneficial effect in thrombosis disorders when given after clot formation, hence enable an acute or post-acute treatment of thrombosis.

Thus, the invention relates to the use of a protein or peptide for the manufacture of a medicament for the treatment of neurological or neurodegenerative disorders, in particular stroke, wherein the protein or peptide is selected form the group consisting of:

(a) a kringle protein or peptide comprising or consisting of

(i) an amino acid sequence according SEQ ID NO:1, SEQ ID NO:2 or SEQ ID NO:3; or

(ii) an amino acid sequence with at least 70% identity, preferably 80%, more preferably 90% and most preferably 95% identity to the amino acid sequence given in SEQ ID NO:3; or

(iii) an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES-$X_3$-C-$X_2$-WNS-

$X_2$-L-$X_4$-Y-$X_4$-PXA-$X_2$-LGLGXHNYCRNP-$X_4$-KPWCXVXK-$X_6$-EXC-$X_2$-PXC", wherein X denotes an arbitrary amino acid; or

(iv) an amino acid sequence with at least 92% identity, preferable at least 95% or 100% identity to the DARK motif defined by the sequence: "CY-$X_3$-G-$X_2$- YRGTXS-$X_2$-ESR-$X_2$C-$X_2$-WNS-$X_2$-LXR-$X_2$-Y-$X_3$-MPXAFN-LGLGXHNYCRNPNXAXKPWCXVXK-$X_3$-F-$X_2$-ESC-$X_2$-PXC", wherein X denotes an arbitrary amino acid;

wherein said protein or peptide does not exhibit a serine protease activity;
or

(b) an isolated antibody or fragment thereof which binds to the N-terminal domain of the NMDA receptor subunit NR1 (anti-ATD-NR1 antibody), whereas the binding of the antibody or the fragment thereof prevents the cleavage of the extracellular domain of the NR1 subunit, or the fragment, whereas the NR1 preferably

(i) has the amino acid sequence SEQ ID NO: 4 or 5,
(ii) is encoded by the nucleotide sequences SEQ ID NO: 6 or 7
(iii) nucleic acid molecule that specifically hybridizes to the complement of the nucleic acid molecule of SEQ ID NO: 6 or 7 under conditions of high stringency, where the hybridisation is performed in 5x SSPE, 5 x Denhardt's solution and 0.5% SDS overnight at 55 to 60°C.

The inventors could identify the kringle domain as the relevant domain for the inhibition of the t-PA transport through the BBB with the final proof that a protein consisting only of a plasminogen activator-related kringle (PARK) domain is also a t-PA transport inhibitor. Hence a protein or peptide comprising or consisting of this domain can be used as a vasoprotectant and/or neuroprotectant. This is not limited to a kringle domain of desmoteplase (Desmodus rotundus plasminogen activator) DSPA but applies also to the kringle domains of other plasminogen activators since it was found that kringle domains of different plasminogen activators were able to inhibit trans-BBB transport of rt-PA.

Hence the invention relates to the use of proteins with a plasminogen activator-related kringle (PARK) domain or a desmoteplase activator-related kringle (DARK) domain for the treatment of neurological disorders. In particular these proteins can be applied as vasoprotectants and/or neuroprotectants.

The protective activity of the kringle proteins is not due to its protease activity. Hence the proteins of the invention do not exhibit the serine protease activity commonly shared by plasminogen activators.

This could be shown by the inventors in an *in vitro* model of BBB penetration. Herein was demonstrated that the transport of rt-PA can be blocked not only by the plasminogen activator desmoteplase but also by an inactivated so called 'clogged' DSPA (cDSPA). This opened the way to a new therapy approach since an inactivated DSPA will neither augment the plasminogen activating capacity of t-PA nor interfere with the plasminogen-activating and therefore clot-lysing efficacy of t-PA. Rather, it inhibit only the detrimental side effects of t-PA related thrombolytics. Furthermore, the inactivated DSPA is not capable of cleaving the NR1 subunit of the NMDA receptor and hence poses no risk of neurotoxicity on its own.

Furthermore the inventors could show that the co-incubation of rt-PA and clogged DSPA (or the isolated and purified kringle domain) had no toxic effect on the blood brain barrier.

A kringle is a triple looped polypeptide structure formed by three disulfide bonds. Kringles vary in length from about 79 to 82 amino acid groups. A high degree of sequence homology is shared among the single kringle of human urokinase (Günzler et al., Hoppe-Seyler's Z. Physiol. Chem. 363, 1155, 1982), the two kringles of human tissue plasminogen activator (Pennica, et a/. Nature, 301,214,1983*),* the two kringles of human prothrombin (Walz et al., Proc. Nat'l. Acad. Sci. USA, 74, 1969, 1977), and the five kringles of human plasminogen (Sottrup-Jensen et a/., in Progress in Chemical Fibrinolysis and Thrombolysis (eds. Davidson et al), 3, 191, 1978). The relative positions of the six cysteines involved in the intra-kringle disulfide bridges are conserved in all kringles.

The term, "plasminogen activator related kringle domain" (PARK domain) as used in this application refers to proteins or peptides with an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES-$X_3$-C-$X_2$-WNS-$X_2$-L-$X_4$-Y-$X_4$-PXA-$X_2$-LGLGXHNYCRNP-$X_4$-KPWCXVXK-$X_6$-EXC-$X_2$-PXC", wherein X denotes an arbitrary amino acid (Fig. 1B).

This term in particular encompasses proteins comprising or consisting of the amino acid sequences SEQ ID NO:1, SEQ ID NO:2 and SEQ ID NO:3. It is also understood that polymorphic forms in the kringle region of these proteins may exist in nature where one or more amino acids may be added, deleted or substituted. Similar changes in a protein may also be brought about *in vitro* with the advent of the present day technology of point mutation of the gene or by chemical synthesis of the gene with any desired sequence. These modified structure(s) are therefore also included in the term, kringle(s), used in this application.

Sequences SEQ ID NO:1 and SEQ ID NO:2 show the amino acid sequences of the kringle 1 and 2 domains of the recombinant t-PA (alteplase). SEQ ID NO:3 shows the amino acid sequence of the kringle domain of desmoteplase (DSPA alpha 1).

>Seq ID No.1: t-PA_kringle 1
CYEDQGISYRGTWSTAESGAECTNWNSSALAQKPYSGRRPDAIRLGLGNHNYCRNP DRDSKPWCYVFKAGKYSSEFCSTPAC

>Seq ID No. 2 t-PA_kringie 2
CYFGNGSAYRGTHSLTESGASCLPWNSMILIGKVYTAQNPSAQALGLGKHNYCRNPD GDAKPWCHVLKNRRLTWEYCDVPSC

>Seq ID No.3: DSPA_kringle
CYEGQGVTYRGTWSTAESRVECINWNSSLLTRRTYNGRMPDAFNLGLGNHNYCRN PNGAPKPWCYVIKAGKFTSESCSVPVC

Other preferred kringle domains for said peptide or protein include a kringle domain from urokinase and prothrombin.

As used herein the" DARK domain" is defined as a peptide with an amino acid sequence of at least 90 % identity and preferable at least 95% or 100% identity to the following amino acid sequence motive resulting from the kringle sequence of DSPA alpha 1 as given in Figure 1B:

CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNX-AXKPWCXVXK-$X_3$-F-$X_2$-ESC-$X_2$-PXC, wherein X denotes an arbitrary amino acid

In a further aspect of the invention the kringle protein consists of an amino acid sequence of either the DARK or the PARK domain as of Fig. 1B or Fig. 1A, respectively:

CY-$X_3$-G-$X_2$-YRGTXS-$X_2$-ES-$X_3$-C-$X_2$-WNS-$X_2$-L-$X_4$-Y-$X_4$-PXA-$X_2$-LGLGXHNYCRNP-$X_4$-KP-WCHXVXK-$X_6$-EXC-$X_2$-PXC

or

CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNX-AXKPWCXVXK-$X_3$-F-$X_2$-ESC-$X_2$-PXC.

The kringle proteins of the invention preferably have a length of not more than 200 amino acids (aa), preferably not more than 150 aa, most preferably not more than 100 aa, and comprise the PARK or DARK motif as above. This has a length of 82 aa.

The kringle proteins furthermore can include a lysine binding site, which is preferably defined by the presence of the amino acids Y36, W62 and H64, according to the numbering of the 82 aa kringle domain as used herein.

The kringle proteins of the invention preferably inhibit the t-PA transport across the blood brain barrier (BBB) in mammals by at least 20%, preferably by at least 30%, most preferred by at least 40% or 50% assessable by the method described in Example A, Chapter 2 of the present invention.

Said t-PA inhibitor can bind specifically to the low-density lipoprotein (LDL) receptor-related protein (LRP). The LRP is a multifunctional endocytosis receptor that binds a variety of ligands, including t-PA. the β-amyloid precursor protein, alpha 2-macroglobulin, apolipoprotein E-enriched beta-very-low-density lipoprotein, and Pseudomonas exotoxin A, some of which are implicated in neurological diseases such as Alzheimer's disease. Due to LRP binding said protein or peptide can block the binding of t-PA or other ligands to the LRP thereby inhibiting the LRP-mediated transport over the BBB.

Furthermore, the t-PA inihibtor of the invention can bind to the NR1 subunit of the NMDA receptor. Due to this NR1 binding said t-PA inhibitor can block the binding of other substrates or ligands to the NR1 subunit thereby inhibiting its proteolytical cleavage.

In a further aspect the invention relates to the use of an isolated antibody or an antigen-binding portion thereof for the treatment of neurological disorders, in particular of stroke, which specifically binds to the N-terminal domain of the NMDA receptor subunit NR1 (anti-ATO-NR1 antibody) or a fragment thereof, in particular to an antigen comprising or consisting of SEQ ID NO:4 or 5.

In a further embodiment the antibody of the invention or a fragment thereof specifically hybridizes to the complement of the nucleic acid molecule of SEQ ID NO 6 or 7 under conditions of high stringency. This high stringency conditions can be provided by e.g. a hybridisation that is performed in 5x SSPE, 5 x Denhardt's solution and 0.5% SDS overnight at 55 to 60°C.

The inventors have now found that an antibody-based therapy targeting the interaction of t-PA with the amino-terminal domain of the NR1 subunit of the NMDA receptor can strongly and durably improve neurological outcome after stroke, by limiting brain damage and inhibiting the disruption of BBB.

This beneficial efficacy of the anti-ATD NR1 antibody effect was shown in a clinically relevant model of stroke that has the ability to reproduce the observed time window for t-PA treatment in humans (Orset et al., 2007). In this model, the in situ microinjection of purified murine thrombin triggers a local clot in the middle cerebral artery resulting in reproducible clot formation and cortical brain injury and lack of surgery-associated lethality. In this thrombosis model the efficacy of t-PA as well as the time window for t-PA treatment is similar to the human clinical situation. In sum this animal model is well suited for the preclinical evaluation of stroke therapies.

The anti-ATD NR1 antibody is directed only against a small part of one of the NMDA subunits. This allows a specific intervention at the NMDA receptor that does not interfere with the physiological function of the NMDA receptor but only inhibits the t-PA-induced potentiation of the NMDA activity.

Due to the fact that the antibody is not directed against t-PA, therapeutically administered t-PA or other plasminogen activators will retain their normal thrombolytic function that is mandatory for a beneficial activity in thrombotic disorders.

The anti-ATD NR1 antibody can be used for passive immunization of patients that are in risk of a thrombotic disorder or suffer from a thrombotic disorder. In contrast to the active immunization which is achieved by pretreatment with a suitable antigen, the blocking effect at the NMDA receptor is immediately achieved. This is of high importance, since in thrombotic disorders a timely treatment is a hallmark for a successful outcome.

As t-PA and glutamate, the endogenous agonist at the NMDA receptor, exert critical functions in synaptic remodeling and plasticity underlying memory and learning processes, the risk of corresponding side effects is apparent. For the passive immunization as used herein, no alterations of cognitive functions including spatial memory, contextual and fear conditioning were evident. This supports a superior safety profile for this strategy of brain protection.

While therapeutic efficacy of the antibodies against detrimental effects of administration of t-PA would be a logical consequence of their design, they most surprisingly were also found to be effective without co-administration of t-PA: Mice treated with antibodies in the mouse model of thromboembolic stroke displayed strongly reduced brain damage (See Figure 12A; 43.3% of protection compared to control). It is remarkable that this protective effect is comparable to the protective effect of an early rt-PA treatment in this model.

As a result of these findings the anti-ATD NR1 antibody is also able to inhibit the endogenously expressed t-PA.

Hence according to the invention the anti-ATD NR1 antibody can be used as a monotherapy (i.e. without t-PA co-treatment or coadministration of any other drug substance) for the treatment of thrombotic disorders, particularly stroke.

The analysis of the blood brain barrier leakage in the stroke model revealed that both the early and the late administration of the anti-ATD NR1 antibodies alone significantly reduce the extent of BBB leakage induced by stroke. In addition the anti-ADT-NR1 treatment efficiently reduced the damaging effect of rt-PA on the integrity of the BBB in this stroke model. Furthermore the kringle domain containing protein prevents t-PA-induced damage of the BBB.

Hence in a preferred embodiment of the invention the t-PA inhibitor can be used for the treatment of neurological or neurodegenerative disorders that are associated with enhanced permeability of the blood brain barrier. These disorders comprise ischaemic or thrombotic disorders such as stroke or TIA, epilepsy such as temporal lobe epilepsy (TLE), amyotrophic lateral sclerosis, multiple sclerosis, brain tumours, Parkinson disease, Alzheimer's disease, brain oedema, or CNS complications resulting from parasitic, bacterial, fungal or viral infections such as meningitis or encephalitis.

Antibodies are commonly believed to be effectively excluded from the brain by an intact blood brain barrier and gain only access to brain tissue in case of BBB injury. As a surprising finding, immunostaining and quantification of fluorescent anti-ATD-NR1 antibodies in the brain parenchyma (see Figure 22) revealed that the antibodies are capable to reach the brain in non-operated and in sham animals; they do so at higher rates in ischemic animals with a clear increase in the damaged hemisphere. Thus, contrary to current beliefs, the invention shows that antibodies can be used in the absence of BBB damage. In the presence of such damage, when exogenous t-PA can cross the BBB at high rates, also the antibodies will reach the brain tissue at larger quantities to antagonize the t-PA at the NMDA receptor.

Hence according to the invention the anti-ATD NR1 antibody can be used also for disorders without an enhanced permeability of the BBB or as a preventive treatment that even before a pathophysiological enhanced permeability of the BBB therapeutically effective amounts of the antibody are present in the brain parenchyma to initially protect neuronals cells.

In the context of the invention the antibody is directed against the N-terminal region of the NR1-1a subunit encoding the amino acids 19 to 480, preferably encoded by the nucleotide sequence of SEQ ID NO:6 or 7 or encoding amino acids 19 to 371 as disclosed in SEQ ID NO:4 or 5 or a fragment thereof comprising of 8 to 30, preferably 10 to 20 contiguous amino acids wherein the antibody which is directed said fragment prevents the cleavage of the NR1 subunit by t-PA.

SEQ 10 NO:4 (rat NR1-1a, NP_058706.1, as 19 to 371)
RAACDPKIVNIGAVLSTRKHEQMFREAVNQANKRHGSWKIQLNATSVTHKPNAIQMAL
SVCEDLISSQVYAILVSHPPTPNDHFTPTPVSYTAGFYRIPVLGLTTRMSIYSDKSIHLS
FLRTVPPYSHQSSVWFEMMRVYNWNHIILLVSDDHEGRAAQKRLETLLEERESKAEK
VLQFDPGTKNVTALLMEARELEARVIILSASEDDAATVYRAAAMLNMTGSGYVWLVGE
REISGNALRYAPDGIIGLQLINGKNESAHISDAVGWAQAVHELLEKENITDPPRGCVG
NTNIWKTGPLFKRVLMSSKYADGVTGRVEFNEDGDRKFANYSIMNLQNRKLVQVGIY NGTH

SEQ ID NO:5 (human NR1-1, NP_000823.4. as 19 to 371)
RAACDPKIVNIGAVLSTRKHEQMFREAVNQANKRHGSWKIQLNATSVTHKPNAIQMAL
SVCEDLISSQVYAILVSHPPTPNDHFTPTPVSYTAGFYRIPVLGLTTRMSIYSDKSIHLS
FLRTVPPYSHQSSVWFEMMRVY**S**WNHIILLVSDDHEGRAAQKRLETLLEERESKAEK
VLQFDPGTKNVTALLMEARELEARVIILSASEDDAATVYRAAAMLNMTGSGYVWLVGE
REISGNALRYAPDGIIGLQLINGKNESAHISDAVGWAQAVHELLEKENITDPPRGCVG
NTNIWKTGPLFKRVLMSSKYADGVTGRVEFNEDGDRKFANYSIMNLQNRKLVQVGIY NGTH

SEQ ID NO:6 (rat NR1-1a, Nucleotide sequence to be inserted)

SEQ ID NO:7 (human NR1-1, Nucleotide sequence to be inserted)

According to the invention the antibody prevents cleavage of the extracellular domain of the NR1 subunit by a protease, preferably by t-PA and most preferably at the amino acid residue Arg260.

In one embodiment of the invention, the anti-ATD NR1 antibody is a monoclonal antibody, preferably a humanized antibody, a single domain antibody or $V_H$H antibody (so called nanobody), a chimeric antibody or a deimmunized antibody.

According to a further embodiment of the invention, an antigen-binding portion of the antibody can be used, preferably a scFv molecule or a Fab fragment. The antigen-binding portion can be humanized or possess the human sequence.

Further experiments in the mouse model of thrombo-embolic stroke revealed another surprising aspect of the therapeutic relevance of the anti-ATD NR1 antibody. Late t-PA thrombolysis, despite restoring cerebral blood flow comparably to early reperfusion, was associated with increased rather than reduced brain damage. However in mice treated with anti-ATD NR1 antibodies in addition, brain protection was recovered to a similar extent as observed in early thrombolysed animals.

Based on these findings the invention relates to a method for the treatment of neurological and neurodegenerative disorders, particularly stroke, whereby the patient is treated with a therapeutically effective amount of a thrombolytic drug and an effective amount of a t-PA inhibitor, in particular an anti-ATD NR1 antibody. The invention further relates to a composite comprising a thrombolytic drug and a t-PA inhibitor, particularly an antibody directed against the N-terminal domain of the NMDA receptor subunit NR1.

Accordingly, the anti-ATD NR1 antibody can be used as an adjunct to a thrombolytic therapy, in particular for late t-PA-induced thrombolysis.

The inventors could also demonstrate that a single intravenous injection of the anti-ATD NR1 antibody was highly protective even after clot formation. Early delivery (20 minutes post clot formation) of the anti-ATD NR1 antibody in the thromboembolic stroke model conferred a significant brain protection (44% of protection compared to control, see Figure 16A) which is comparable to the protective effect of an early rt-PA treatment in this model. Very surprisingly the delayed injection of anti-ATD NR1 antibodies (4 hours after clot formation) was not only able to inhibit the deleterious effect of co-administered t-PA but was also highly protective when given alone. This protective effect of a single late injection of the anti-ATD NR1 antibody as determined by MRI analysis was already visible 24 hours after ischaemia and maintained up to 15 days post-surgery (Fig. 18A vs. 18B) thereby conferring a long-term benefit after stroke.

Accordingly, in a further aspect of the invention the anti-ATD NR1 antibody can be used for the acute or post-acute treatment of a neurological or neurodegenerative disorder, in particularly stroke. This acute or post- acute treatment can be performed as a monotherapy or in combination with further drugs, preferably an anticoagulant, an antiplatelet drug, or a thrombolytic drug, more preferably with t-PA or a variant thereof or DSPA alpha 1.

Since the deleterious effects of the endogenous t-PA also counteract the beneficial effect of an exogenously applied thrombolytic drug, the combined use of the t-PA inhibitors should even improve the outcome of a thrombolytic therapy for thrombotic drugs that are not associated with neurotoxic and/or vasculotoxic side effects, such as e.g. DSPA alpha 1.

Hence the t-PA inhibitor can be given in combination with a thrombolytic drug that does not adversely interact with the BBB or the NMDA receptor or does not possess a deleterious effect on the vasculature or on the neural cells For such a combination DSPA alpha 1 is preferred.

According to a further embodiment of the invention the thrombolytic drug and the t-PA inhibitor are given in equimolar concentrations.

The composite according to the invention can comprise either a single preparation including a therapeutically effective amount of at least both components (the thrombolytic drug and the t-PA inhibitor) or represents two separate preparations, each containing at least a therapeutically effective amount of one of the components, the thrombolytic drug or the t-PA inhibitor. When two separate preparations are used, they can be administered to the patient either simultaneously or subsequently. Accordingly, both components can either be administered as a combinatorial preparation or concomitantly as separate preparations. Where applicable and suitable, the preparations can be administered as a single bolus or as an infusion, or a combination thereof. Also, multiple consecutive infusions can be applied.

The t-PA inhibitor and the thrombolytic drug, particularly a plasminogen activator, are given preferably non-orally as a parenteral application e.g. by intravenous or subcutaneous application. An intravenous or subcutaneous bolus application is possible. Thus, according to the invention a pharmaceutical composition is provided, which is suitable for parenteral administration.

In a further embodiment of the invention an oral administration can be performed for a nanobody specifically binding to the ATD of the NR1 subunit.

According to the invention the therapy using a thrombolytic drug, in particular t-PA or variants thereof or DSPA alpha 1 in combination with said t-PA inhibitor comprises one or more of the following characteristics:

(a) the thrombolytic drug, in particular a plasminogen activator, can be administered to the patient more than 3 hours, preferably more than 4.5 hours, preferably more than 6 hours and even more than 9 or 12 hours later than the stroke onset;

(b) the dose of the thrombolytic drug, in particular t-PA, for the treatment can be increased in comparison to the dose that is recommended for the monotherapy with said thrombolytic drug;

(c) the thrombolytic treatment can be applied in patients that are currently not eligible for a thrombolytic therapy, namely patients which suffered the stroke earlier than 3 hours, more preferably earlier than 4,5 hours before presented for the possible treatment or patients with an increased bleeding risk.

Accordingly the invention allows a thrombolytic stroke therapy beyond a 3 hours, preferably beyond a 4,5 hours therapeutic time window, even more preferred beyond 9 or even 12 hours from the onset of stroke.

Thus in one aspect of the invention the t-PA inhibitor is used to reduce, prevent or delay one or more adverse side effects that occur as a result of thrombolytic treatment. These side effects include bleedings such as intracranial or intracerebral bleedings.

In a further aspect of the invention the application of the t-PA inhibitor allows an increased dose of a thrombolytic drug above the levels given normally in the respective indication, preferably above the doses that are recommended by the manufacturer of the thrombolytic drug.

The invention allows for the use of various thrombolytic drugs. Preferably the thrombolytic drug is a plasminogen activator, more preferably recombinant t-PA (rt-PA, e.g. Alteplase) or variants of t-PA such as Pamiteplase, Lanoteplase, Reteplase, Tenecteplase or Monteplase), urokinase or DSPA variants such as DSPA alpha 1, DSPA alpha 2, DSPA beta or DSPA gamma.

As outlined above the kringle proteins of the invention can be constituted by an inactivated plasminogen activator, preferably selected from the group consisting of DSPA alpha 2, DSPA beta or DSPA gamma, urokinase, Alteplase, or variants of t-PA such as Reteplase, Pamiteplase, Lanoteplase, Reteplase, Tenecteplase or Monteplase.

The variants of DSPA (Desmodus rotundus plasminogen activator) are subject of the US patents US 5, 830, 849 and US 6,008,019, which are fully incorporated herein by reference.

Particularly the inactivated DSPA alpha 1 can be used, wherein it is preferably linked to a suicide substrate, more preferably to D-phenyl-prolyl-arginine chloromethyl ketone (PPACK).

According to this aspect of the invention said kringle protein or peptide includes one or more domains, regions or catalytic sites found in plasminogen activators or related proteins, such as e.g. the finger domain (F) the epidermal growth factor domain (EGF) or the lysine binding site. As a source for this sequence(s) recombinant t-PA (rt-PA, e.g. Alteplase) or variants of t-PA such as Pamiteplase, Lanoteplase, Reteplase, Tenecteplase or Monteplase), urokinase or DSPA variants such as DSPA alpha 1, DSPA alpha 2, DSPA beta or DSPA gamma are preferred. In a further embodiment domains, regions or catalytic sites from different plasminogen activators proteins are combined with each other.

In one preferred aspect of the invention said kringle protein or peptide consists only of the kringle domain, or a fragment thereof, wherein the fragment comprises at least 8, preferably at least 15 to 30 contiguous amino acids of a kringle domain, wherein said peptide inhibits the t-PA transport across the BBB by at least 20% as assessable according to example A, chapter 2 below.

In a specific aspect of the invention said kringle peptide consists of the kringle domain from, DSPA alpha 2, DSPA beta or DSPA gamma, urokinase, Alteplase, or variants of t-PA such as Reteplase, Pamiteplase, Lanoteplase, Reteplase, Tenecteplase or Monteplase and preferably of the kringle domain from DSPA alpha 1.

Thus the invention further relates to a protein or peptide that comprises

(a) an amino acid sequence according SEQ ID NO:3 (DSPA kringle); or

(b) an amino acid sequence with at least 70% identity, preferably 80%, more preferably 90% and most preferable 95% identity to the amino acid sequence given in SEQ ID NO:3; or

(c) an amino acid sequence with at least 92% identity, preferable at least 95% or 100% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNY-CRNPNXAXKPWCXVXK-$X_3$-F-$X_2$-ESC-$X_2$-PXC";

wherein X denotes an arbitrary amino acid;
and wherein the protein or peptide does not exhibit a serine protease activity and with the proviso that said protein or peptide is not the kringle domain of t-PA or urokinase, and is not the DSPA alpha 1 protein or t-PA covalently linked to a suicide substrate.

In another aspect of the invention an isolated antibody or fragment thereof is provided, wherein the antibody or the fragment binds to the N-terminal domain of the NMDA receptor subunit NR1 (anti-ATD-NR1 antibody) encoded by a amino acid sequence selected from SEQ ID NO: 4 or 5 or by a nucleic acid sequence selected from SEQ ID NO: 6 or 7, or alternatively binds to a protein encoded by a nucleic acid molecule that specifically hybridizes to the complement of the nucleic acid molecule of SEQ ID NO: 6 or 7 under conditions of high stringency. The high stringency can be achieved by e.g. a hybridisation that is performed in 5x SSPE, 5 x Denhardt's solution and 0.5% SDS overnight at 55 to 60°C.

According to the invention said t-PA inhibitor can be used as a neuroprotectant.

An additional aspect of the invention provides a pharmaceutical composition selected from the group consisting of one ore more of said t-PA inhitors together with or without a thrombolytic drug, wherein said pharmaceutical composition may further comprise one or more pharmaceutically acceptable carriers or excipients.

In a further aspect of the invention a method of treating a neurological or neurodegenerative disorder, in particular stroke is provided, comprising administering a therapeutically effective amount of said t-PA inhibitor, alone or in combination with therapeutically effective amount of a thrombotic drug, preferably a plasminogen activator, more preferably t-PA.

The term "neurological disorder" as used herein is defined as disease, disorder or condition which directly or indirectly affects the normal functioning or anatomy of a subject's nervous system.

In the context of the invention the term "neurodegenerative disorder" is defined as disease, in which cells of the central or peripheral nervous system are lost.

Examples for neurological and/or neurodegenerative disorders are spinal cord injury, intracranial or intravertebral lesions including, but not limited to, contusion, penetration, shear, compression or laceration lesions of the spinal cord or whiplash shaken infant syndrome.

In the context of the present invention the neurological disorders also include ischaemic events, or ischaemia or ischaemic disorders which can be defined as any local or regional state of hypoxia in cells or tissues which are usually due to an inadequate blood supply (circulation), e.g. caused by a blockage or obstruction of a blood vessel in this area.

The hypoxia can cause acute injury as in hypoxia and/or ischemia including, but not limited to, cerebrovascular insufficiency, cerebral ischemia or cerebral infarction (including cerebral ischemia or infarctions originating from embolic occlusion and thrombosis), retinal ischemia (diabetic or otherwise), glaucoma, retinal degeneration, multiple sclerosis, ischemic optic neuropathy, reperfusion following acute cerebral ischemia, perinatal hypoxic-ischemic injury, or intracranial hemorrhage of any type (including, but not limited to, epidural, subdural, subarachnoid or intracerebral hemorrhage).

Accordingly the term "ischaemic disorder" encompasses thrombotic disorders which include conditions associated with or resulting from thrombosis or a tendency towards thrombosis. These conditions include conditions associated with arterial or venous thrombosis that can be treated with a thrombolytic drug.

The term "t-PA" as used herein includes native t-PA and recombinant t-PA, as well as modified forms of rt-PA that retain the enzymatic or fibrinolytic activities of native t-PA. The enzymatic activity of t-PA can be measured by assessing the ability of the molecule to convert plasminogen to plasmin. The fibrinolytic activity of t-PA may be determined by any *in vitro* clot lysis activity known in the art. Recombinant t-PA has been described extensively in the prior art and is known to the person of skill. rt-PA is commercially available as alteplase (Activase® or Actilyse®). Modified forms of rt-PA ("modified rt-PA") have been characterized and are known to those skilled in the art. Modified rt-PAs include, but are not limited to, variants having deleted or substituted amino acids or domains, variants conjugated to or fused with other molecules, and variants having chemical modifications, such as modified glycosylation. Several preferred modified rt-PAs have been described in PCT Publication No. WO93/24635; EP 352,119; EP382174.

In the context of the invention the term "suicide substrate" is defined as a compound that resembles a normal substrate closely enough that it undergoes an enzymatic reaction to a product that inhibits the enzyme. As a substrate analogue it often binds irreversibly to an amino acid of the enzyme, usually the catalytic amino acid, thereby blocking the active site of the enzyme to other molecules and effectively and mostly irreversibly inhibits the enzyme.

The term "stroke" as used herein is used for any event of cerebral ischaemia.

The term "treating" or "treatment" refers to any medical measure for preventing, reducing, mitigating or curing physiological disorders within a mammal, in particular a human, in the need thereof.

A "therapeutically effect amount" is defined as the amount of active ingredient that will reduce the symptoms associated with a neurological or neurodegenerative disease, such as stroke. "Therapeutically effective" also refers to any improvement in disorder severity or the frequency of incidences compared to no treatment. The term "treatment" encompasses either curing or healing as well as mitigation, remission or prevention.

The term "N-terminal domain of the NMDA receptor subunit NR1" (anti-ATD NR1) as used in the context of the present is defined as the region of the NR1 subunit of the NMDA receptor that interacts with t-PA. This encompasses NMDA receptors of different species like mouse, rat, pig, bovine, cat, dog, or monkey. Preferably the human NMDA receptor is used. Different isoforms of the NR1 subunits are also included in this definition, preferably the isoforms generated by alternative splicing and including the isoforms NR1-1a, NR1-1b. NR1-2a, NR1-2b, NR1-3a, NR1-3b, NR1-4a, NR1-4b. Preferably the region of the NR1-1a subunit encoding the amino acids 19 to 480, more preferably encoding the amino acids 19 to 371 (see SEQ ID NO:4 or SEQ ID NO:5) is used.

The term "antibody" encompasses the various forms of antibodies including but not being limited to whole antibodies, human antibodies, humanized antibodies and genetically engineered antibodies like monoclonal antibodies, chimeric antibodies or recombinant antibodies as well as fragments of such antibodies as long as the characteristic properties according to the invention are retained.

The terms "monoclonal antibody" or "monoclonal antibody composition" as used herein refer to a preparation of antibody molecules of a single amino acid composition. Accordingly, the term "human monoclonal antibody" refers to antibodies displaying a single binding specificity which have variable and constant regions derived from human germline immunoglobulin sequences. In one embodiment, the human monoclonal antibodies are produced by a hybridoma which includes a B cell obtained from a transgenic non-human animal, e.g. a transgenic mouse, having a genome comprising a human heavy chain transgene and a light human chain transgene fused to an immortalized cell. Preferably said type of anti-ATD NR1 antibody is a monoclonal antibody.

The term "chimeric antibody" refers to a monoclonal antibody comprising a variable region, i.e., binding region, from one source or species and at least a portion of a constant region derived from a different source or species, usually prepared by recombinant DNA techniques. Chimeric antibodies comprising a murine variable region and a human constant region are especially preferred. Such murine/human chimeric antibodies are the product of expressed immunoglobulin genes comprising DNA segments encoding murine immunoglobulin variable regions and DNA segments encoding human immunoglobulin constant regions. Other forms of "chimeric antibodies" encompassed by the present invention are those in which the class or subclass has been modified or changed from that of the original antibody. Such "chimeric" antibodies are also referred to as "class-switched antibodies." Methods for producing chimeric antibodies involve conventional recombinant DNA and gene transfection techniques now well known in the art. See, e.g., Morrison, S.L., et al., Proc. Natl. Acad. Sci. USA 81 (1984) 6851-6855; US 5,202,238 and US 5,204,244.

The term "humanized antibody" refers to antibodies in which the framework or "complementarity determining regions" (CDR) have been modified to comprise the CDR of an immunoglobulin of different specificity as compared to that of the parent immunoglobulin. In a preferred embodiment, a murine CDR is grafted into the framework region of a human antibody to prepare the "humanized antibody." See, e.g., Riechmann, L., et al., Nature 332 (1988) 323-327; and Neuberger, M.S., et al., Nature 314 (1985) 268-270. Particularly preferred CDRs correspond to those representing sequences recognizing the antigens noted above for chimeric and bifunctional antibodies.

The term "human antibody", as used herein, is intended to include antibodies having variable and constant regions derived from human germline immunoglobulin sequences. Human antibodies are well-known in the state of the art (van Dijk, M.A., and van de Winkel, J. G., Curr. Opin. Pharmacol. 5 (2001) 368-374). Based on such technology, human antibodies against a great variety of targets can be produced. Examples of human antibodies are for example described in Kellermann, S. A., et al., Curr Opin Biotechnol. 13 (2002) 593-597.

The term "recombinant human antibody", as used herein, is intended to include all human antibodies that are prepared, expressed, created or isolated by recombinant means, such as antibodies isolated from a host cell such as a NSO or CHO cell or from an animal (e.g. a mouse) that is transgenic for human immunoglobulin genes or antibodies expressed using a recombinant expression vector transfected into a host cell. Such recombinant human antibodies have variable and constant regions derived from human germline immunoglobulin sequences in a rearranged form.

As used herein, the term "neuroprotectant" means an agent that is capable of providing neuroprotection, i.e., protecting a neural entity, such as a neuron, at a site of injury, for example, an ischaemic injury or traumatic injury.

In the context of the invention the term "acute treatment" refers to a short-term medical treatment, usually in a hospital, for patients having an acute illness or injury or recovering from surgery. The term "post-acute treatment" refers to a mid-term medical treatment, usually in a hospital, for patients having an acute illness or injury or recovering from surgery.

## EXAMPLES

### A. INHIBITION OF T-PA NEUROTOXICTY BY A PROTEIN CONTAINING A PARK DOMAIN.

MATERIALS AND METHODS

The ability of some proteins comprising the PARK domain (DSPA alpha 1, cDSPA, Reteplase, DSPA alpha 2, DSPA beta, and kringle 2 (K2) of alteplase) to inhibit alteplase trans-BBB transport was demonstrated using the materials and methods outlined below.

DSPA alpha 1, cDSPA, Reteplase, DSPA alpha 2, and DSPA beta were provided by PAION Deutschland GmbH. Reteplase was purchased in a Pharmacy and provided by PAION Deutschland GmbH. kringle 2 (from alteplase) is a compound developed and provided by UMR CNRS 61185/INSERM-Avenir, Caen France.

1. TESTED SUBSTANCES

**DSPAα1**

| | |
|---|---|
| Name of agent: | DSPAα1 (Desmoteplase) |
| Source: | PAION Deutschland GmbH. |
| Chemical / biological name: | Desmoteplase |
| Molecular weight: | 52 kDα |
| Mode of dissolution: | PBS, distilled water |

**cDSPA**

| | |
|---|---|
| Name of agent: | clogged Desmoteplase |
| Source: | PAION Deutschland GmbH. |
| Chemical / biological name: | inactivated desmoteplase, prepared by exposing DSPA to the suicide substrate, D-phenyl-prolyl-arginine chloromethyl ketone |
| Molecular weight: | 52 kDa |

**DSPAα2**

| | |
|---|---|
| Name of agent: | DSPA α2 (alpha 2) |
| Source: | PAION Deutschland GmbH. |
| Molecular weight: | 52 kDa |

**DSPAβ**

| | |
|---|---|
| Name of agent: | DSPA β (beta) |
| Source: | PAION Deutschland GmbH |
| Molecular weight: | 48.2 kDa |

**Reteplase**

| | |
|---|---|
| Name of agent: | Reteplase |

(continued)

| | |
|---|---|
| Source: | Rapilysin, pharmacy purchase, purified by PAION Deutschland GmbH (removal of excipients) |
| Chemical / biological name: | Reteplase |
| Molecular weight: | 39 kDa |

**kringle 2 (alteplase)**

| | |
|---|---|
| Name of agent: | Recombinant kringle 2 domain of alteplase |
| Source: | INSERM-Avenir, Dir D. Vivien |
| Chemical / biological name: | K2alteplase, expressed and purified from *E.coli* |
| Molecular weight: | 9.3 kDa |

**rt-PA (alteplase)**

| | |
|---|---|
| Name of agent: | alteplase |
| Source: | Boehringer Ingelheim, pharmacy purchase |
| Chemical / biological name: | Recombinant human t-PA |
| Molecular weight: | 70 kDa |

Stock solutions were diluted to obtain the required concentration (0.3 μM or 10 nM) in Ringer HEPES buffer.

2. DESCRIPTION OF THE *IN VITRO* BLOOD-BRAIN BARRIER MODEL.

2.1 ESTABLISHMENT OF THE MODEL.

To provide an *in vitro* system for studying brain capillary functions, a co-culture has been developed that closely mimics the *in vivo* situation by culturing brain capillary endothelial cells on one side of a filter and glial cells on the other (**figure 5**). Endothelial cells were cultured in the upper compartment on a filter while glial cells were maintained in the lower compartment on the plastic of a six-well plate. It is known, that under these conditions, endothelial cells retain all the endothelial markers (factor VIII-related antigen, nonthrombogenic surface, production of prostacyclin, angiotensin converting enzyme activity) and the characteristics of the blood-brain barrier (presence of tight junctions, paucity of pinocytotic vesicles, monoamine oxidase activity, gamma-glutamyltranspeptidase activity and P-glycoprotein).

2.2 CELL CULTURES

Bovine brain endothelial cells were isolated from brain capillaries, grown in DMEM supplemented with 10% (v/v) heat-inactivated calf serum and 10% (v/v) horse serum (Hyclone Laboratories, Logan, UT, USA), 2 mM glutamine, 50 μg/mL gentamicin and basic fibroblast growth factor (1 ng/mL, added every other day). Sub-clones of endothelial cells frozen at passage 3 were recultured on a 60-mm-diameter gelatin-coated Petri dish and grown to confluency.

Primary glial cultures were isolated from newborn rat cerebral cortex. After removing the meninges, the brain tissue was gently forced through a nylon sieve. DMEM supplemented with 10 % (v/v) fetal calf serum (FCS), 2 mM glutamine, and 50 μg/mL of gentamycin was used for the dissociation of cerebral tissue and development of glial cells. The glial cells were plated at a concentration of $1.25 \times 10^5$ cells $ml^{-1}$ on plastic in six-well plates and incubated at 37°C with 5 % $CO_2$. The medium was changed twice a week. Three weeks after seeding, glial cultures were confluent and composed of astrocytes (~60%), oligodendrocytes and microglial cells.

2.3 PREPARATION OF FILTERS

Culture plate inserts (Millicell PC 3 μm pore size) were coated on the upper side with rat-tail collagen prepared according to the method of Bomstein (1958).

For the analysis of sucrose permeation and compounds tested at 0.3 μM, filters with a surface of 4.2 $cm^2$ were used. For compounds tested at 10 nM, filters with a surface of 3 $cm^2$ were used.

2.4 CO-CULTURE OF BOVINE BRAIN CAPILLARY ENDOTHELIAL CELLS AND GLIAL CELLS.

Coated filters were placed in six-well dishes containing glial cells for co-culture. The co-culture medium was the same as that for brain capillary endothelial cells. Confluent endothelial cells were trypsinized and plated on the upper side of the filters at a concentration of $4 \cdot 10^5$ cells/ml. Under these conditions, endothelial cells formed a confluent monolayer within 12 days. Experiments were performed 5 days after confluence.

4. TRANSPORT EXPERIMENTS USING THE *IN VITRO* BBB MODEL.

4.1 TOXICITY TEST

Sucrose was used as a paracellular marker allowing the evaluation of possible effects of the test compounds on the integrity of the BBB. This small hydrophilic molecule shows a low cerebral penetration and its endothelial permeability coefficient is a measure of the endothelial cell monolayer integrity.

The day of the experiments, Ringer-HEPES was added to the lower compartment (abluminal side) of a six-well plate (2.5 ml per well). One filter containing a confluent monolayer of endothelial cells was transferred to the first well of the plate.

1.5 ml Ringer-HEPES containing the compound in co-incubation with [$^{14}$C]-sucrose (addition of 0.5 μCi of [$^{14}$C]-sucrose per filter) was placed in the upper compartment (luminal side). At defined times after addition of the tested compound, the insert was transferred to another well of the six-well plate to minimize the possible reflux of substances from the lower to the upper compartment (refer to **figure 6**). At the end of each incubation period, aliquots of abluminal and luminal liquid were collected for radioactive analyses.

During the 60-min experiment, the clearance volume increased linearly with time. The average cleared volumes were plotted versus time, and the slope was estimated by linear regression analysis to yield the mean and the associated Standard Error. The slope of the clearance curves for the coculture is denoted $PS_l$, where PS is the permeability x surface area product (in microliters per minute). The slope of the clearance curve for the filter only covered with collagen is denoted $PS_f$.

The PS value for the endothelial monolayer ($PS_e$) was calculated from:

$$\frac{1}{PSe} = \frac{1}{PSt} - \frac{1}{PSf}$$

The $PS_e$ values were divided by the surface area of the filter (4.2 cm$^2$ for Millicell PC and CM and 4.7 cm$^2$ for Transwell inserts) to obtain the endothelial permeability coefficient ($P_e$, in centimeter per minute).

Stability of sucrose permeability was tested with each study compound in triplicate monolayers to confirm the absence of toxic effects on the BBB.

4.2 TRANSPORT OF COMPOUNDS AT 37°C; INTEGRITY TEST

Translocation of rt-PA was determined as described under 4.1 for sucrose.

In these and all other experiments, the integrity of the BBB was monitored using [$^{14}$C]-sucrose as a paracellular marker, allowing the evaluation of possible effects on the integrity of the BBB.

Data evaluation: The passage of alteplase was evaluated by means of a plasminogen-based zymography assay. It was quantified using an image quantitative system. Moreover, triplicates of luminal and abluminal solutions were assayed for alteplase by means of a spectrozyme assay.

The sequential stages of the study are described in **figure 7.**

5. TRANSPORT STUDIES

Filter study

In this step, the ability of alteplase to cross the filter was tested to exclude the possibility of transport restriction by the filter. Alteplase was applied at the non-toxic concentration determined in the integrity test.

Transport studies

DSPA-related molecules, reteplase and kringle 2 (alteplase) were studied at equimolar concentrations for their abilities to influence alteplase permeation, evaluated by means of a plasminogen-based zymography assay and quantification by spectrozyme assay.

**EXAMPLE 1: rt-PA in combination with PARK containing proteins exerts no toxic effects on the BBB in vitro.**

Endothelial permeability coefficients obtained with sucrose alone and with alteplase alone or alteplase in combination with a DSPA-related molecule, reteplase or K2 (alteplase) are summarized in the following **table 1.**

**TABLE 1: THE SUCROSE PERMEABILTY WITH OR WITHOUT DSPA-RELATED MOLECULES**

| | PSt (μL/min) | Pe ($cm.min^{-1}$) |
|---|---|---|
| Sucrose alone (Control) | 1.21 | $0.32 \times 10^{-3}$ |
| alteplase alone (0.3 μM) | 1.14 | $0.30 \times 10^{-3}$ |
| alteplase (0.3 μM) with CDSPA (0.3 μm) | 1.15 | $0.30 \times 10^{-3}$ |
| alteplase (0.3 μM) with DSPAalpha1 (0.3 μM) | 1.06 | $0.28 \times 10^{-3}$ |
| alteplase (0.3 μM) with DSPAalpha2 (0.3 μM) | 1.06 | $0.28 \times 10^{-3}$ |
| alteplase (0.3 μM) with Reteplase (0.3 μM) | 1.11 | $0.29 \times 10^{-3}$ |
| alteplase (0.3 μM) with K2 (alteplase) (0.3 μM) | 0.98 | $0.26 \times 10^{-3}$ |
| alteplase alone (10 nM) | 0.88 | $0.23 \times 10^{-3}$ |
| alteplase (10 nM) with DSPAβ (10 nM) | 0.87 | $0.22 \times 10^{-3}$ |
| alteplase (10 nM) with DSPAγ (10 nM) | 0.81 | $0.21 \times 10^{-3}$ |

The endothelial permeability coefficients obtained for sucrose showed that alteplase in coincubation with DSPA-related molecules does not exert toxic effects on the *in vitro* BBB at the respective concentrations of 0.3 μM or 10 nM.

**EXAMPLE 2: Alteplase is able to cross the collagen filter.**

The transport of alteplase (0.3 μM) through collagen-coated filter was investigated over a period of 120 minutes.

After the experimental period, the concentration of alteplase was determined in luminal and abluminal samples by means of spectrozyme assays **(table 2).**

**TABLE 2: RESULTS FOR THE FILTER STUDY WITH ALTEPLASE (SPECTROZYME ASSAY)**

| Filter study | % passage at 120 min |
|---|---|
| alteplase at 0.3 μM | 7.88 +/- 0.33 § |
| § PERCENTAGE OF ABLUMINAL ALTEPLASE ALONE | |

The results show that alteplase permeation is not restricted by the empty filter, since alteplase showed the potential to cross the filter to an extent of 7.88%. This value was registered as the maximum percentage of diffusional passage through the empty filter and used in the endothelial transport studies as a reference (the so called "versus filter").

**EXAMPLE 3: Transport of rt-PA through the BBB is blocked in vitro by co-incubation with proteins containing a PARK domain.**

Translocation of alteplase alone and in coincubation with DSPA-related molecules (DSPAα1, cDSPA, DSPAα2), Reteplase and K2 (alteplase) was studied over a 120 min period. Equimolar concentrations (0.3 μM) were used.

After the experimental period, the luminal and abluminal concentrations of alteplase were determined by means of a plasminogen containing zymography assay (**figure 8**) and quantified using an image system.

As shown in figure 8, the zymography assay revealed inhibition of alteplase transport across the BBB by cDSPA, DSPAα1, DSPAα2 and K2 (alteplase) as well as by Reteplase, all of which caused a decrease in the zymography signal. Quantitated data are summarized in **table 3.**

**TABLE 3: INHIBITION OF ALTEPLASE TRANSPORT (ZYMOGRAPHY ASSAY)**

| | % of abluminal alteplase |
|---|---|
| Control alteplase at 0.3 μM | 100 |
| alteplase (0.3 μM) and cDSPA (0.3 μM) | 73.3 +/- 1.43 § |
| alteplase (0.3 μM) and DSPAalpha1 (0.3 μM) | 46.6 +/- 1.11 § |
| alteplase (0.3 μM) and DSPAalpha 2 (0.3 μM) | 25.1 +/- 1.28 § |
| alteplase (0.3 μM) and Reteplase (0.3 μM) | 51.6 +/- 1.81 § |
| alteplase (0.3 μM) and K2 (alteplase) (0.3 μM) | 75.2 +/- 1.93 § |
| § PERCENTAGE OF ABLUMINAL ALTEPLASE ALONE | |

In a further series, spectrozyme assays were performed in both the luminal and abluminal compartment to quantify the passage of alteplase (0.3 μM) during coincubation with DSPA-related molecules and K2 (alteplase) (all at 0.3 μM) (table 4).

**TABLE 4: EFFECTS OF POTENTIAL INHIBITORS ON ALTEPLASE TRANSLOCATION ACROSS THE BBB (SPECTROZYME ASSAY); ND : NOT DETERMINED**

| | alteplase (% of passage versus filter) § |
|---|---|
| Control (alteplase at 0.3 μM) | 100 |
| alteplase (0.3 μM) and cDSPA (0.3 μM) | 76.30 +/- 1.30 |
| alteplase (0.3 μM) and DSPAalpha1 (0.3 μM) | 66.95 +/-1.55 |
| alteplase (0.3 μM) and DSPAalpha2 (0.3 μM) | 61.92 +/- 1.75 |
| alteplase (0.3 μM) and Reteplase (0.3 μM) | Nd |
| alteplase (0.3 μM) and K2 (alteplase) (0.3 μM) | 50.54 +/- 2.15 |
| § PERCENTAGE OF PASSAGE VERSUS FILTER FOR ALTEPLASE ALONE. | |

Because of the protease activity of reteplase the spectrozyme assay could not be used, as it was impossible to distinguish between activities related to alteplase and reteplase.

As illustrated in **figure 9**, the spectrozyme assay revealed a decrease in the global proteolytic activity of alteplase in the presence of DSPA-related molecules and K2 (alteplase). Figures are also shown in table 4.

**EXAMPLE 4: DSPA antagonized rt-PA mediated neurotoxic effects after i.v. coadministration but not after coadministration into the striatum.**

The inhibitory effect of a protein comprising a PARK domain was also shown in an animal model as outlined below:

Human recombinant alteplase (rt-PA, alteplase) was from Boehringer Ingelheim (France), NMDA from Tocris (U.K.). Desmoteplase was provided by PAION Deutschland GmbH (Germany).

Animals

Male Sprague Dawley rats (270 to 330 g) were housed in a temperature-controlled room on a 12-hour light/12-hour dark cycle, with food and water *ad libitum.* Experiments were performed in accordance with French (act no. 87 to 848; Ministère de l'Agriculture et de la Forêt) and European Communities Council (Directives of November 24, 1986, 86/609/EEC) guidelines for the care and use of laboratory animals.

Striatal Excitotoxic Lesions

Rats were anesthetized with isoflurane (5%, maintenance 2% in oxygen/$N_2O$ (1:3) at 0.8 l/min). Body temperature was maintained at 37±0.5°C. Injection pipette (internal diameter 0.32 mm and calibrated at 15 mm/μl; Hecht Assistent, Germany) was stereotaxically implanted in the right striatum (3.5 lateral and 5.5 ventral to the bregma). NMDA (50 nmol) was injected in a volume of 1 μl and the pipette was removed 3 minutes later. In the first set of experiments, excitotoxic treatment was complemented after 15 minutes by intravenous injection of alteplase (1 mg/kg), desmoteplase (1 mg/kg), alteplase plus desmoteplase (1 mg/kg each), alteplase vehicle (L-Arg 35 mg/kg, phosphoric acid 10 mg/kg and polysorbate 80, 0.2 %) or desmoteplase vehicle (glycine 4 mg/kg and mannitol 10.64 mg/kg). In the second set of experiments, NMDA was coinjected into the striatum with alteplase (3 μg), desmoteplase (3 μg), alteplase plus DSPA (3 μg each) or the corresponding vehicles, all in a volume of 1 μl.

Histological Analysis

After 24 hours, rats were euthanized and the whole brain was removed and frozen in isopentane. For volume analysis, one coronal section (20 μm) out of every 20 was stained with thionine and analyzed, covering the entire lesion. Regions of interest were determined using a stereotaxic atlas for the rat (Paxinos and Watson). An image-analysis system (BIOCOM RAG 200. Paris, France) was used to measure the lesion given by the non stained area.

This analysis showed that Desmoteplase antagonized rt-PA mediated neurotoxic effects when both were co-injected intravenously but not when co-administered into the striatum **(figures 10 and 11),** consistent with a relevant *in vivo* competition at the BBB level between these two thrombolytic agents.

**CONCLUSION**

These results show the ability of various proteins comprising a PARK domain to inhibit alteplase trans-BBB transport.

Alteplase alone and in co-incubation with DSPAα1, cDSPA, DSPAα2, Reteplase and K2 (alteplase), all at a concentration of 0.3 μM, had no toxic effects on BBB integrity. As analyzed by means of the spectrozyme assay, DSPAα1 and DSPα2 were able to reduce alteplase transport across the BBB to approximately 67 and 62%, respectively, confirming that DSPA-related molecules can be used to block alteplase blood-to-brain translocation. Similar results (reduction to about 76%) were obtained for clogged DSPA, a DSPA variant that does not exert proteolytic activity. Inhibition was confirmed in zymography quantitation of alteplase.

In addition, isolated kringle 2 was able to reduce alteplase transport by about 50 % (spectroscopy data, confirmed by zymography).

Also Reteplase was able to inhibit alteplase translocation as demonstrated in alteplase zymography analysis. As Reteplase possesses protease activity, its effect could not be quantified by means of the spectrozyme assay.

The molecules tested in this study are able to inhibit rt-PA trans-BBB transport, most likely by competing for LRP-mediated transport. According to the spectrozyme data, they can be ranked as follows: K2 (t-PA) > DSPAα2 > DSPAα1 > cDSPA.

**B. INHIBITION OF T-PA NEUROTOXICTY BY AN ANTI ATD-NR1 ANTIBODY**

MATERIALS AND METHODS

*Production of recombinant A TD/Leucine-Isoleucine-Valine-binding Protein (LIVBP)-like Domain*: The region of the NR1-1a subunit encoding amino acids 19-371 corresponding to the amino terminal domain of NR1, identified as the domain of interaction with t-PA, was amplified from the full-length rat NR1-1a cDNA, as previously described (Femandez-Monreal et al., 2004). The recombinant protein, designed rATD-NR1, was purified from inclusion bodies of isopropyl 1-thio-β-D-galactopyranoside-induced bacterial cultures (Escherichia coli, M15 strain) on a nickel affinity matrix as described by the manufacturer (Qiagen, France).

*Active immunisation*: Briefly, as previously described with slight modifications (Benchenane et al., 2007), mice were immunised by intraperitoneal injection of immunogenic mixtures: Complete Freund's adjuvant (Sigma Aldrich, France used for the first injection) and Incomplete Freund's adjuvant (Sigma Aldrich, France; later injections, once a week during 3 weeks) containing the rATD-NR1 (30 μg); the same mixture without the rATD-NR1 was used as a control serum.

*Production and purification_of polyclonal_antibodies*: Sera were collected 2 weeks after the last inoculation from mice immunised with rATD-NR1 or control adjuvant as described for active immunisation. Polyclonal antibodies (anti-ATD-NR1 antibodies or control Igs) were then purified from the serum hydroxyapatite columns (Proteogenix, France).

*Immunoblotting*: rATD-NR1 (20 μg) or protein extracts as mentioned in the corresponding figures were loaded and separated by 10% SDS-PAGE and transferred onto a PVDF membrane. Membranes were blocked with Tris-buffered saline (10 mM Tris and 200 mM NaCl, pH 7.4) containing 0.05% Tween-20, 5% dry milk. Blots were exposed overnight either with a mouse anti-ATD-NR1 primary antibody (1:2000; 4°C), mouse control Igs primary antibody (1:2000; 4°C) or a control goat histidine protein primary antibody (1:1000; 4°C, Qiagen, France). After mouse or goat peroxydase-conjugated secondary antibodies (1:5000; Sigma Aldrich, France), proteins were visualized with an enhanced chemiluminescence ECL-Plus detection system (Perkin Elmer-NEN, France).

*Thromboembolic focal cerebral ischaemia:* As previously described (Orset et al., 2007), male Swiss mice (28-30 g, CERJ, Paris, France) were deeply anesthetised (using isoflurane 5% with 70%/30% mixture of $NO_2/O_2$) and placed in a stereotaxic device; the skin between the orbit and ear was incised and the temporal muscle was retracted. A small craniotomy was performed, the dura was excised and the middle cerebral artery (MCA) exposed. The pipette was introduced into the lumen of the MCA and 1μL of purified murine alpha-thrombin (0.75 UI, Gentaur, Belgium) was pneumatically injected (by applying positive pressure with a syringe connected to the pipette via a catheter) to induce the in situ clot formation. The pipette was removed 10 min after the injection of the alpha-thrombin at which time the clot is stabilized. The muscle and soft tissue was replaced and the incision sutured. Cerebral blood velocity was monitored by laser Doppler flowmetry (Oxford Optronix, United Kingdom) and the temperature was controlled over the whole surgical procedure. To induce thrombolysis, rt-PA (10 mg/kg; Actilyse®, Boehringer Ingelheim, Germany) was intravenously injected (tail vein, 10% bolus, 90% perfusion during 40 minutes) 20 minutes or 4 hours after the injection of alpha-thrombin. The control group received the same volume of saline under identical conditions.

*Passive immunisation:* 20 minutes or 4 hours after clot formation, purified polyclonal antibodies raised against the rATD-NR1 were intravenously injected in the tail vein by a bolus of 0.2 mg of anti-ATD-NR1 or control immunoglobulins (200 μl).

*Histological analysis of brain lesions:* After 24 hours, mice were killed by anaesthetic overdose and the brains were removed and frozen in isopentane. Cryostat-cut coronal brain sections (20 μm) were stained with thionine and analysed by using an image analyser (BIOCOM RAG 200, Paris, France). For volume analysis, one section of 20 μm out of 10 was stained and analysed (covering the whole lesion). Regions of interest (non-stained areas corresponding to infarcted tissues) were determined through the use of a stereotaxic atlas for the mouse (Franklin and Paxinos, 1997). The infarct volume was determined as the sum of the unstained areas of the sections multiplied by their thickness according to the equation: $V_{ischaemic}$ lesion =P(Areas of ischaemic lesion) distance between sections. This method has been validated previously, demonstrating an excellent reproducibility of the volumetric assessment of the ischaemic lesion (Osborne et al., 1987).

*MRI analyses*: were performed at 24 hours, 72 hours, 7 days, 15 days after thromboembolic ischaemia performed in mice intravenously injected with saline or the α-ATD-NR1 antibody 4 hours after clot formation. MRI experiments were carried out on a Pharmascan 7 T/12 cm system with Paravision 4.0 software (Bruker, Ettlingen, Germany) using a 72mm inner diameter birdcage for radio frequency transmission and a 25mm diameter surface coil for reception. During the MRI experiments, anesthesia was maintained using isoflurane (70%/30% mixture of $NO_2/O_2$). Mice were monitored for changes in their respiratory rate in order to adjust the anesthetic concentration. T2-weighted images were acquired using a RARE sequence: TE/TR 41/ 3000 ms with 4 averages (Matrix 256x256, FOV 23.7x20mm). Diffusion-weighted images were acquired with a 6 directions EPI-DTI sequence: TE/TR 34/3750 ms using two b values (0 and 800 s/mm2) with 2 experiments for each direction (Matrix 128x128, FOV 20.5x19.2mm). Apparent diffusion coefficients (ADCs) in mm2/s were calculated by pixel-by-pixel curve fitting using a monoexponential model. Angiographies were acquired with TE/TR 12/7 ms with 2 averages (Matrix 256x256, FOV 20x20mm).

*Evan's blue extravasation*: Evan's blue (200 μl, 2%, Sigma-Aldrich) was intravenously injected in ischaemic mice 3 hours before euthanasia. The mice were perfused at their mean arterial pressure with heparinised saline prior to harvesting their brain. The cortices were dissected, homogenised in phosphate buffered saline with sodium dodecyl sulphate 1% and centrifuged (10000g, 15 minutes). Evan's blue was quantified in supernatants from the absorbance at 620nm and divided by the wet weight of each hemisphere. Each observation was repeated three times.

*Behavioural analyses*: Mice were subjected to a global neurological function test 24 hours post cerebral ischaemia This behavioral test was performed in a chamber (67x53x55 cm, BIOSEB®, Chaville, France) formed by black methacrylate walls and featuring a Plexiglas front door. The floor of the chamber consisted of 22 stainless steel bars (3 mm in diameter, spaced 1.1 cm apart, center-to-center), which were wired to a shock generator with scrambler for the delivery of unconditioned foot shock stimuli. The signal generated by mouse movement was recorded over a 5 min period and analysed by means of a high sensitivity Weight Transducer system. The analog signal was transmitted to the Freezing software module through the load cell unit for recording and off-line analysis for mobility as indicated by periods of activity/immobility (Freezing). An additional interface associated with corresponding hardware allowed controlling the intensity of the shock from the Freezing software.

*Cell culture*: Primary cultures of cortical neurons were prepared from foetal mice (E15-E16). Dissociated cortical cells were resuspended in Dulbecco's modified Eagle's medium (OMEM) supplemented with 5% fetal bovine serum, 5% horse serum and 2 mmol/L glutamine and plated in 24 well dishes previously coated with poly-D-lysine and laminin. After 3 days, the cells were exposed to 10 μmol/L of Ara-C to inhibit glial proliferation. Cultures were used after 12 days in vitro.

*Excitotoxicity*: Slowly or rapidly triggered excitotoxicity respectively, was induced at 37° C by exposing neuronal cultures to 10 μmol/L NMDA for 24 hours and 50 μmol/L NMDA for 1 hour in DMEM supplemented with glycine (10 μmol/L). The exposure to NMDA was performed alone, or in combination with rt-PA (20 μg/mL) and/or α-ATD-NR1 or control Igs (0.01 mg/ml). In both cases, neuronal death was assessed after 24 hours using phase-contrast microscopy and quantified by measurement of lactate dehydrogenase (LDH) release from damaged cells into the bathing medium.

*Calcium videomicroscopy analysis*: Primary cultures of cortical neurons were loaded with HEPES-buffered saline solution containing 5 μmol/L of fura-2/AM plus 0.1 % pluronic F-127 (Molecular Probes, Leiden, the Netherlands) (30 minutes, at 37°C) and incubated for an additional 30 minutes period in a HEPES-buffered saline solution. Experiments were performed at 22°C on the stage of a Nikon Eclipse inverted microscope equipped with a 75 W Xenon lamp and a Nikon x40, 1.3 numerical aperture epifluorescence oil immersion objective. Fura-2 (excitation: 340, 380 nm, emission: 510 nm) ratio images were acquired with a CCD camera (Princeton Instrument, Trenton, New Jersey), and digitized (256 x 512) using Metafluor 4.11 software (Universal Imaging Corporation, Cherter, Pennsylvania).

*Induction of apoptosis*: Serum deprivation (SD) was induced by the exposure of neuronal cultures (DIV7) to a serum-free DMEM supplemented with 10 microM of glycine and characterized as previously described (Koh et al. 1995; Nicole et al. 2001 a; Liot et al. 2004). Controls were maintained in serum-containing fresh medium. MK-801 (1 microM) was added to prevent secondary NMDA receptor activation. Before fixation in 4% paraformaldehyde, cells were stained with 0.4% trypan blue for 15 min. SD was performed alone, or in combination with rt-PA (20μg/mL) and/or anti-ATD-NR1 antibodies (0.01 mg/ml). Neuronal cell injury was quantified by counting trypan blue positive cells in three random fields per well. The percentage of neuronal death was determined as the number of trypan blue positive neurones after SD compared with the total number of neurones. The mean values of trypan blue positive neurones in sham washed control conditions were subtracted from experimental values. Densitometric analysis data (Image J software) of corresponding immunoblots were normalized to mean values obtained for control conditions.

*Gelatin zymography analysis for MMP-2 and -9:* Gelatinase assay was performed to evaluate MMP-9 and MMP-2 levels in ischaemic mice cerebral sample. Briefly, tissues lysates (25 μg of proteins) were loaded onto 10% SDS-polyacrylamide gels copolymerized with 1 mg/mL gelatin (Sigma Aldrich, France) for electrophoresis. After electrophoresis, gels were washed in 2.5% Triton X-100 and then incubated for 36 hours at 37°C with a developing Tris buffer before staining with Coomassie blue. Gels were then destained and MMP-2 and -9 band intensity was quantified.

**EXAMPLE 1: Active immunization against the interaction site of t-PA on NMDAR is protective in a model of in situ thromboembolic stroke with rt-PA-induced late reperfusion.**

Focal ischaemia was induced in mice by in situ injection of plasma purified murine thrombin into the MCA (Orset et al., 2007). Immediately after thrombin injection, clot formation was evidenced by a dramatic reduction (mean reduction of 80%) of the cerebral blood velocity (CBV) as measured by laser Doppler flowmetry (Fig. 12B and 12D). The hypo perfusion was stably established, unless rt-PA was administered iv (early or late), restoring CBV to 60-70 % of baseline values within 25 minutes post rt-PA infusion (Fig. 12B and 12D, n= 10, p<0.001). After 24 hours, regardless of the treatment, all mice showed brain infarction (thionine staining) which was restricted to the cortex. Early rt-PA-induced thrombolysis (started 20 minutes after clot onset) significantly reduced the extent of ischaemic brain damages, with a mean lesion volume of 18.94 $mm^3 \pm 1.85$ (n=10), demonstrating a 27.76% (p<0.01) protective effect of rt-PA compared to untreated animals (26.22 $mm^3 \pm 2.47$; n=10; Fig. 12A).

Previous active immunization of mice with the recombinant amino-terminal domain of the NR1 subunit of the NMDAR (rATD-NR1) as an antigen altered neither clot formation nor rt-PA-induced reperfusion (Fig. 12B and 12D). rATD-NR1-immunised mice displayed strongly reduced brain damages (43.3% of protection compared to controls, n=10, p<0.002). There was no additive beneficial effect on lesion volume when reperfusion was produced by early (20 min post-stroke) rt-PA treatment in these mice (12A). The same treatment 4 hours after clot formation, was associated with increased brain damage when rt-PA was injected in mice not immunized with rATD-NR1 (21.56 $mm^3$ for PBS-injected mice compared to 28.60 $mm^3$ for rt-PA-injected animals, n=10, p<0.001), even though rt-PA was able to restore CBV to an extent comparable to that induced by early reperfusion (Fig. 12D; n=10, p<0.001). However, when late thrombolysis was performed using t-PA in rATD-NR1 immunized mice, brain protection was recovered to a similar extent as observed in parallel mice, that had also been immunized, but were injected with saline and secondly, in animals undergoing early thrombolysis (54.91 % and 66.01% of protection compared to control and t-PA, respectively, n=7, p<0.01; Fig. 12C). Altogether these data show that, although early reperfusion by t-PA is beneficial, delayed rt-PA-induced reperfusion worsens brain damage in the face of efficient recanalization. Second, the data show that rATD-NR1 immunization in a murine model of thromboembolic stroke confers brain protection, alone or as an adjunct to late t-PA-induced thrombolysis.

**EXAMPLE 2: Antibodies raised against the ATD-NR1 prevent t-PA-promoted NMDAR-mediated neurotoxicity.**

The results given in example 1 provided proof of concept for the idea that antibodies against the amino-terminal domain of the NR1 subunit of the NMDA receptor are beneficial in acute ischemic stroke. However, as active immunization is not feasible as a means to treat an acute disorder, the inventors developed a strategy of passive immunization (antibody-based immunotherapy), based on purified serum immunoglobulin from rATD-NR1-vaccinated mice. The inventors first controlled by immunoblotting that purified polyclonal anti-ATD-NR1 antibodies can recognize the immunogenic peptide. The anti-ATD-NR1 antibodies could independently recognize two forms of rATD-NR1, coupled with either a histidine-tag (Fig. 13A; 37 kDa) or a Fc-tag (data not shown). Similarly, anti-ATD-NR1 antibodies were found to interact with a protein of around 120 kDa in human brain tissue, corresponding to the expected molecular weight of the NR1 subunit of NMDAR (Fig. 13B). Control antibodies purified from Freund's adjuvant mixture-injected mice showed no positive staining (Fig. 13A).

The next step was to validate the ability of these antibodies to prevent the aggravating effect of rt-PA on NMDA toxicity (Nicole et al., 2001). In primary cultures of murine cortical neurons exposed to either a high concentration of NMDA (50$\mu$M) for a short period (1 hour) or a moderate concentration of NMDA (10$\mu$M) for a long period (24 hours), the co-application of the anti-ATD-NR1 antibodies completely prevented the promotion of neuronal loss induced by rt-PA (0.3$\mu$M) (Fig. 14A, N=3, n=12, p<0.01 and Fig. 14B, N=3, n=12, p<0.01. Control antibodies had no effect on t-PA-promoted, NMDA-induced neuronal death (Fig. 14C and 14D). We then investigated whether the effects observed in the excitotoxic paradigms were correlated with a modulation of NMDA-evoked $Ca^{2+}$ influx, one of the critical events in excitotoxic necrosis. Fura-2 fluorescence video microscopy measurements revealed that rt-PA (0.3 $\mu$M) increased NMDA-induced $Ca^{2+}$ influx in cultured cortical neurons by approximately 30%. The co-application of the anti-ATD-NR1 antibodies prevented rt-PA-induced potentiation of NMDA-mediated calcium influx (Fig.15, N=3, n=108, p<0.05). Control antibodies were without effect (Fig. 15). Interestingly, the beneficial non-proteolytic activity of t-PA against serum-deprivation-induced NMDAR-independent apoptotic neuronal death (Liot et al., 2006) was not prevented by the co-application of anti-ATD-NR1 antibodies (Figure 23; N=3, n=12), a finding supporting the specificity of the antibodies in their targeting the NMDA receptor.

**EXAMPLE 3: Antibody-based immunotherapy targeting the ATD-NR1 improves neurological outcome, protects the brain against stroke and increases the therapeutic window of rt-PA-induced thrombolysis.**

The therapeutic value of the anti-ATD-NR1 antibodies (passive immunisation) was then investigated in vivo. First, excitotoxic lesions were induced in mice by administrating NMDA (10 nmol) into the striatum together with a single intravenous injection of control or purified antibodies. In control animals, NMDA led to an excitotoxic lesion of 17 $\pm$ 2 $mm^3$, while in anti-ATD-NR1 antibodies-treated mice, the lesion (9 $\pm$ 1 $mm^3$) was reduced in size by 47.06 % (n=8 in each group, p<0.01; Fig 20. Thus, a single intravenous injection of the anti-ATD-NR1 antibodies can prevent the deleterious effect of endogenous t-PA in this model of excitotoxicity. Accordingly, anti-ATD-NR1 antibodies (0.8 mg/ml, iv, single bolus; n=8-10 per group) were then tested in a model of thromboembolic stroke in mice. It is important to note that the co-administration of antibodies (control or anti-ATD-NR1) did not alter the ability of early rt-PA injection to induce reperfusion (Fig. 16B). Early rt-PA-induced thrombolysis led to a reduction of the volume of ischaemic lesion by 31.53 % (Fig. 16A). Similarly, early (20 minutes post-ictus) delivery of the anti-ATD-NR1 antibodies conferred a significant brain protection (44 % of protection compared to control, n=10, p<0.001). This effect was not improved when the antibodies were combined with rt-PA-induced reperfusion (Fig. 16A). When performed 4 hours after clot formation, rt-PA treatment still restored the CBV as efficiently as in the early reperfusion experiments (Fig. 16D) (80% of the initial CBF after clot formation versus 85% after rt-PA treatment, n=9, p<0.01): However, brain lesions were dramatically worsened under these conditions (24.90 $mm^3$ for PBS-injected mice compared to 33.03 $mm^3$ for rt-PA-injected animals 4 hours after clot formation, n=10, p<0.0025; Fig. 16C). This deleterious effect was not observed when rt-PA was co-administered with a single bolus of anti-ATD-NR1 antibodies. Rather, reductions of lesion volume by 50.52 % and 62.7 % were observed, compared to control experiments (only saline) and rt-PA-treatment, respectively (n=8, p<0.005). Interestingly, also the delayed injection of anti-ATD-NR1 antibodies alone was highly protective (-41 % when compared to control animals, n=10, p<0.002). Altogether these data show two important capabilities of a single intravenous injection of antibodies against the ATD-NR1, targeting the potentiating effect of t-PA on NMDAR signaling and neurotoxicity: Such an injection is of therapeutic value by itself and, second, can also extend the therapeutic window of thrombolysis by rt-PA.

Twenty four hours post-occlusion, stroke-induced cognitive impairment was evaluated in a contextual freezing task (n=10), which is considered to allow a global cognitive evaluation of anxiety, memory and learning processes. Compared to sham animals, ischaemic mice displayed an increase in freezing (+214 %, p<0.025), an effect which was dramatically reduced (-170 %; p<0.05) by the intravenous administration of the α-ATD-NR1 antibodies (Fig. 19).

**EXAMPLE 4: Antibody-based immunotherapy against the ATD-NR1 prevents rt-PA-Induced BBB leakage after stroke.**

Since ischaemic brain injuries as well as t-PA's beneficial or deleterious effects are highly related to the blood-brain barrier (BBB), Evan's blue (EB) extravasation and matrix metalloproteinases activation (MMP-2 and MMP-9) were evaluated in the conditions described above. As expected, rt-PA was found to be able to induce the activity of MMP-9 and enhance extravasation of Evan's Blue into brain parenchyma. The later after clot formation rt-PA was administered, the stronger were the effects on MMP-9 activity (Fig. 17B and C) and extravasation of EB (Fig. 17A). Consistent with their effects on ischaemic lesions, both early and late administrations of the anti-ATD-NR1 antibodies alone significantly reduced the extent of BBB leakage induced by stroke (-63 %, 20 minutes after clot formation, and -68%, 4 hours after clot onset, when compared to control clot animals, n=3, p<0.05; Fig. 17A). In addition, anti-ATD-NR1 immunotherapy efficiently reduced the damaging effect of rt-PA on the integrity of the BBB (28 % of reduction 20 minutes after clot formation, 42 %, 4 hours after clot onset, when compared to control animals, n=3, p<0.05; Fig. 17). Also important, MMP-9, which is instrumental in the effects of rt-PA on the BBB, showed reduced activity in the presence of the anti-ATD-NR1 antibodies, regardless of rt-PA being co-administered or not (Fig 17B). These findings allow the conclusion that the antibodies protect the integrity of the BBB from endogenous t-PA (released from neurons in response to ischemia) as well as from exogenous rt-PA and, second, that this protection involves interference with the activity of MMP-9.

**EXAMPLE 5: Antibody-based immunotherapy targeting the ATD-NR1 confers long-term benefits after stroke.**

Of highest clinical relevance are long-term follow-ups of the consequences of acute treatment. Thus, an MRI-based longitudinal follow-up of animals treated 4 hours post clot onset with a single injection of anti-ATD-NR1 antibodies was undertaken over a 3 month period. T2 MRI analyses clearly confirmed the patterns of thionine staining observed earlier, evidencing a brain protective effect of the antibodies which was already visible 24 hours after inducing ischaemia and maintained for at least 15 days post-surgery (**x**% of reduction when compared to control animals, n=3, **p<x**; Figs. 18a,b). Furthermore, ADC sequences revealed the absence of edema at all time points in the ischaemic animals treated with the anti-ATD-NR1 antibodies (figures 18a, 18b).

**CONCLUSIONS**

A single intravenous injection of anti-ATD-NR1 antibodies is sufficient to cause dramatic neuroprotection when applied either early (20 minutes) or late (4 hours) after clot onset in a murine model of thrombo-embolic stroke. Of note, the current invention shows a time-dependent loss of the benefit of rt-PA treatment: Although early rt-PA-induced reperfusion produces benefits, as observed in the clinical use of this drug, delayed rt-PA-induced reperfusion (4 hours post clot formation) is even associated with a deleterious outcome, despite comparable levels of reperfusion. This negative outcome is healed by the co-administration of anti-ATD-NR1 antibodies, which restore the neuroprotective efficacy and, by this, extend the time window, in which rt-PA-induced thrombolysis is able to confer therapeutic benefit.

Further, the present invention of immunotherapy with anti-ATD-NR1 antibodies provides means to reduce the increase in BBB (considered a prelude to intracerebral hemorrhage), which is attendant to cerebral ischaemia and aggravated by late rt-PA-treatment under ischemic conditions. These observations reinforce the therapeutic interest in the anti-ATD-NR1 antibodies, since brain edema and bleeding are associated with a poor clinical outcome in stroke patients.

To be borne in mind, t-PA has also been reported to serve critical physiological functions in: (i) synaptic remodeling and plasticity, (ii) neuronal migration during perinatal development, (iii) control of mechanisms involved in memory and learning processes such as the Late-phase Long Term Potentiation (L-LTP) in the hippocampus, and (iv) activation of pro-BDNF into BDNF. Hence it is important to note that the antibodies of the current invention have been found innocuous where tested for a possible impact on these functions: In the absence If ischaemic lesions, no changes of cognitive functions, including spatial memory, contextual and cue fear conditioning, could be identified, suggesting a safe profile of the presented strategy of immunotherapy.

**REFERENCES:**

- Benchenane et al., 2005a, Circulation 111(17): 2241-2249
- Benchenane et al., 2005b, Stroke 36(5): 1065-1070
- Benchenane et al., 2007, J. Cell Science120(Pt4): 578-585
- Fernandez-Monreal et al., 2004, J. Biol. Chem. 279(49) :50850-50856
- Franklin and Paxinos, 1997;"The mouse brain in stereotactic coordinates, Academis Press.
- Günzler et al. 1982, Hoppe-Seyler's, Z. Physiol. Chem. 363(10): 1155-1165
- Kellermann et al, 2002, Curr. Opin. Biotechnol. 13: 593-597
- Liot et al., 2004, J. Cereb. Blood Flow Metab. 24(10): 1153-1159
- Liot et al., 2006, J. Neurochem 98(5) : 1458-1464.
- Morrison et al. 1984, Proc. Natl. Acad. Sci. USA , 81: 6851-6855
- Neuberger et al. 1985, Nature 314: 268-270
- Orset et al., 2007, Stroke 38(10): 2771-2778
- Pennica, et al. 1983, Nature, 301(5897): 214-221
- Riechmann et al. 1988, Nature, 332 : 323-327
- Sottrup-Jensen et al. 1978, in "Progress in Chemical Fibrinolysis and Thrombolysis" (eds. Davidson et al), 3: 191ff
- van Dijk and van de Winkel, 2001, Curr. Opin. Pharmacol. 5: 368-374
- Walz et al. 1977; Proc. Nat'l. Acad. Sci. USA, 74: 1969-1972
- Yepes et al., 2009; Trends Neurosci 32(1): 48-55


**Legend to figures**

**Figure 1: Computer-based 3D model illustrating conserved and similar amino acids comparable to the PARK domain.** Amino acids highlighted in green represent conserved amino acids, while red displays similar amino acid residues.

**Figure 2: Binding site of the PARK domain exemplified pictured by kringle of DSPA alpha1, and the kringle 1 and kringle 2 of rt-PA.**

**Figure 3: 3D computer-based model of clogged DSPA alpha 1.** The structure of DSPA after 4400 psec of MD simulation by in silico methods (Swiss Model, GROMOS96, POVRAY) in ribbon representation including the D-phenyl-prolyl-arginine chloromethyl ketone coupled to the catalytic leading to clogged, catalytically inactive DSPA. Light Blue: Finger domain, red: EGF domain; yellow: kringle domain, green: catalytic domain

**Figure 4: 3D models of alteplase and desmoteplase.** The structure of DSPA and rt-PA after 4400 psec and 4000 psec respectively of MD simulation by in silico methods (Swiss Model, GROMOS96, POVRAY) in ribbon representation. Light Blue: Finger domain, red: EGF domain; yellow: kringle domain of DSPA and kringle 1 domain of t-PA; blue: kringle 2 domain of t-PA, green: catalytic domain

**Figure 5: Scheme of the co-culture of endothelial cells and glial cells.**

**Figure 6: Scheme of the permeability studies**

**Figure 7: Experimental procedure of the determination of the BBB transport**

**Figure 8: Zymography assays for rt-PA transport competition (0.3 μM)** After 2 hours of treatment in the presence of the different compounds indicated, bathing media corresponding to the abluminal compartments (see figure (5) were harvested and subjected to plasminogen and casein containing zymography assays to reveal plasminogen-like activators based on their respective molecular weights and their abilities to activate plasminogen (2 hours of incubation at 37°C) with subsequent casein digestion.

**Figure 9: Bars, lefthand axis: Transport of rt-PA with and without DSPA-related molecules and K2 (t-PA).** The transport was assessed using a spectrozyme assay. Line, righthand axis: unchanged permeability of sucrose (Pe sucrose)

**Figure 10: In vivo antagonism of NMDA-mediated neurotoxicity by DSPA.** Effects of intravenous injection of rt-PA and/or desmoteplase (DSPA) (1 mg/kg each) and their vehicles on the extent of neuronal death induced by the striatal administration of NMDA (50 nmol). *$P$~0.01. ANOVA with Bonferroni correction.

**Figure 11: In vivo antagonism of NMDA-mediated and t-PA enhanced neurotoxicity by DSPA.** Effects of intrastriatal injection of rt-PA and/or desmoteplase (DSPA) (3 μg each) on the extent of neuronal death induced by striatal administration of NMDA (50 nmol). *$P$~0.001, ANOVA with Bonferroni correction

**Figure 12: Active immunisation against the ATD-NR1 is protective in a model of in situ thromboembolic stroke with rt-PA-induced reperfusion.**

(A, C) Ischaemic lesions were performed 11 days after the last inoculation of the recombinant rATD-NR1, by an in situ injection of thrombin (0.75 U.I) into the MCA in Swiss mice (n=10 per group). After 20 minutes (A) or 4 hours (C), rt-PA (10 mg/kg) or saline was injected over 40 minutes (10 % Bolus, 90 % infusion). Brains were harvested 24 hours later, and cerebral lesions were measured by analysing thionine-stained cryostat sections (see representative section for each group). Results are expressed as mean ± SD. *: $p<0.01$

(B, D) The normalized cerebral blood velocity measured by laser Doppler showed that immunisation against the ATD of the NMDA receptor NR1 subunit does not affect the ability of early (20 min) or late (4 hours) rt-PA administration to induce reperfusion (n=10 per group, results were normalised to baseline values and expressed as mean ± SD, *: $p<0.001$).

**Figure 13: Polyclonal antibodies raised against the rATD-NR1 are able to recognize the recombinant ATD domain of NR1 and the NMDA receptor NR1 subunit in cerebral parenchyma.**

(A) 20 g of recombinant N-terminal domain of NR1 (ATD-NR1, amino acids 19-371) (coupled to a Histidine-tag or an Fc-tag) was separated by SDS-PAGE prior to detection with anti-ATD-NR1 polyclonal antibody, anti-histidine antibody, anti-Fc antibody and control IGs. Immunoblot is representative of three independent experiments. (B) Protein extracts from cerebral cortices of human and mice were separated by SDS-PAGE prior to detection with anti- ATD-NR1 polyclonal antibody or a commercial NR1-ct antibody which is able to recognize the NMDA receptor NR1 subunit. Immunoblots are representative of three independent experiments.

**Figure 14: Polyclonal antibodies raised against the ATD-NR1 prevent t-PA-promoted, NMDA receptor-mediated neurotoxicity.**

Neuronal death rate was assessed by the LDH released into the bathing medium assessed 24 h after excitotoxic exposure to NMDA. (A) Mixed cortical cultures underwent a prolonged exposure to NMDA (10 μM) alone or in the presence of rt-PA (20 μg/ml), of anti-ATD-NR1 (0.01 mg/ml), or both. rt-PA caused a significant increase in the neuronal death rate, which was antagonized by anti-ATD-NR1. (C) Mixed cortical cultures with a short exposure to NMDA (50 μM), otherwise conditions and effects as in panel A. (B, D) Controls were performed in the presence of the control IGs. For each experiments N = 3; n = 12 well; $p<0.01$.

**Figure 15: Polyclonal antibodies raised against the ATD-NR1 prevent t-PA-promoted, NMDA receptor-mediated $Ca^{2+}$ influx.**

rt-PA treatment enhanced the NMDA-evoked Ca++ increase in cortical neurons. Intracellular free $Ca^{2+}$ ($[Ca^{2+}]i$) was measured using fura-2 fluorescence video microscopy. Neurons for intracellular Ca++ imaging experiments were plated on glass-bottomed 35 mm dishes. Experiments were performed on day 12 *in vitro.* (A) A 30 s exposure to 25 μM NMDA produced a rapid increase in neuronal $[Ca^{2+}]i$ which recovered over the following minutes. After a 15 min exposure to t-PA (20 μg/ml), the NMDA-evoked $Ca^{2+}$ influx was potentiated to such an extent that the net integrated increase in $[Ca^{2+}]i$ (area under the curve expressed in arbitrary units) was enhanced by 37% (N = 3; n (cell number) = 108; $p<0.05$). (B) Co-application of the anti-ATD-NR1 antibody (0.01 mg/ml) with rt-PA (20 μg/ml) completely blocked rt-PA potentiation of the NMDA-induced $Ca^{2+}$ influx (N = 3; n (cell number) = 108; $p<0.01$). (C) Co-application of control Igs (0.01 mg/ml) with rt-PA (20 μg/ml) did not cause any modification of the NMDA-evoked $Ca^{2+}$ influx (N = 3; n (cell number) = 109; $p<0.05$). (D) Quantization of the experiments illustrated in panels A-C. Area under the curve-values is normalized to the initial NMDA challenge.

**Figure 16: Antibody-based immunotherapy targeting the rATD-NR1 protects the brain against stroke and increases the therapeutic window of rt-PA-induced thrombolysis.**

(A, C) A single intravenous injection of anti-ATD-NR1 antibodies (0.8 mg/ml) against the rATD-NR1, targeting the potentiating effects of endogenous and exogenous t-PA after an in situ thromboembolic stroke, either with early (A: 20 min) or late (C: 4 hours) reperfusion by rt-PA (10 mg/kg) (filled bars), or without reperfusion (saline injection, empty bars) (n = 8-10 mice per group, $p<0.05$; * $p<0.01$). Antibodies were injected as boli after the bolus of t-PA or saline solution. (B, D) baseline values) Cerebral blood velocity measured by laser Doppler and normalised to baseline, showed that immunisation against the ATD of the NMDA receptor NR1 subunit does not affect rt-PA-induced reperfusion, implemented early (20 min, B) or late (4 hours, D) after clot onset (n = 8-10 mice per group, p<0.001).

**Figure 17: Antibody-based immunotherapy against the ATD-NR1 prevents rt-PA-induced BBB leakage after stroke.**
(A) Evans blue dye extravasation 24 hours after MCAO was normalized to mean values of sham condition (mice without clot). A single injection of anti-ATD-NR1 polyclonal antibodies (0.01 mg/ml), 20 min or 4 hr post-stroke, was able to decrease stroke-induced BBB leakage, combined or not with rt-PA (10 mg/kg) (n=3 mice per group, p<0.05). This ability could be linked with the MMP-9 activity. (B) MMP-9 proteolytic activity was normalized to mean values obtained with saline injection. A single injection of anti-ATD-NR1 polyclonal antibodies, 20 min or 4 hr post stroke, was able to decrease the MMP-9 activity in the ipsilateral cortex, combined or not with rt-PA (10 mg/kg) (n = 3 mice per group, p<0.05). Panel (C) shows representative pictures of MMP-2 and MMP-9 activities. MMP-9 was increased in saline and rt-PA conditions. Most interestingly, they were decreased after a single injection of anti-ATD-NR1 antibodies.

**Figure 18: Antibody-based immunotherapy targeting the ATD-NR1 confers long-term benefits after stroke.**
Mice were treated with saline (A) or anti-ATD-NR1 antibody (0.8 mg/ml) (B), 4 hours after stroke. 24, 72 hours, 7 days and 15 days post ischaemia, they were placed in a 7T MRI (Pharmascan Brucker) for T2 brain imaging and apparent diffusion coefficient (ADC) at 24 hours. 3 animals par group. Representative images are shown including 4 different brain sections.

**Figure 19: Antibody-based immunotherapy targeting the ATD-NR1 improves long-term cognitive recovery after stroke.**
Mice were treated with saline or anti-ATD-NR1 antibody (0.8 mg/ml), 20 minutes (A) or 4 hours after stroke (B). 24 hr post ischaemia, they were placed in a fear conditioning room. Results are expressed in percentage of freezing time during a 5 min period and normalized to results obtained with naive mice (n = 10 per group, * p<0.025; $ p<0.05).

**Figure 20: Antibodies against the rATD-NR1 prevent the neurotoxic effect of endogenous t-PA in an excitotoxic lesion model.**
Excitotoxic lesions were produced by injecting NMDA (10 nmol) into the right striatum (coordinates: 0.0 mm posterior, 2.0 mm lateral, 4.0 mm ventral to the bregma) After 30 minutes, anti-ATD-NR1 antibodies or control IGs were injected in the tail vein (0.8 mg/ml). Compared to controls, anti-ATD-NR1 antibodies afforded a significant protection of the striatum (n = 8, p<0.01)

**Figure 21: Antibodies against the rATD-NR1 bind specifically to the human NR1 subunit of the NMDA receptor.**
Protein extracts were isolated post mortem from the ipsilateral and contralateral hemisphere of a patient died two days after ischemic stroke, subjected to SDS-PAGE gel chromatography, blotted onto a nylon membrane and analysed in a Western blot using (A) the anti-ATD NR1 antibody or (B) the anti-human NR1 antibody.

**Figure 22: Antibodies against the rATD-NR1 reach the brain of non-operated animals.**
(A) XX, (B) XX. (C) Immunohistochemical analysis directed against the anti-ATD-NR1 antibody using brain tissue from non-operated or sham animals reveal the presence of anti-ATD-NR1 antibodies in the brain parenchyma.

**Figure 23: Polyclonal antibodies raised against the ATD-NR1 do not influence the ability of t-PA to antagonize neuronal apoptosis induced by serum deprivation.**
Tested was the influence of rt-PA (20μg/mL) and anti-ATD-NR1 antibodies (0.01 mg/ml) on apoptosis caused by serum deprivation of neuronal cultures (DIV7). **Upper panels**: Neuronal death caused by serum deprivation, ability of rt-PA to antagonize this effect (p < 0.01), and lack of influence of anti-ATD-NR1 antibodies on the effect of rt-PA. The percentage of neuronal death was determined by the proportion of trypan blue positive neurons (counting trypan blue positive cells in three random fields per well). Results were corrected for the respective values obtained in sham washed control neurons.
**Lower panel**: Cleavage of caspase 3 as an indicator of an apoptotic setting. Cleaved molecules were revealed with an antibody raised against the cleaved caspase-3. Again, the protective effect of rt-PA was not influenced by the anti-ATD-NR1 antibodies.
(N=3, n=12)

## Claims

1. Use of a protein or peptide for the manufacture of a medicament for the treatment of neurological or neurodegenerative disorders, in particular stroke, wherein the protein or peptide is selected form the group consisting of

   (a) A kringle protein or peptide comprising or consisting of

      (i) an amino acid sequence according SEQ ID NO:1, SEQ ID NO:2 or SEQ ID NO:3; or
      (ii) an amino acid sequence with at least 70% identity, preferably 80%, more preferably 90% and most preferably 95% identity to the amino acid sequence given in SEQ ID NO:3; or
      (iii) an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES- $X_3$-C- $X_2$-WNS- $X_2$-L- $X_4$-Y- $X_4$-PXA- $X_2$-LGLGXHNYCRNP- $X_4$-KPWCXVXK- $X_6$-EXC- $X_2$-PXC", wherein X denotes an arbitrary amino acid; or
      (iv) an amino acid sequence with at least 92% identity, preferable at least 95% or 100% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNXAXKPWCXVXK-$X_3$-F-$X_2$.ESC-$X_2$-PXC", wherein X denotes an arbitrary amino acid

   wherein said protein or peptide does not exhibit a serine protease activity; or
   (b) an isolated antibody or fragment thereof which binds to the N-terminal domain of the NMDA receptor subunit NR1 (anti-ATD-NR1 antibody), whereas the binding of the antibody or the fragment thereof prevents the cleavage of the extracellular domain of the NR1 subunit, or the fragment, whereas the NR1 preferably

      (i) has the amino acid sequence SEQ ID NO: 4 or 5,
      (ii) is encoded by the nucleotide sequences SEQ ID NO: 6 or 7
      (iii) nucleic acid molecule that specifically hybridizes to the complement of the nucleic acid molecule of SEQ ID NO: 6 or 7 under conditions of high stringency, where the hybridisation is performed in 5x SSPE, 5 x Denhardt's solution and 0.5% SDS overnight at 55 to 60°C.

2. Use according to claim 1 in combination with a medicament comprising a thrombolytic drug, preferably a plasminogen activator.

**3.** Use according to claim 2, wherein a therapy using a thrombolytic drug comprises one or more of the following characteristics:

(a) the thrombolytic drug, in particular a plasminogen activator, can be administered to the patient more than 3, preferably more than 4.5 hours, preferably more than 6 hours and even more than 9 or 12 hours later than the stroke onset;
(b) the dose of the thrombolytic drug, in particular t-PA, for the treatment can be increased in comparison to the dose that is recommended for the monotherapy with said thrombolytic drug;
(c) the thrombolytic treatment can be applied in patients that are currently not eligible for a thrombolytic therapy, namely patients which suffered the stroke earlier than 3, more preferably earlier than 4,5 hours before presented for the possible treatment or patients with an increased bleeding risk.

**4.** Use according to claim 2 or 3, wherein the thrombolytic drug is a plasminogen activator, more preferably recombinant t-PA (rt-PA, e.g. Alteplase) or variants of t-PA such as Pamiteplase, Lanoteplase, Reteplase, Tenecteplase or Monteplase, urokinase or DSPA variants such as DSPA alpha 1, DSPA alpha 2, DSPA beta or DSPA gamma.

**5.** Use according to one of the above claims, wherein the protein or peptide according to claim 1 (a) is an inactivated plasminogen activator. preferably selected from the group consisting of recombinant t-PA (rt-PA, e.g. Alteplase) or variants of t-PA such as Pamiteplase, Lanoteplase, Reteplase, Tenecteplase or Monteplase), urokinase or DSPA variants such as DSPA alpha 2, DSPA beta or DSPA gamma.

**6.** Use according to one of the above claims, wherein the protein or peptide according to claim 1(a) is the inactivated DSPA alpha 1, which are preferably linked to a suicide substrate, more preferably to D-phenyl-prolyl-arginine chloromethyl ketone (PPACK).

**7.** Use according to one of the above claims, wherein the protein or peptide according to claim 1(a) includes one or more of further domains found in plasminogen activators, such as the finger domain (F) or the epidermal growth factor domain (EGF), preferably from t-PA or desmoteplase.

**8.** Use according to one of the above claims, wherein the protein or peptide according to claim 1 (a) consists only of the kringle domain, or a fragment thereof.

**9.** An isolated kringle protein or peptide comprising or consisting of:

(a) an amino acid sequence according SEQ ID NO:3 (DSPA kringle); or
(b) an amino acid sequence with at least 70% identity, preferably 80%, more preferably 90% and most preferable 95% identity to the amino acid sequence given in SEQ ID NO:3; or
(c) an amino acid sequence with at least 92% identity, preferable at least 95% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNY-CRNPNXAXKPWCXVXK-$X_3$-F-$X_{2}$-ESC-$X_2$-PXC", wherein X denotes an arbitrary amino acid;

wherein the protein or peptide does not exhibit a serine protease activity and with the proviso that said protein or peptide is not the kringle domain of t-PA or urokinase, and not the DSPA alpha 1 protein or t-PA covalently linked to a suicide substrate.

**10.** An isolated antibody or fragment thereof which binds to the N-terminal domain of the NMDA receptor subunit NR1 (anti-ATD-NR1 antibody), whereas the binding of the antibody or the fragment thereof prevents the cleavage of the extracellular domain of the NR1 subunit, or the fragment, whereas the NR1 preferably

(a) has the amino acid sequence SEQ ID NO: 4 or 5,
(b) is encoded by the nucleotide sequences SEQ ID NO: 6 or 7
(c) nucleic acid molecule that specifically hybridizes to the complement of the nucleic acid molecule of SEQ ID NO: 6 or 7 under conditions of high stringency, where the hybridisation is performed in 5x SSPE, 5 x Denhardt's solution and 0.5% SDS overnight at 55 to 60°C.

**11.** Use of a protein or peptide as a neuroprotectant, wherein the protein or peptide is selected form the group consisting of

(a) A kringle protein or peptide comprising or consisting of

(i) an amino acid sequence according SEQ ID NO:1, SEQ ID NO:2 or SEQ ID NO:3; or

(ii) an amino acid sequence with at least 70% identity, preferably 80%, more preferably 90% and most preferably 95% identity to the amino acid sequence given in SEQ ID NO:3; or

(v) an amino acid sequence with at least 90% identity, preferable at least 95% or 100% identity to the plasminogen activator-related kringle (PARK) motif defined by the sequence: "CY-$X_3$-G-$X_2$-YRGTXS-$X_2$-ES- $X_3$-C- $X_2$-WNS- $X_2$-L- $X_4$-Y- $X_4$-PXA- $X_2$-LGLGXHNYCRNP- $X_4$-KPWCXVXK- $X_6$-EXC- $X_2$-PXC", wherein X denotes an arbitrary amino acid; or

(vi) an amino acid sequence with at least 92% identity, preferable at least 95% or 100% identity to the DARK motif defined by the sequence: "CY-$X_3$-G-$X_2$-YRGTXS-$X_2$-ESR-$X_2$-C-$X_2$-WNS-$X_2$-LXR-$X_2$-Y-$X_3$-MPXAFN-LGLGXHNYCRNPNXAXKPWCXVXK-$X_3$-F-X2-ESC-$X_2$-PXC", wherein X denotes an arbitrary amino acid

wherein said protein or peptide does not exhibit a serine protease activity; or

(b) an isolated antibody or fragment thereof which binds to the N-terminal domain of the NMDA receptor subunit NR1 (anti-ATD-NR1 antibody), whereas the binding of the antibody or the fragment thereof prevents the cleavage of the extracellular domain of the NR1 subunit, or the fragment, whereas the NR1 preferably

(i) has the amino acid sequence SEQ ID NO: 4 or 5,

(ii) is encoded by the nucleotide sequences SEQ ID NO: 6 or 7

(iii) nucleic acid molecule that specifically hybridizes to the complement of the nucleic acid molecule of SEQ ID NO: 6 or 7 under conditions of high stringency, where the hybridisation is performed in 5x SSPE, 5 x Denhardt's solution and 0.5% SDS overnight at 55 to 60°C.

**12.** A pharmaceutical composition selected from the group consisting of:

(a) An protein or peptide according to claim 1(a) and, or

(b) An isolated antibody or an antigen-binding portion according to claim 10; together with or without a thrombolytic drug,

wherein said pharmaceutical composition may further comprise one or more pharmaceutically acceptable carriers or excipients.

**13.** Method of treating a neurological or neurodegenerative disorder, in particular stroke, comprising administering a therapeutically effective amount of an isolated protein or peptide according to claim 1(a) or an isolated antibody or antigen-binding portion thereof according to claim 10, with or without a therapeutically amount of a thrombotic drug, preferably a plasminogen activator, more preferably t-PA, according to any of the above claims.

## Figure 1

**A**

K2 domain of rt-PA | K1 domain of rt-PA | Kringle of DSPA alpha 1

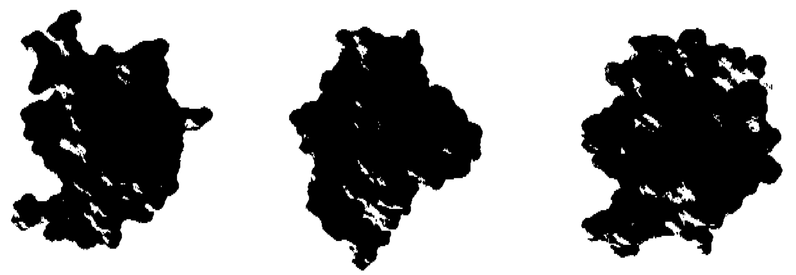

The lower pictures represent the corresponding kringle domains rotated by 180°

K2 domain of rt-PA | K1 domain of rt-PA | Kringle of DSPA alpha 1

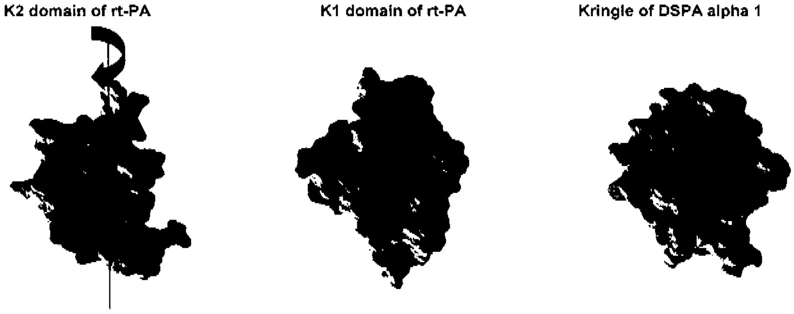

**B**

```
htPA__K1  CYEDQGISYRGTWSTAESGAECTNWNSSALAQKPYSGRRPDAIRLGLGNHNYCRNPDRDS 60
DSPA_K    CYEGQGVTYRGTWSTAESRVECINWNSSLLTRRTYNGRMPDAFNLGLGNHNYCRNPNGAP 60
htPA__K2  CYFGNGSAYRGTHSLTESGASCLPWNSMILIGKVYTAQNPSAQALGLGKHNYCRNPDGDA 60
          ** .:* :**** * :** ..*  ***  *  : *..: *.*  ****:*******:  .

htPA__K1  KPWCYVFKAGKYSSEFCSTPAC 82
DSPA_K    KPWCYVIKAGKFTSESCSVPVC 82
htPA__K2  KPWCHVLKNRRLTWEYCDVPSC 82
          ****:*:*  : : * *..* *
```

An asterisk denotes an amino acid that is conserved in all three PARK domains..

## Figure 2

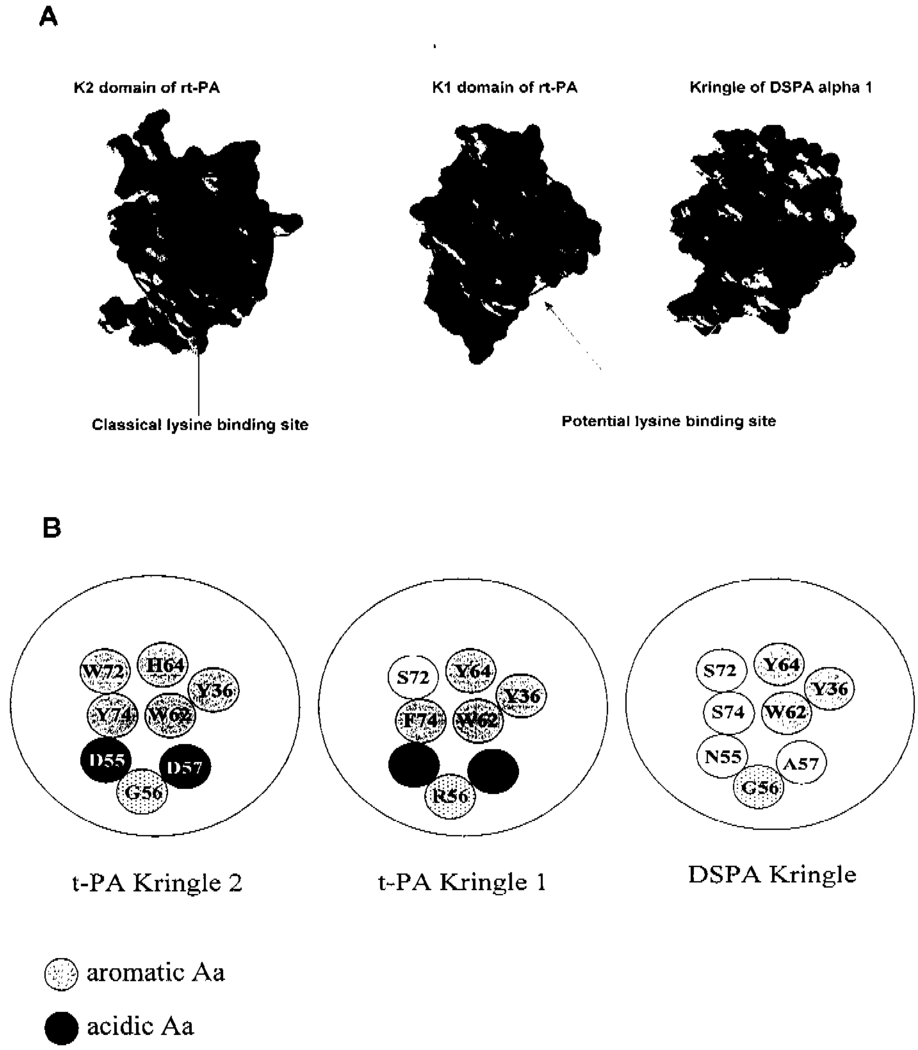
A
K2 domain of rt-PA
K1 domain of rt-PA
Kringle of DSPA alpha 1
Classical lysine binding site
Potential lysine binding site
B
W72
H64
Y36
Y74
W62
D55
D57
G56
S72
Y64
Y36
F74
W62
R56
S72
Y64
Y36
S74
W62
N55
A57
G56
t-PA Kringle 2
t-PA Kringle 1
DSPA Kringle
aromatic Aa
acidic Aa

Figure 3

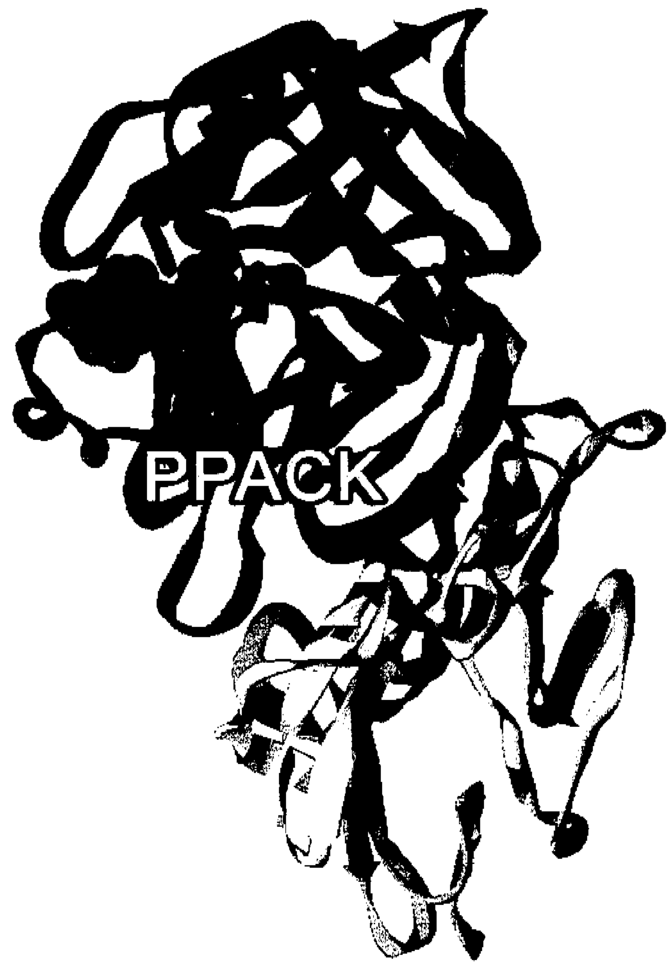

**Figure 4**

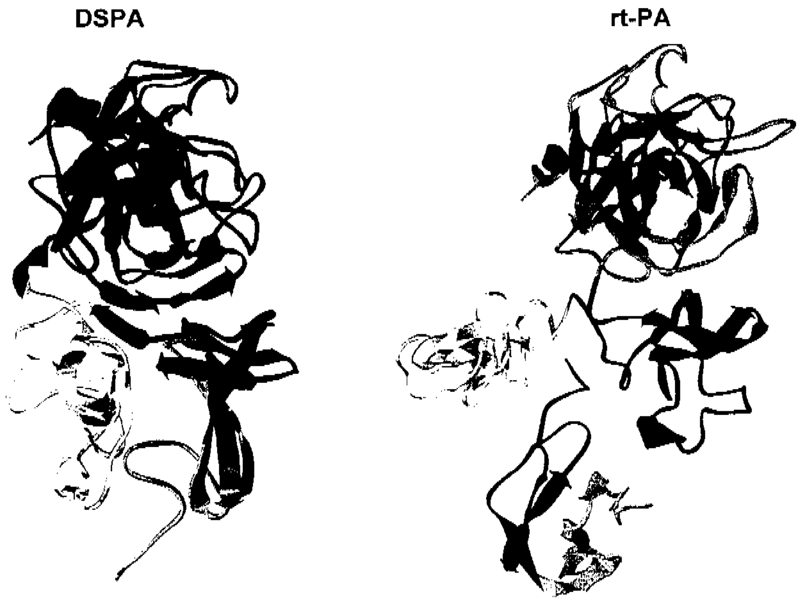

**Figure 5**

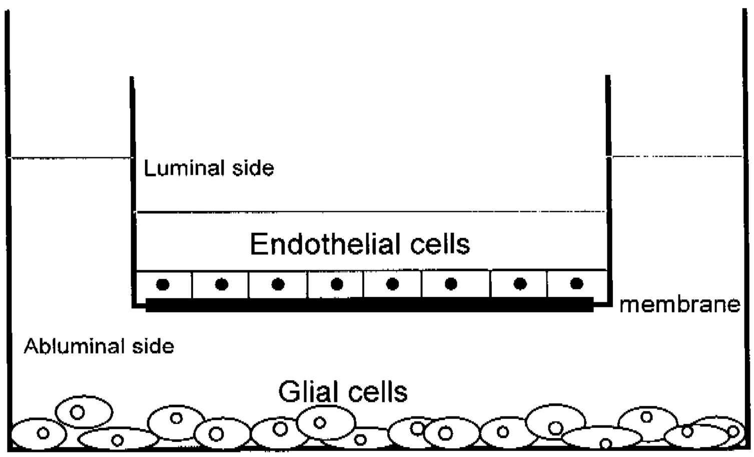
Luminal side
Endothelial cells
membrane
Abluminal side
Glial cells

Figure 6

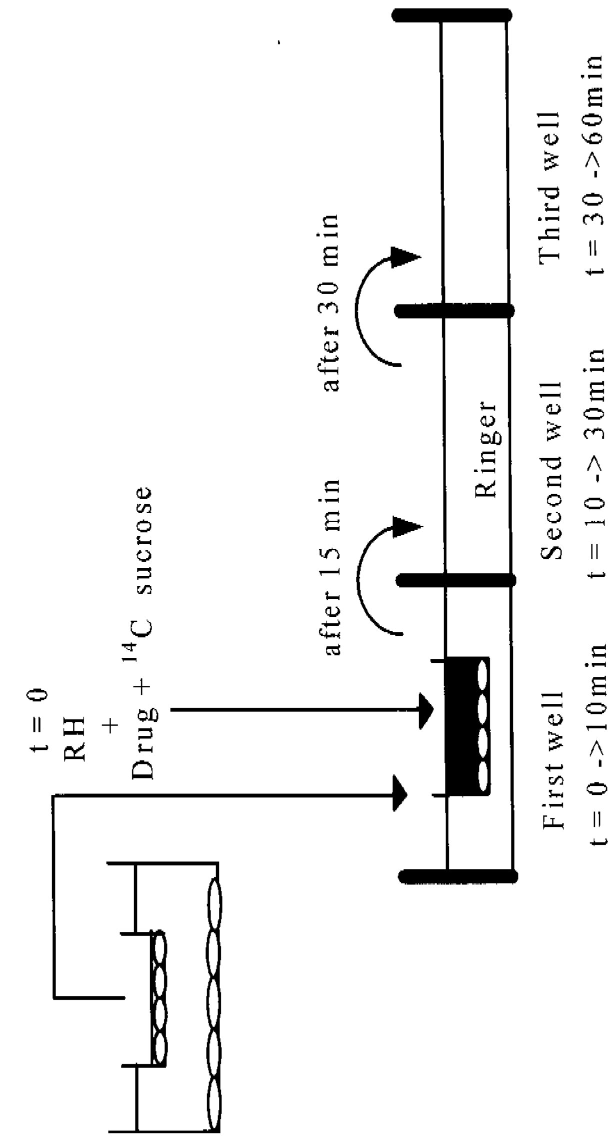
t = 0
RH
+
Drug + $^{14}C$ sucrose
after 15 min
after 30 min
Ringer
First well
t = 0 ->10min
Second well
t = 10 -> 30min
Third well
t = 30 ->60min

**Figure 7**

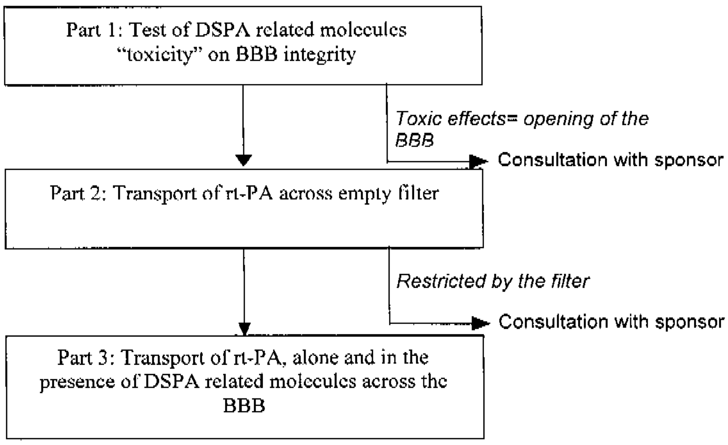
Part 1: Test of DSPA related molecules "toxicity" on BBB integrity
Toxic effects= opening of the BBB
Consultation with sponsor
Part 2: Transport of rt-PA across empty filter
Restricted by the filter
Consultation with sponsor
Part 3: Transport of rt-PA, alone and in the presence of DSPA related molecules across the BBB

**Figure 8**

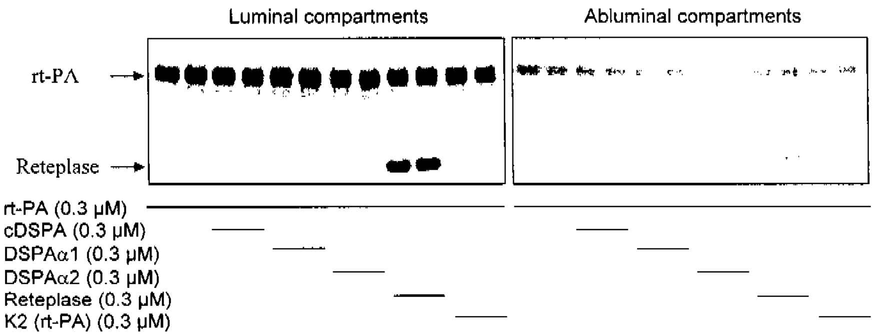
Luminal compartments
Abluminal compartments
rt-PA
Reteplase
rt-PA (0.3 µM)
cDSPA (0.3 µM)
DSPAα1 (0.3 µM)
DSPAα2 (0.3 µM)
Reteplase (0.3 µM)
K2 (rt-PA) (0.3 µM)

Figure 9

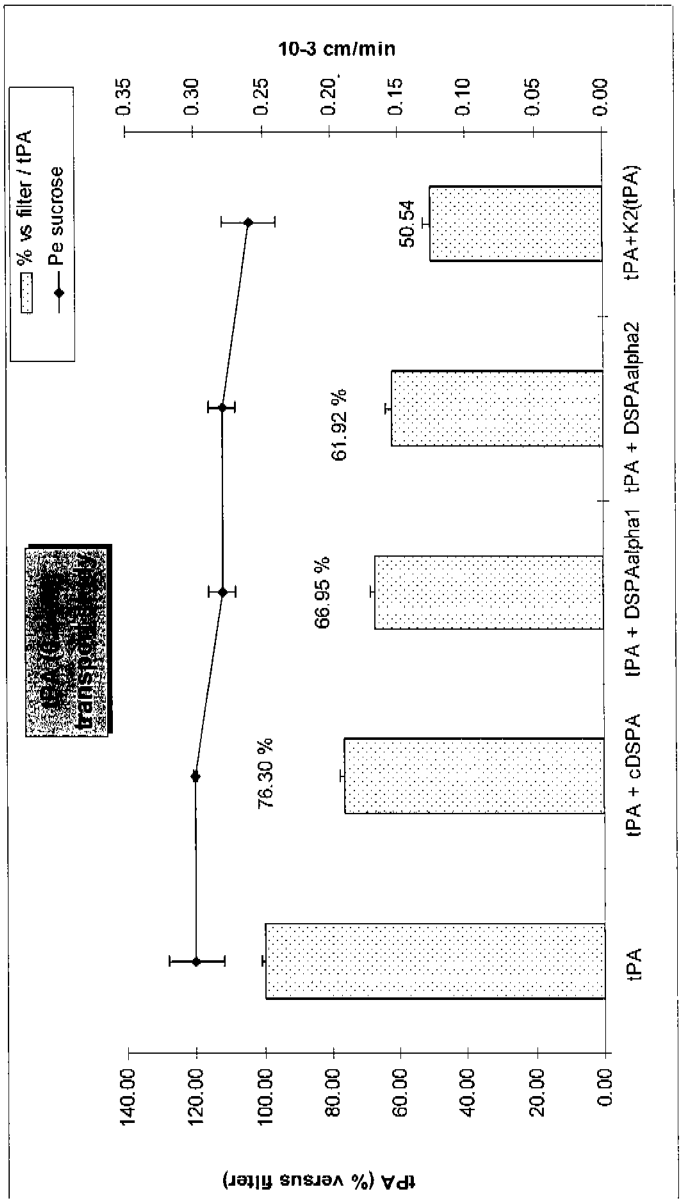
% vs filter / tPA
Pe sucrose
10-3 cm/min
0.35
0.30
0.25
0.20
0.15
0.10
0.05
0.00
tPA (% versus filter)
140.00
120.00
100.00
80.00
60.00
40.00
20.00
0.00
76.30 %
66.95 %
61.92 %
50.54
tPA
tPA + cDSPA
tPA + DSPAalpha1
tPA + DSPAalpha2
tPA+K2(tPA)

Figure 10

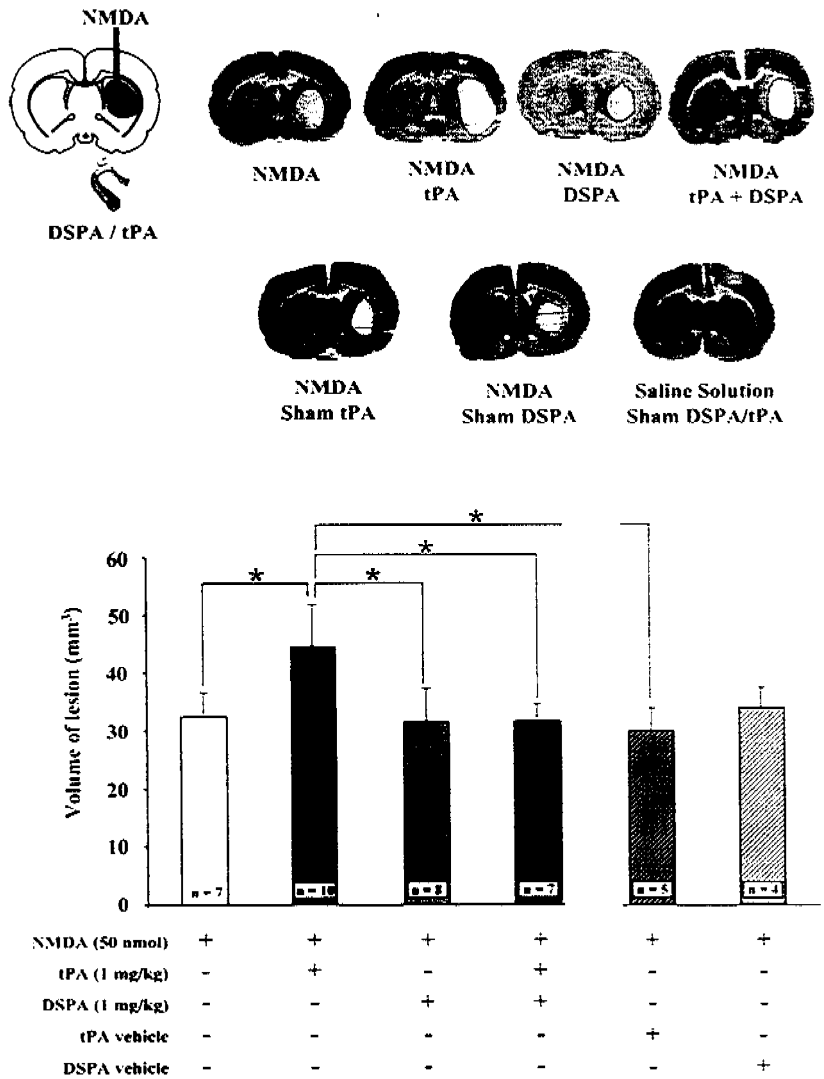
NMDA
DSPA / tPA
NMDA
NMDA
tPA
NMDA
DSPA
NMDA
tPA + DSPA
NMDA
Sham tPA
NMDA
Sham DSPA
Saline Solution
Sham DSPA/tPA
Volume of lesion (mm³)
60
50
40
30
20
10
0
*
*
*
*
n = 7
n = 10
n = 8
n = 7
n = 5
n = 4
NMDA (50 nmol) + + + + + +
tPA (1 mg/kg) - + - + - -
DSPA (1 mg/kg) - - + + - -
tPA vehicle - - - - + -
DSPA vehicle - - - - - +

Figure 11

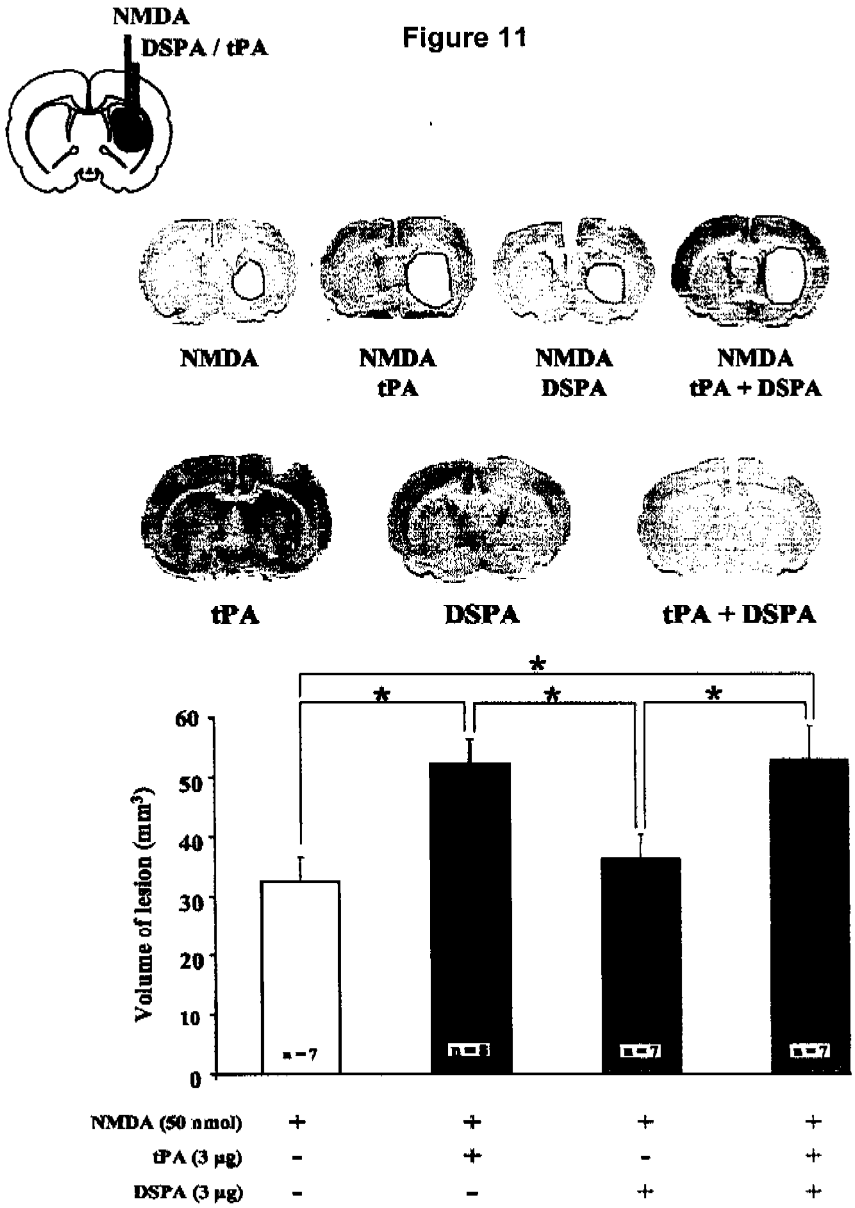
NMDA
DSPA / tPA
NMDA
NMDA
tPA
NMDA
DSPA
NMDA
tPA + DSPA
tPA
DSPA
tPA + DSPA
*
*
*
*
Volume of lesion (mm³)
60
50
40
30
20
10
0
n=7
NMDA (50 nmol) + + + +
tPA (3 µg) - + - +
DSPA (3 µg) - - + +

Figure 12

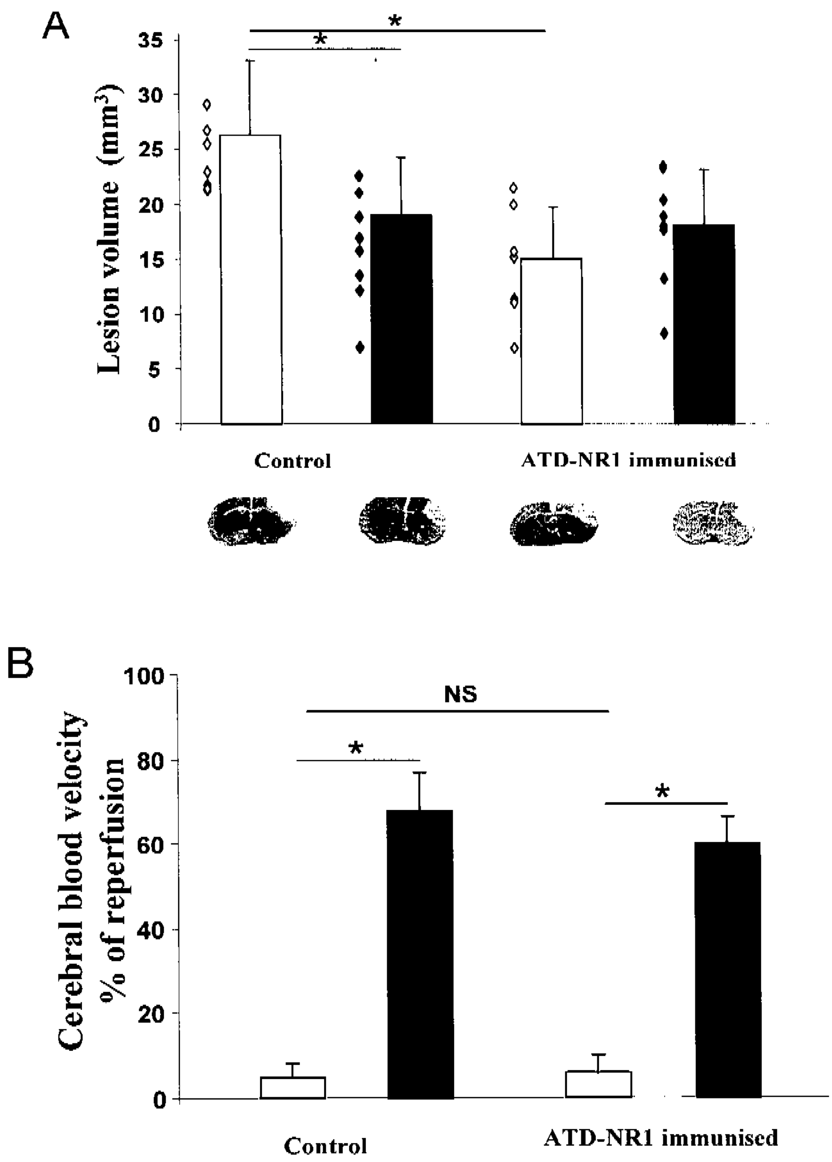
A
35
30
25
20
15
10
5
0
Lesion volume (mm³)
*
*
Control
ATD-NR1 immunised
B
100
80
60
40
20
0
Cerebral blood velocity
% of reperfusion
NS
*
*
Control
ATD-NR1 immunised

Figure 12

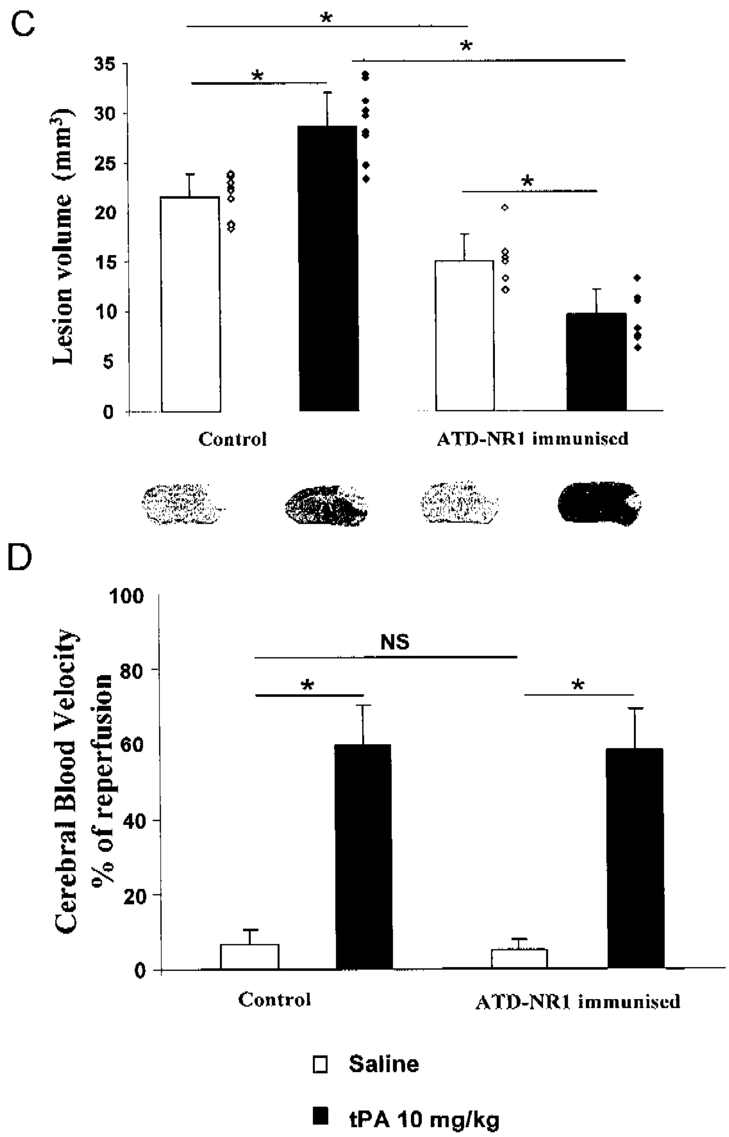
C
*
*
*
*
35
30
25
20
15
10
5
0
Lesion volume (mm³)
Control
ATD-NR1 immunised
D
100
80
60
40
20
0
Cerebral Blood Velocity
% of reperfusion
NS
*
*
Control
ATD-NR1 immunised
Saline
tPA 10 mg/kg

Figure 13

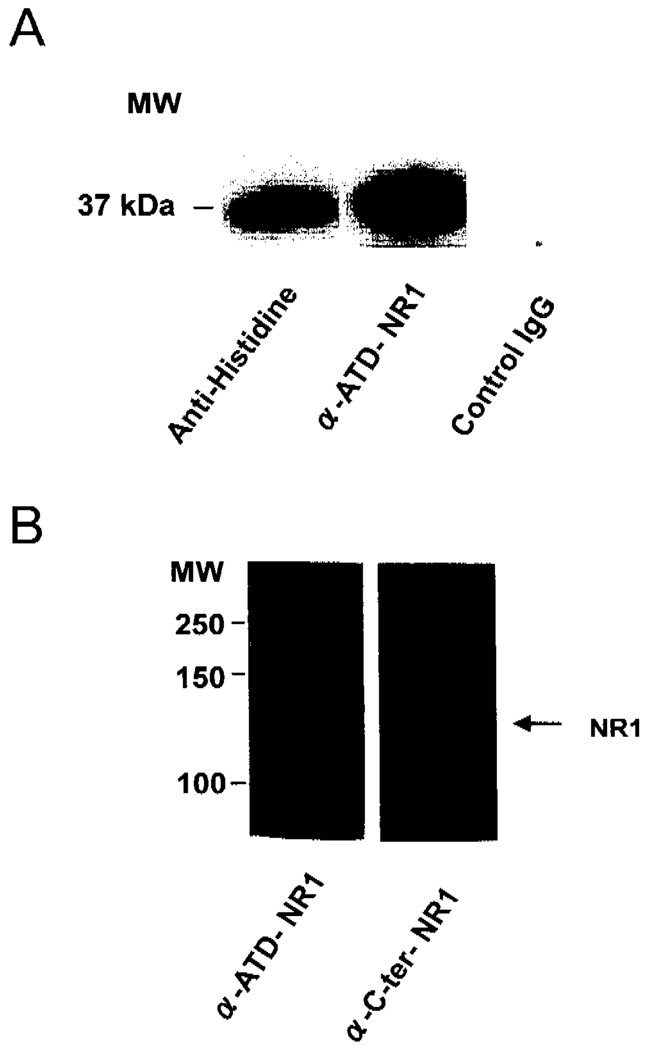
A
MW
37 kDa
Anti-Histidine
α-ATD- NR1
Control IgG
B
MW
250
150
100
NR1
α-ATD- NR1
α-C-ter- NR1

Figure 14

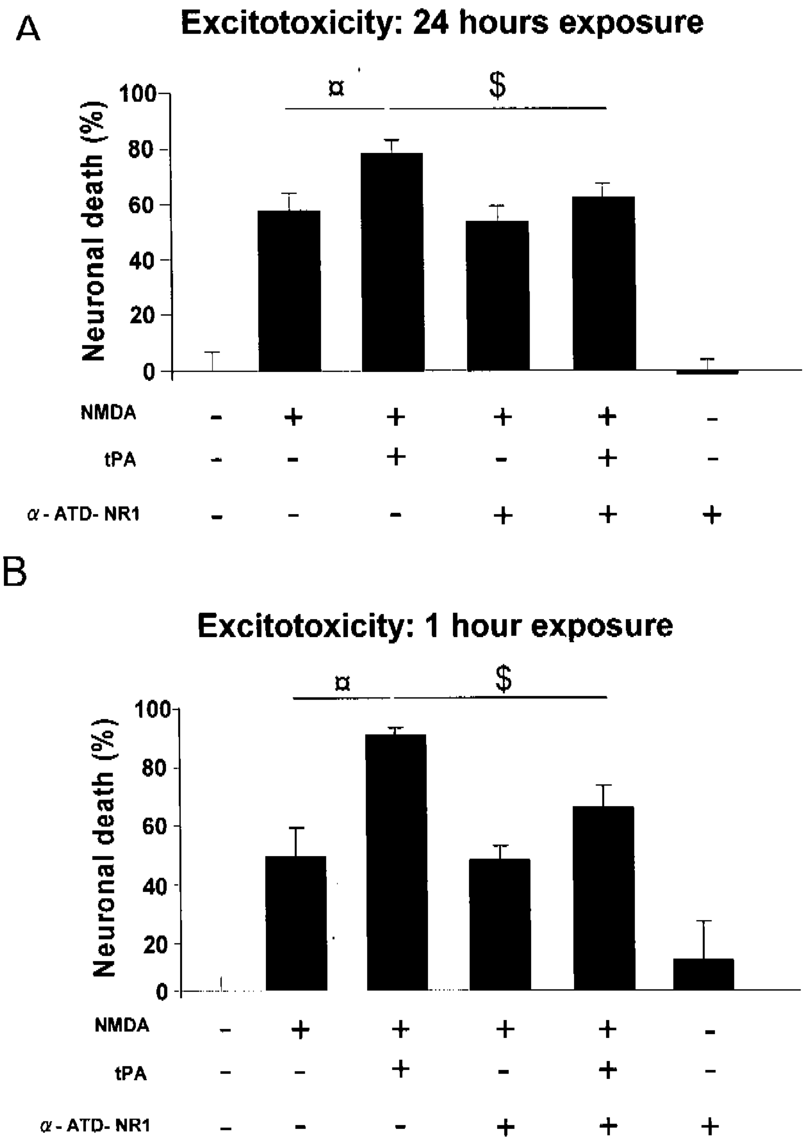
A
Excitotoxicity: 24 hours exposure
¤
$
Neuronal death (%)
100
80
60
40
20
0
NMDA - + + + + -
tPA - - + - + -
α - ATD- NR1 - - - + + +
B
Excitotoxicity: 1 hour exposure
¤
$
Neuronal death (%)
100
80
60
40
20
0
NMDA - + + + + -
tPA - - + - + -
α - ATD- NR1 - - - + + +

Figure 14

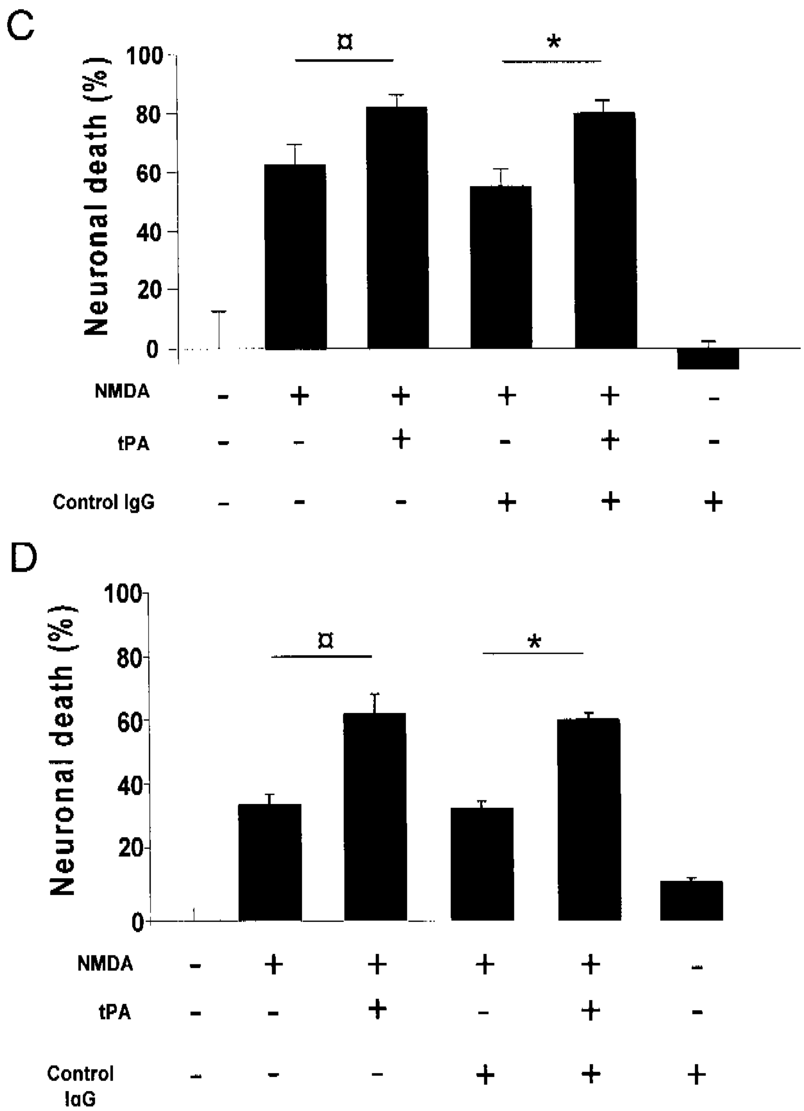
C
Neuronal death (%)
100
80
60
40
20
0
¤
*
NMDA - + + + + -
tPA - - + - + -
Control IgG - - - + + +
D
Neuronal death (%)
100
80
60
40
20
0
¤
*
NMDA - + + + + -
tPA - - + - + -
Control IgG - - - + + +

Figure 15

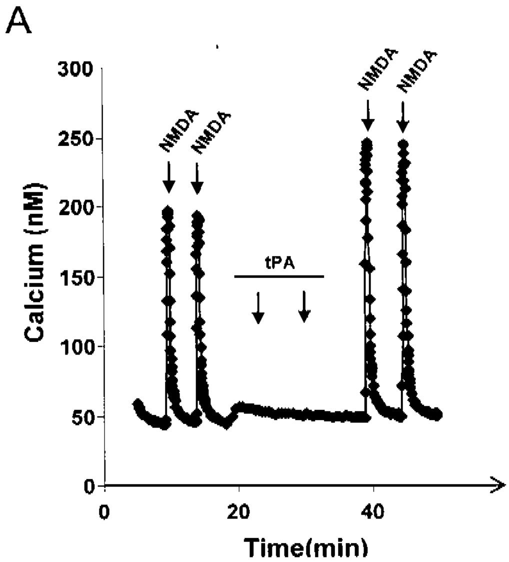
A
300
250
200
150
100
50
0
Calcium (nM)
NMDA
NMDA
NMDA
NMDA
tPA
0
20
40
Time(min)

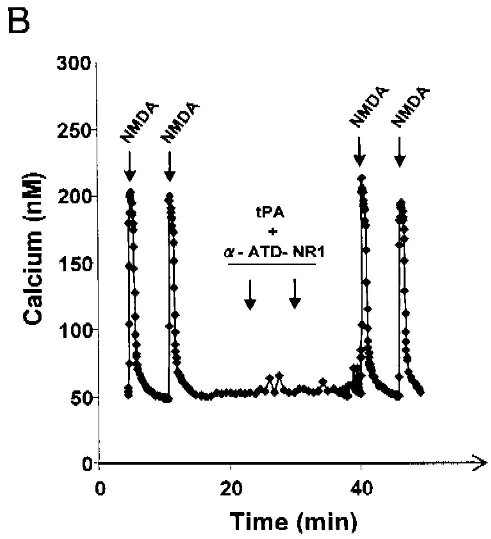
B
300
250
200
150
100
50
0
Calcium (nM)
NMDA
NMDA
NMDA
NMDA
tPA
+
α - ATD- NR1
0
20
40
Time (min)

Figure 15

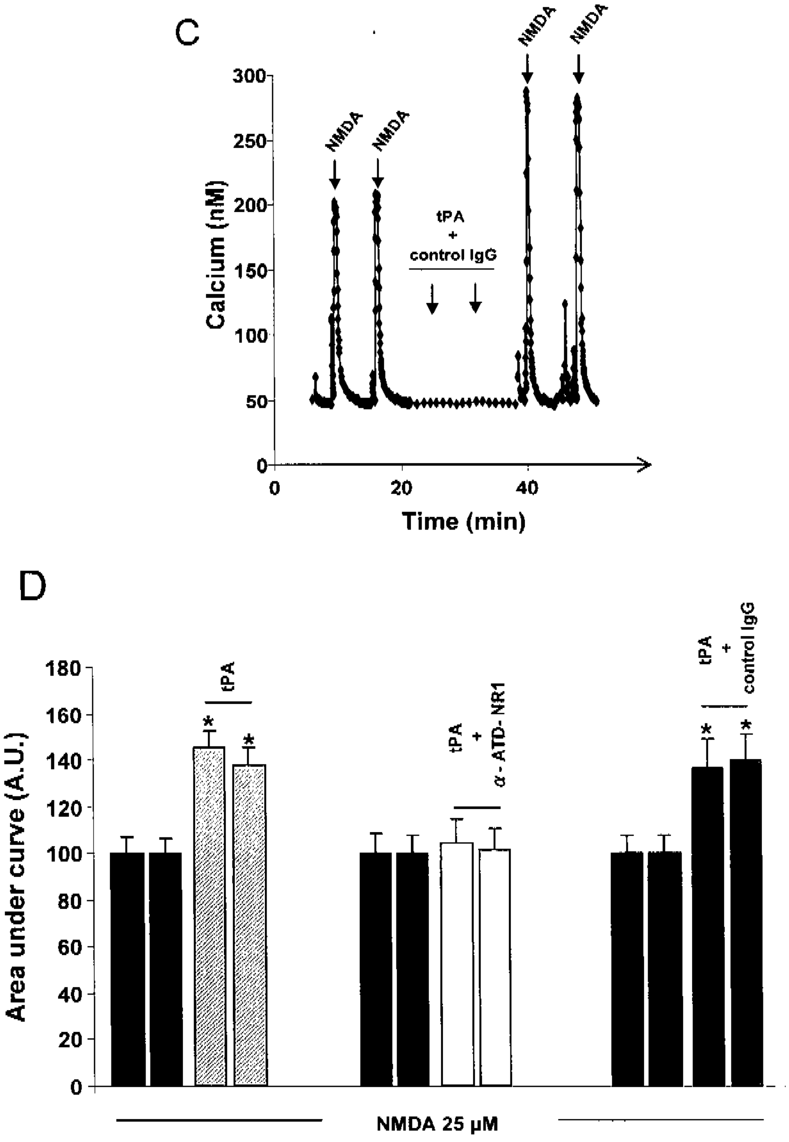
C
NMDA
NMDA
NMDA
NMDA
tPA
+
control IgG
Calcium (nM)
300
250
200
150
100
50
0
0
20
40
Time (min)
D
Area under curve (A.U.)
180
160
140
120
100
80
60
40
20
0
tPA
tPA
+
α - ATD- NR1
tPA
+
control IgG
NMDA 25 μM

Figure 16

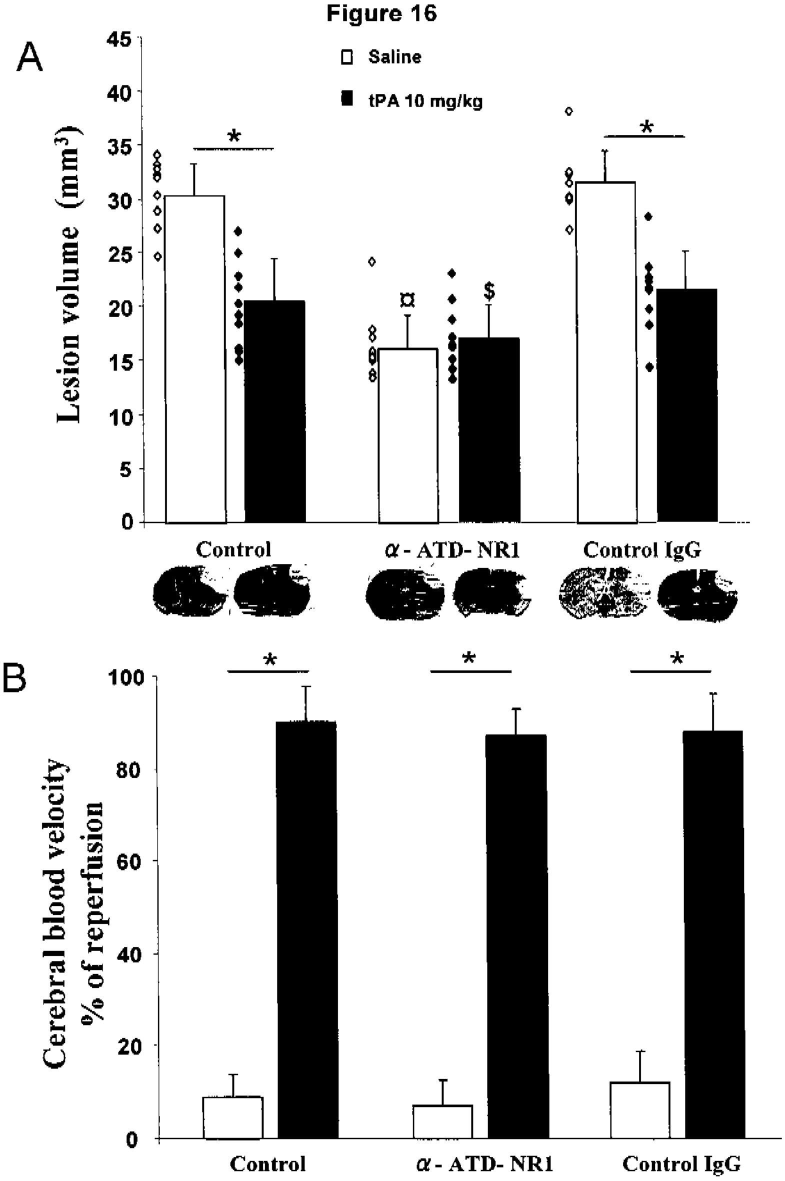
A
Saline
tPA 10 mg/kg
Lesion volume (mm³)
0
5
10
15
20
25
30
35
40
45
*
¤
$
Control
α - ATD- NR1
Control IgG
B
Cerebral blood velocity
% of reperfusion
0
20
40
60
80
100
Control
α - ATD- NR1
Control IgG

Figure 16

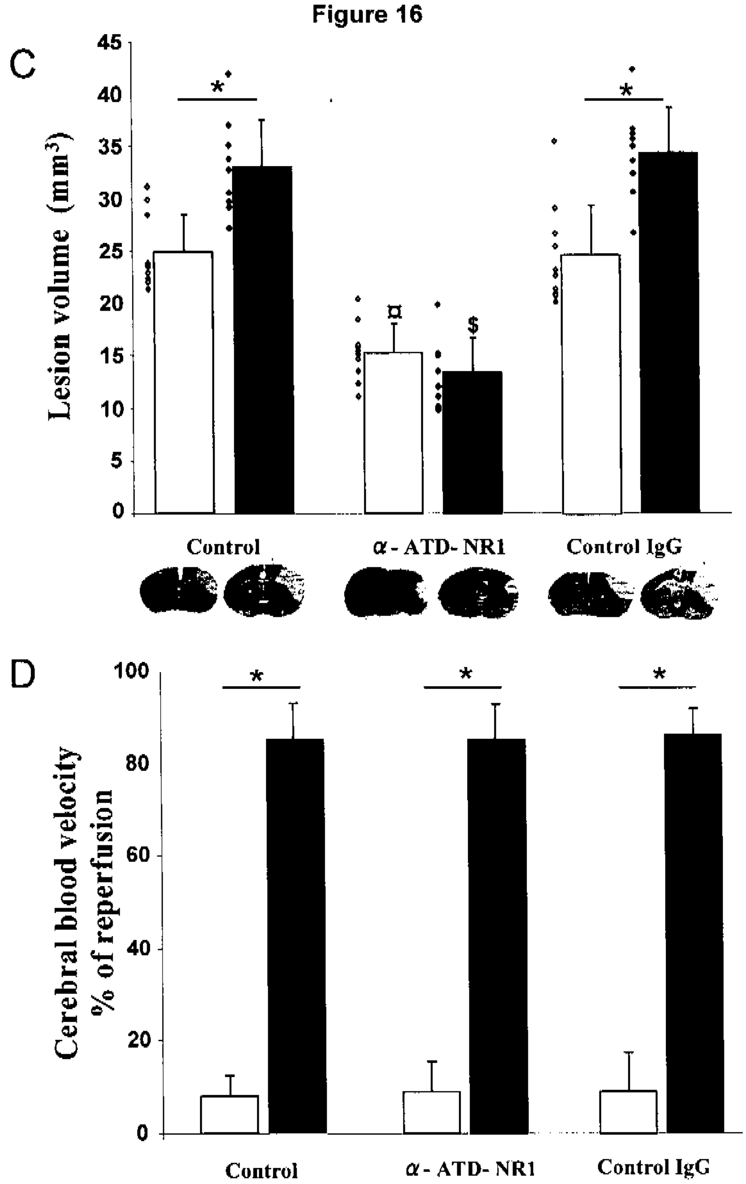
C
Lesion volume (mm³)
45
40
35
30
25
20
15
10
5
0
*
*
¤
$
Control
α - ATD- NR1
Control IgG
D
Cerebral blood velocity
% of reperfusion
100
80
60
40
20
0
*
*
*
Control
α - ATD- NR1
Control IgG

Figure 17

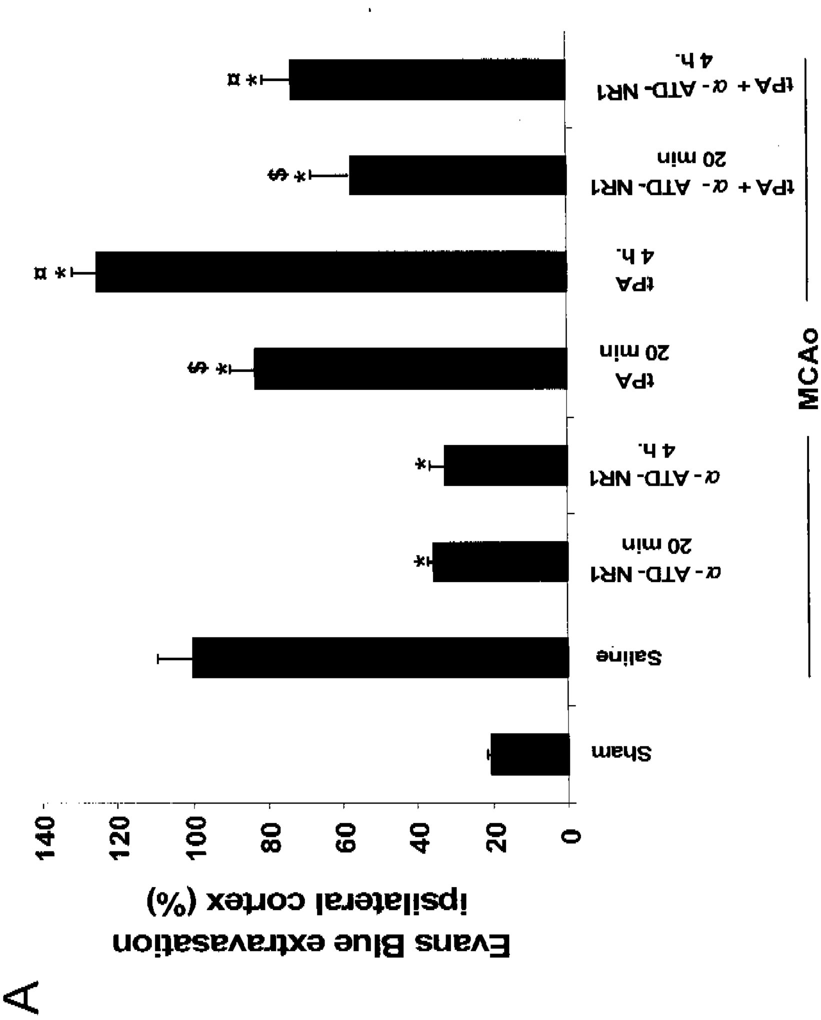
A
Evans Blue extravasation
ipsilateral cortex (%)
140
120
100
80
60
40
20
0
Sham
Saline
α-ATD-NR1
20 min
α-ATD-NR1
4 h.
tPA
20 min
tPA
4 h.
tPA + α- ATD-NR1
20 min
tPA + α- ATD-NR1
4 h.
MCAo

Figure 17

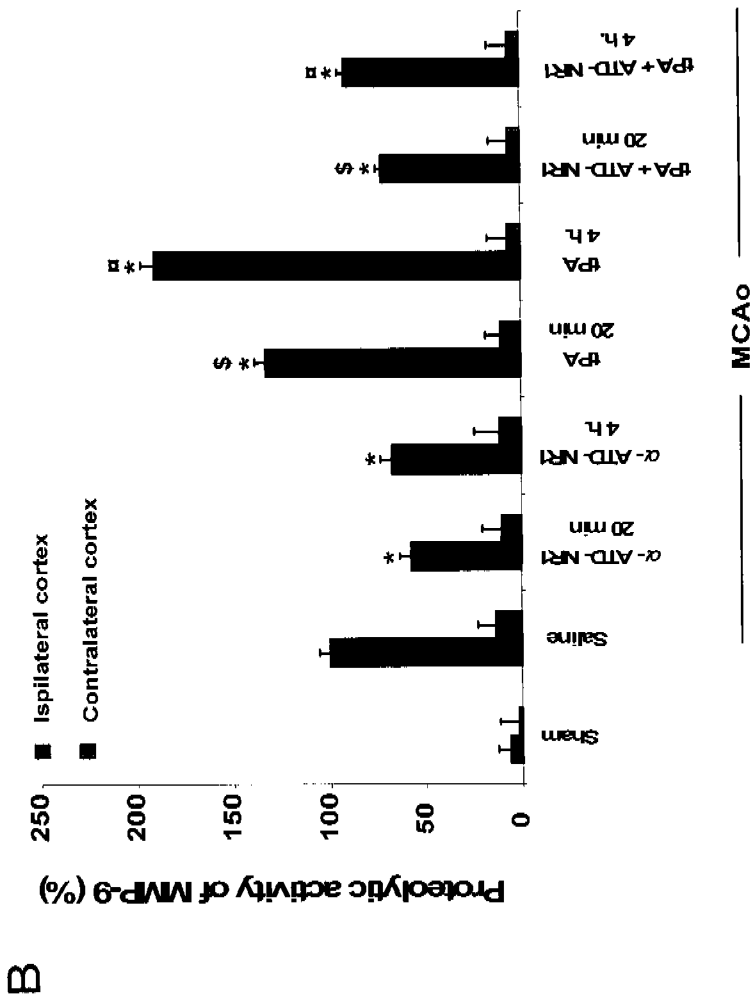
B
Proteolytic activity of MMP-9 (%)
250
200
150
100
50
0
Ispilateral cortex
Contralateral cortex
Sham
Saline
α- ATD- NR1 20 min
α- ATD- NR1 4 h.
tPA 20 min
tPA 4 h.
tPA + ATD- NR1 20 min
tPA + ATD- NR1 4 h.
MCAo

Figure 17

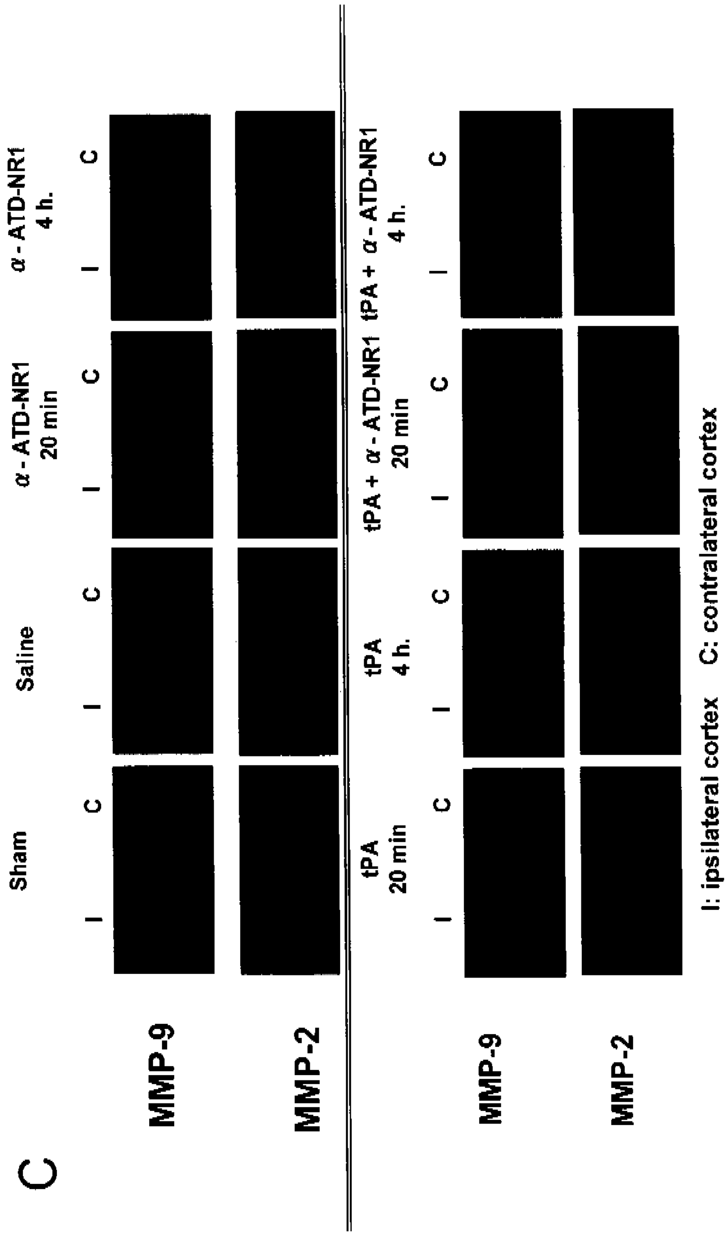
C
Sham
Saline
α - ATD-NR1 20 min
α - ATD-NR1 4 h.
I C I C I C I C
MMP-9
MMP-2
tPA 20 min
tPA 4 h.
tPA + α - ATD-NR1 20 min
tPA + α - ATD-NR1 4 h.
I C I C I C I C
MMP-9
MMP-2
I: ipsilateral cortex C: contralateral cortex

# Figure 18A

$\alpha$ - ATD- NR1 (4 hours after clot formation)

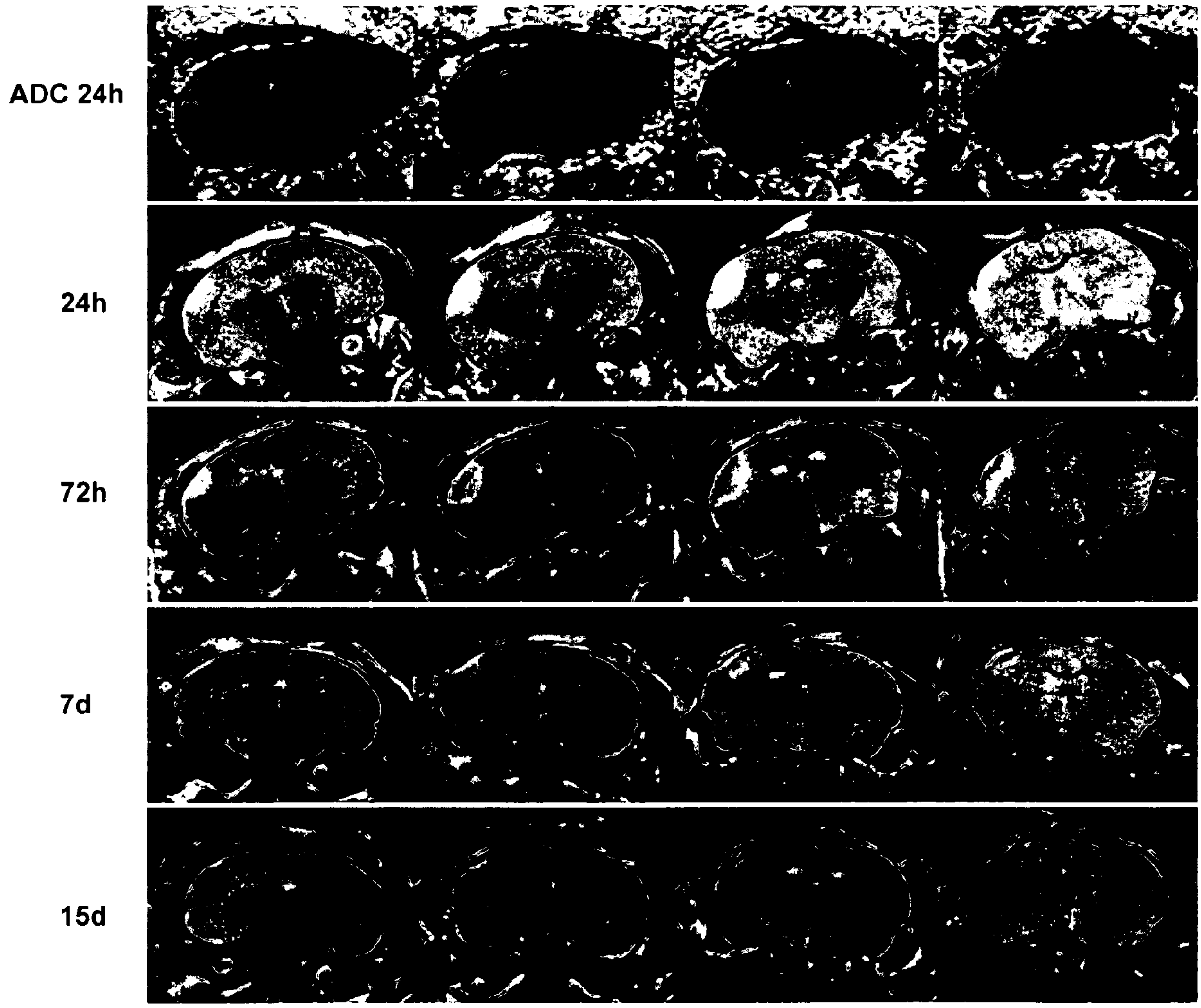

# Figure 18B

Saline (4 hours after clot formation)

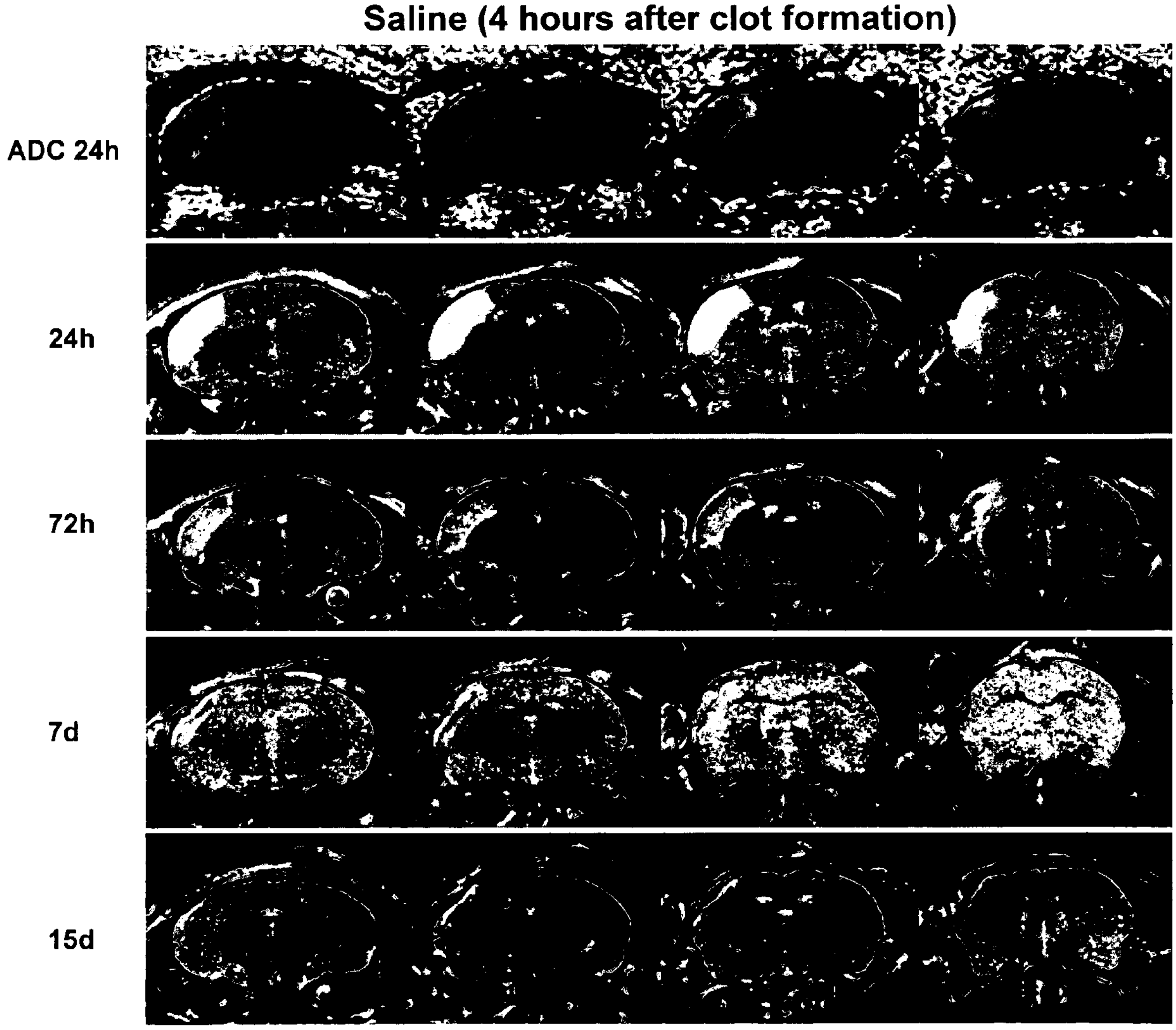

# Figure 19

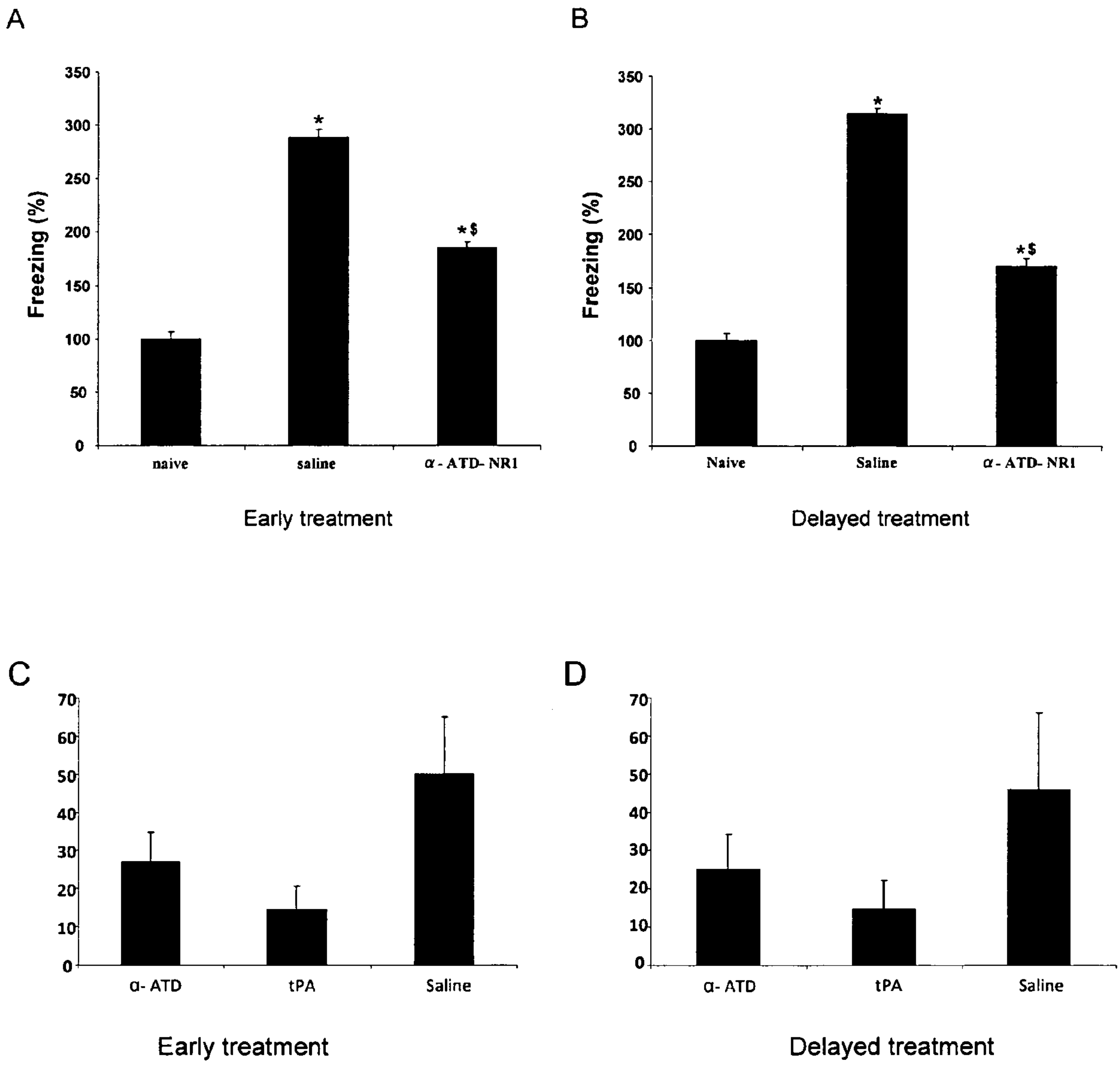
A
B
Freezing (%)
350
300
250
200
150
100
50
0
*
*$
naive
saline
α - ATD- NR1
Early treatment
Naive
Saline
α - ATD- NR1
Delayed treatment
C
D
70
60
50
40
30
20
10
0
α- ATD
tPA
Saline
Early treatment
Delayed treatment

# Figure 20

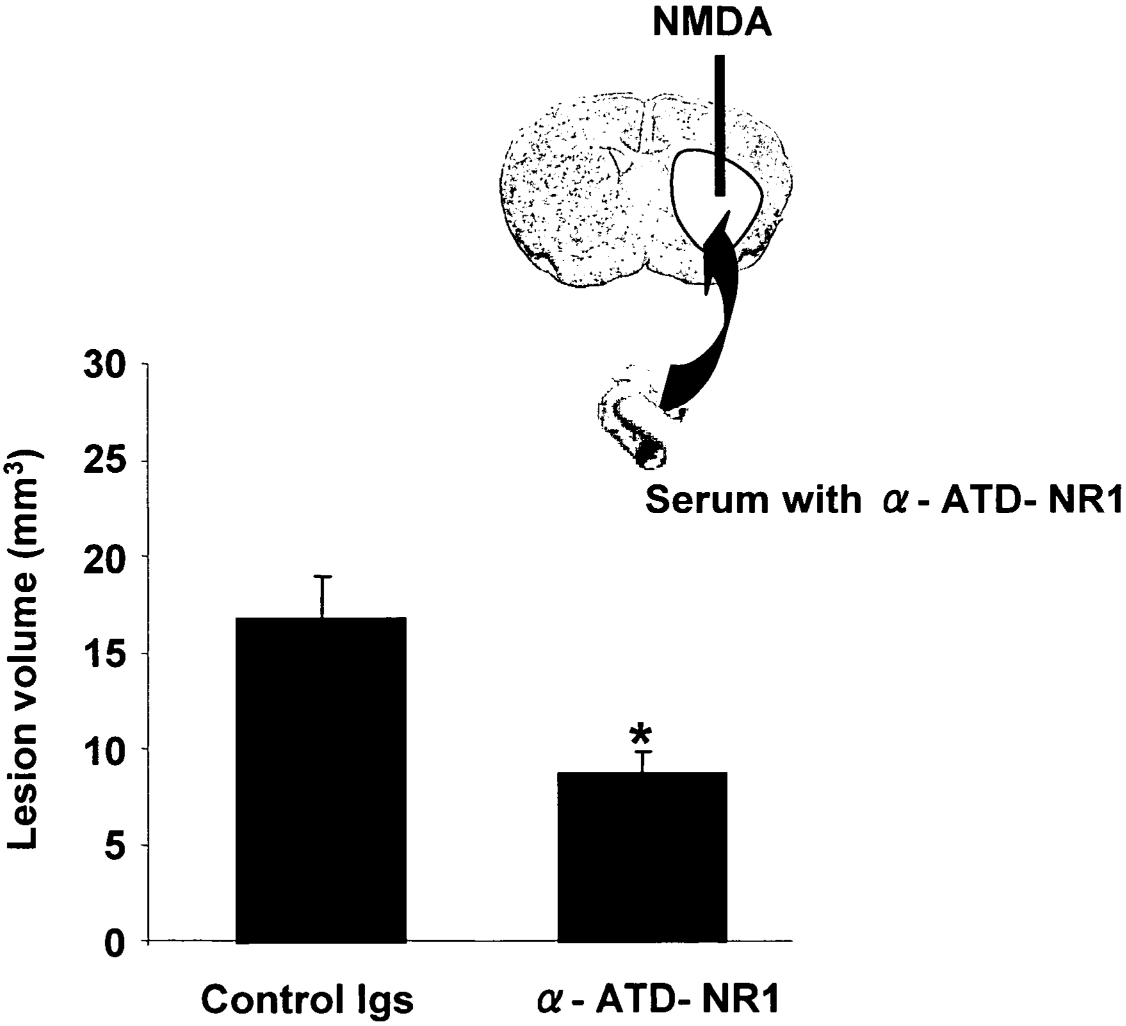
NMDA
Serum with α - ATD- NR1
Lesion volume (mm3)
30
25
20
15
10
5
0
*
Control Igs
α - ATD- NR1

# Figure 21

A

Anti-NTD NR1 antibody

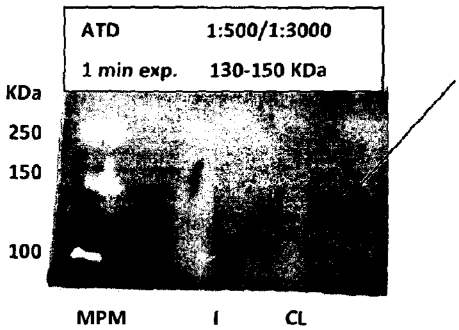


MW marker | Ipsi-lateral | Contra-lateral

B

Commercial anti NR1 antibody

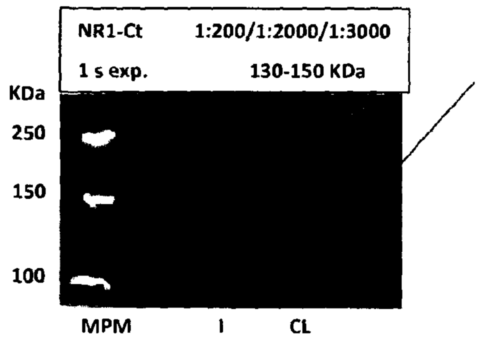


MW marker | Ipsi-lateral | Contra-lateral

Figure 22

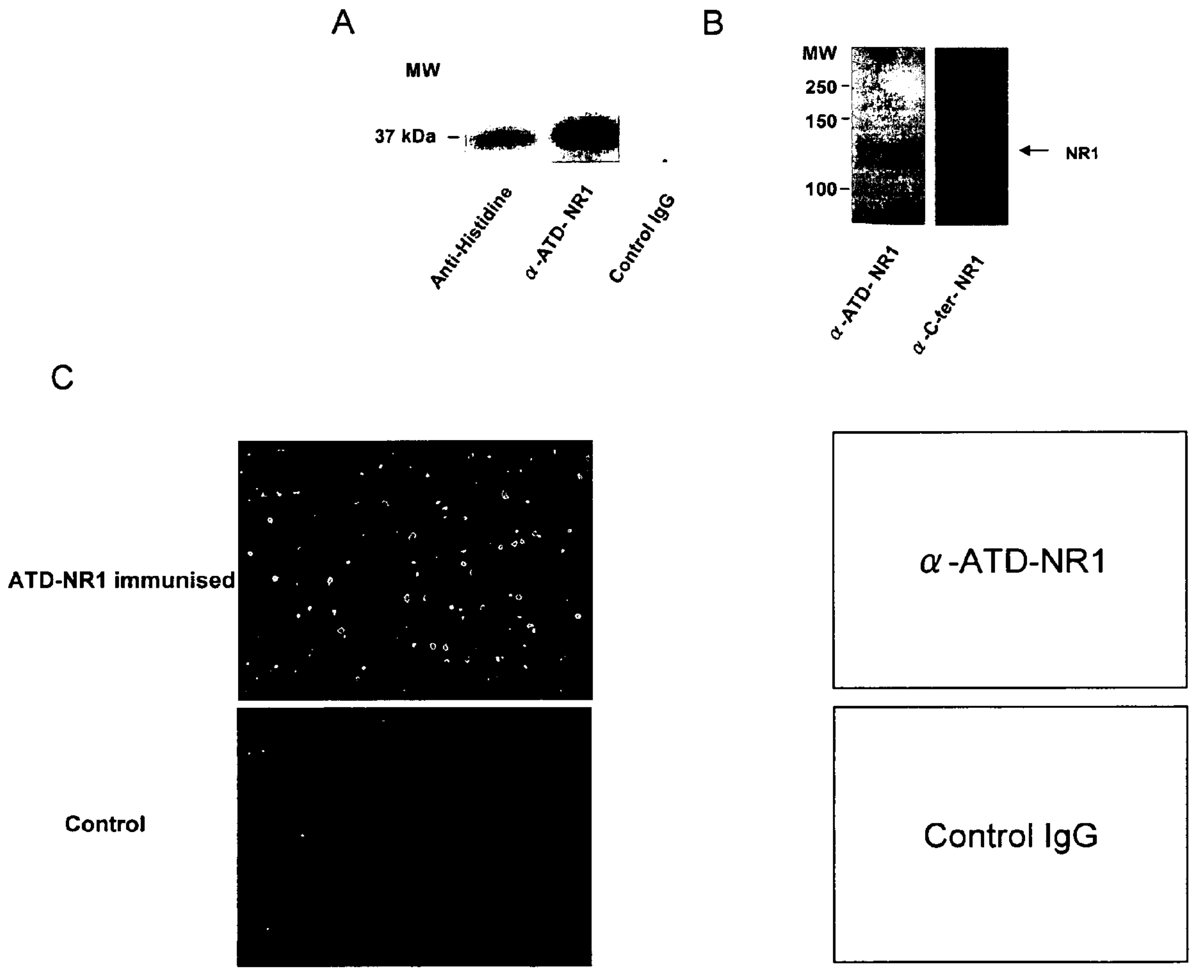
A
MW
37 kDa
Anti-Histidine
α-ATD-NR1
Control IgG
B
MW
250
150
100
NR1
α-ATD-NR1
α-C-ter-NR1
C
ATD-NR1 immunised
Control
α-ATD-NR1
Control IgG

# Figure 23

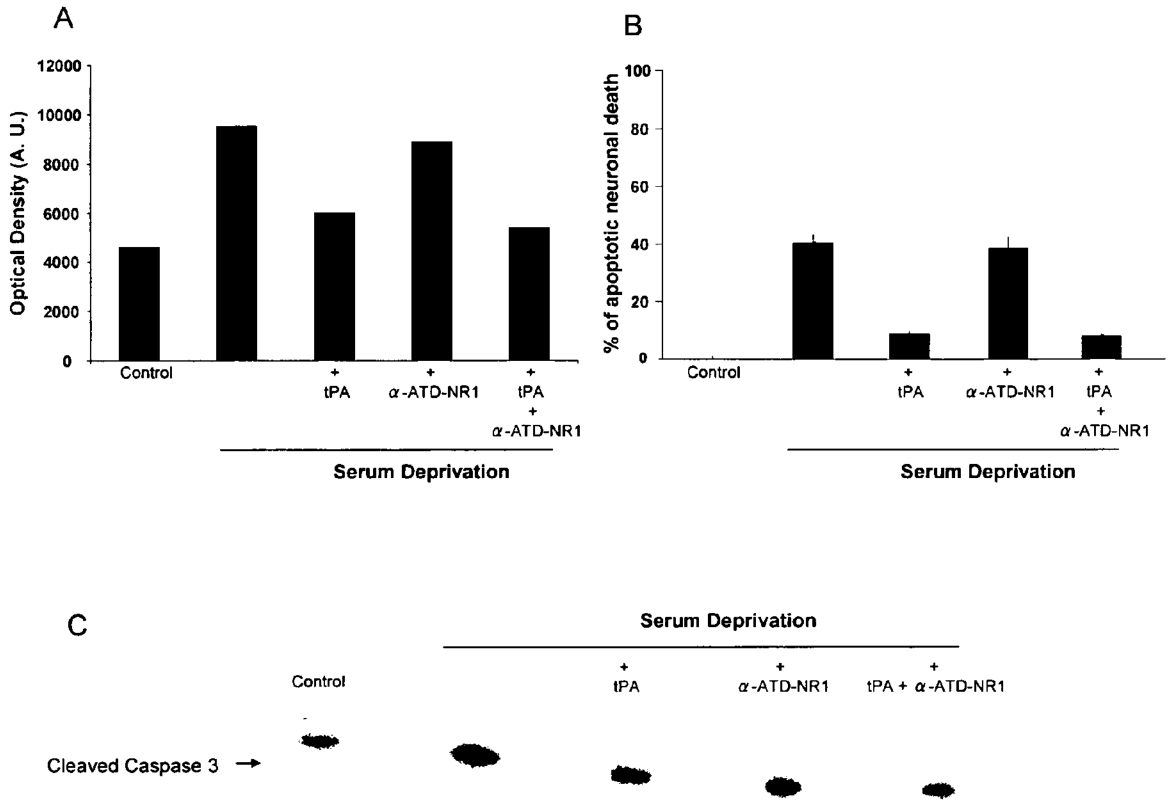
A
Optical Density (A. U.)
12000
10000
8000
6000
4000
2000
0
Control
+ tPA
+ α-ATD-NR1
+ tPA + α-ATD-NR1
Serum Deprivation
B
% of apoptotic neuronal death
100
80
60
40
20
0
Control
+ tPA
+ α-ATD-NR1
+ tPA + α-ATD-NR1
Serum Deprivation
C
Serum Deprivation
Control
+ tPA
+ α-ATD-NR1
+ tPA + α-ATD-NR1
Cleaved Caspase 3

Europäisches Patentamt
European Patent Office
Office européen des brevets

# EUROPEAN SEARCH REPORT

Application Number
EP 09 01 1149

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | ROUSSEL B D ET AL: "PPACK-Desmodus rotundus salivary plasminogen activator (cDSPA[alpha]1) prevents the passage of tissue-type plasminogen activator (rt-PA) across the blood-brain barrier and neurotoxicity" THROMBOSIS AND HAEMOSTASIS, vol. 102, no. 3, September 2009 (2009-09), pages 606-608, XP008118874 SCHATTAUER GMBH DEU ISSN: 0340-6245 prepublished online: July 30,2009 * page 606, column 2, line 12 - page 607, column 1, line 14 * * page 608, column 1, line 1 - line 7 * * page 608, column 2, line 5 - line 9 * ----- | 1-5,7,8, 11-13 | INV. A61K38/49 A61K39/395 C07K14/705 A61P25/00 |
| X | YI J-S ET AL.: "Infarct reduction in rats following intraventricular administration of either tissue plasminogen activator (tPA) or its non-protease mutant S478A-tPA" EXPERIMENTAL NEUROLOGY, vol. 189, no. 2, 1 October 2004 (2004-10-01), pages 354-360, XP004562956 ACADEMIC PRESS, NEW YORK, NY, US ISSN: 0014-4886 abstract * page 357, column 2, paragraph 1 - page 358, column 2, line 2; figures 1-3 * * page 359, column 1, paragraph 5 - paragraph 6 * * page 359, column 2, line 18 - line 24 * ----- -/-- | 1-5,7, 11-13 | TECHNICAL FIELDS SEARCHED (IPC) A61K C07K A61P |

~~The present search report has been drawn up for all claims~~

| Place of search | Date of completion of the search | Examiner |
|---|---|---|
| Berlin | 12 July 2010 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

9

EPO FORM 1503 03.82 (P04C01)

Europäisches Patentamt
European Patent Office
Office européen des brevets

# EUROPEAN SEARCH REPORT

Application Number
EP 09 01 1149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | WO 2009/116035 A (HADASIT MED RES SERVICE [IL]; HIGAZI ABD [IL]; HIJAZI NUHA [IL]) 24 September 2009 (2009-09-24)<br>* page 6, paragraph 3 - page 8, line 3 *<br>----- | 1,5,7, 11-13 | |
| A | YEPES M ET AL: "Tissue-type plasminogen activator in the ischemic brain: more than a thrombolytic"<br>TRENDS IN NEUROSCIENCE,<br>vol. 32, no. 1,<br>1 January 2009 (2009-01-01), pages 48-55, XP025874693<br>ELSEVIER, AMSTERDAM, NL<br>ISSN: 0166-2236 [retrieved on 2008-10-27]<br>* the whole document *<br>----- | 1-5,7,8, 11-13 | |
| A | LOPEZ-ATALAYA J P ET AL: "Toward safer thrombolytic agents in stroke: Molecular requirements for NMDA receptor-mediated neurotoxicity"<br>JOURNAL OF CEREBRAL BLOOD FLOW AND METABOLISM,<br>vol. 28, no. 6, 30 June 2008 (2008-06-30), pages 1212-1221, XP002569820<br>US<br>ISSN: 0271-678X<br>* the whole document *<br>----- | 1-5,7,8, 11-13 | TECHNICAL FIELDS SEARCHED (IPC) |
| A | ROGOVE A D ET AL: "Activation of microglia reveals a non-proteolytic cytokine function for tissue plasminogen activator in the central nervous system"<br>JOURNAL OF CELL SCIENCE 1999 GB,<br>vol. 112, no. 22, 1999, pages 4007-4016, XP002570020<br>ISSN: 0021-9533<br>* page 4008, column 1, paragraph 3 *<br>-----<br>-/-- | 1-5,7,8, 11-13 | |

~~The present search report has been drawn up for all claims~~

| Place of search | Date of completion of the search | Examiner |
|---|---|---|
| Berlin | 12 July 2010 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

9

EPO FORM 1503 03.82 (P04C01)

Europäisches Patentamt
European Patent Office
Office européen des brevets

# EUROPEAN SEARCH REPORT

Application Number
EP 09 01 1149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | BENCHENANE ET AL.: "Anti-NR1N-terminal-domain vaccination unmasks the crucial action of tPA on NMDA-receptor-mediated toxicity and spatial memory" J. CELL SCIENCE, vol. 120, 2007, pages 578-585, XP002591401 invention 6 * page 583, column 2, line 1 - line 11 * * page 583, column 2, paragraph 2 - paragraph 3 * | 1-3, 10-13 | |
| X | -& Anonymous: "Datasheet SC-9058"[Online] 2006, XP002591364 Santa Cruz Biotechnology Retrieved from the Internet: URL:http://datasheets.scbt.com/sc-9058.pdf > [retrieved on 2010-07-09] * the whole document * ----- | 10 | |
| X | WO 00/43039 A1 (DURING MATTHEW JOHN [US]) 27 July 2000 (2000-07-27) invention 6 * page 3, line 26 - page 4, line 13 * * page 38, line 13 - page 41, line 13 * ----- | 1,10-13 | TECHNICAL FIELDS SEARCHED (IPC) |
| X | WO 2007/016357 A1 (UNIV MINNESOTA [US]; ASHE KAREN H [US]; LESNE SYLVAIN E [US]; NEWMAN E) 8 February 2007 (2007-02-08) invention 6 * page 19, line 20 - line 24 * * page 33, line 23 - line 25; claims 19,20 * ----- | 1,10-13 | |

-/--

~~The present search report has been drawn up for all claims~~

9

| Place of search | Date of completion of the search | Examiner |
|---|---|---|
| Berlin | 12 July 2010 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&. : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 01 1149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Anonymous: "Datasheet, Anti-NMDA Receptor, NR1 Subunit"[Online] 24 July 2006 (2006-07-24), XP002591365 PhosphoSolutions, Antibodies that work Retrieved from the Internet: URL:http://www.phosphosolutions.com/store/docs/55_datasheet.pdf> [retrieved on 2010-07-09] invention 6 * the whole document * ----- | 10 | |

TECHNICAL FIELDS SEARCHED (IPC)

~~The present search report has been drawn up for all claims~~

9

| Place of search | Date of completion of the search | Examiner |
|---|---|---|
| Berlin | 12 July 2010 | Schönwasser, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number

EP 09 01 1149

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [X] Only part of the further search fees have been paid within the fixed time limit. The present European search report has been drawn up for those parts of the European patent application which relate to the inventions in respect of which search fees have been paid, namely claims:

  1-3, 10(completely); 11-13(partially)

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

**LACK OF UNITY OF INVENTION**
**SHEET B**

Application Number
EP 09 01 1149

The Search Division considers that the present European patent application does not comply with the requirements of unity of invention and relates to several inventions or groups of inventions, namely:

1. claims: 1-5, 7, 8, 11-13(all partially)

   Use of a protein or peptide for the manufacture of a medicament for the treatment of neurological or neurodegenerative disorders, wherein the protein or peptide is a kringle protein or peptide comprising or consisting of an amino acid sequence according to SEQ ID NO:1, wherein said protein or peptide does not exhibit a serine protease activity; use of a protein or peptide as a neuroprotectant, wherein the protein or peptide comprising or consisting of the amino acid sequence according to SEQ ID NO:1, wherein said protein or peptide does not exhibit a serine protease activity; a pharmaceutical composition selected from said protein or peptide; method of treating a neurological or neurodegenerative disorder, in particular stroke, comprising administering a therapeutically effective amount of said isolated protein or peptide.

   ---

2. claims: 1-5, 7, 8, 11-13(all partially)

   Use of a protein or peptide for the manufacture of a medicament for the treatment of neurological or neurodegenerative disorders, wherein the protein or peptide is a kringle protein or peptide comprising or consisting of an amino acid sequence according to SEQ ID NO:2, wherein said protein or peptide does not exhibit a serine protease activity; use of a protein or peptide as a neuroprotectant, wherein the protein or peptide comprising or consisting of the amino acid sequence according to SEQ ID NO:2, wherein said protein or peptide does not exhibit a serine protease activity; a pharmaceutical composition selected from said protein or peptide; method of treating a neurological or neurodegenerative disorder, in particular stroke, comprising administering a therapeutically effective amount of said isolated protein or peptide.

   ---

3. claims: 1-9(completely); 11-13(partially)

Europäisches Patentamt
European Patent Office
Office européen des brevets

**LACK OF UNITY OF INVENTION SHEET B**

Application Number
EP 09 01 1149

The Search Division considers that the present European patent application does not comply with the requirements of unity of invention and relates to several inventions or groups of inventions, namely:

Use of a protein or peptide for the manufacture of a medicament for the treatment of neurological or neurodegenerative disorders, wherein the protein or peptide is a kringle protein or peptide comprising or consisting of an amino acid sequence according to SEQ ID NO:3, wherein said protein or peptide does not exhibit a serine protease activity; use of a protein or peptide as a neuroprotectant, wherein the protein or peptide comprising or consisting of the amino acid sequence according to SEQ ID NO:3, wherein said protein or peptide does not exhibit a serine protease activity; a pharmaceutical composition selected from said protein or peptide; method of treating a neurological or neurodegenerative disorder, in particular stroke, comprising administering a therapeutically effective amount of said isolated protein or peptide.

---

4. claims: 1-5, 7, 8, 11-13(all partially)

Use of a protein or peptide for the manufacture of a medicament for the treatment of neurological or neurodegenerative disorders, wherein the protein or peptide is a kringle protein or peptide comprising or consisting of an amino acid sequence according to claim 1(a)(iii), wherein said protein or peptide does not exhibit a serine protease activity; use of a protein or peptide as a neuroprotectant, wherein the protein or peptide comprising or consisting of the amino acid sequence according to claim 1(a)(iii), wherein said protein or peptide does not exhibit a serine protease activity; a pharmaceutical composition selected from said protein or peptide; method of treating a neurological or neurodegenerative disorder, in particular stroke, comprising administering a therapeutically effective amount of said isolated protein or peptide.

---

5. claims: 1-5, 7-9, 11-13(all partially)

Europäisches Patentamt
European Patent Office
Office européen des brevets

**LACK OF UNITY OF INVENTION**
**SHEET B**

Application Number
EP 09 01 1149

The Search Division considers that the present European patent application does not comply with the requirements of unity of invention and relates to several inventions or groups of inventions, namely:

Use of a protein or peptide for the manufacture of a medicament for the treatment of neurological or neurodegenerative disorders, wherein the protein or peptide is a kringle protein or peptide comprising or consisting of an amino acid sequence according to claim 1(a)(iv), wherein said protein or peptide does not exhibit a serine protease activity; use of a protein or peptide as a neuroprotectant, wherein the protein or peptide comprising or consisting of the amino acid sequence according to claim 1(a)(iv), wherein said protein or peptide does not exhibit a serine protease activity; a pharmaceutical composition selected from said protein or peptide; method of treating a neurological or neurodegenerative disorder, in particular stroke, comprising administering a therapeutically effective amount of said isolated protein or peptide.

---

6. claims: 1-3, 10(completely); 11-13(partially)

Use of a protein or peptide for the manufacture of a medicament for the treatment of neurological or neurodegenerative disorders, wherein the protein or peptide is selected form the group consisting of an isolated antibody or fragment thereof which binds to the N-terminal domain of the NMDA receptor subunit NR1 (anti-ATD-NRI antibody); an isolated antibody or fragment thereof which binds to the N-terminal domain of the NMDA receptor subunit NR1 (anti-ATD-NRI antibody); use of a protein or peptide as a neuroprotectant, wherein the protein or peptide comprises or consists of an isolated antibody or fragment thereof which binds to the N-terminal domain of the NMDA receptor subunit NR1 (anti-ATD-NRI antibody); a pharmaceutical composition selected from said protein or peptide; method of treating a neurological or neurodegenerative disorder, in particular stroke, comprising administering a therapeutically effective amount of said isolated protein or peptide.

---

## ANNEX TO THE EUROPEAN SEARCH REPORT ON EUROPEAN PATENT APPLICATION NO. EP 09 01 1149

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2010

| Patent document cited in search report | Publication date | Patent family member(s) | Publication date |
|---|---|---|---|
| WO 2009116035 A | 24-09-2009 | NONE | |
| WO 0043039 A1 | 27-07-2000 | AU 775525 B2 | 05-08-2004 |
| | | AU 2861000 A | 07-08-2000 |
| | | CA 2361124 A1 | 27-07-2000 |
| | | EP 1146898 A1 | 24-10-2001 |
| | | JP 2002535289 T | 22-10-2002 |
| WO 2007016357 A1 | 08-02-2007 | CA 2617104 A1 | 08-02-2007 |
| | | EP 1915624 A1 | 30-04-2008 |
| | | JP 2009503520 T | 29-01-2009 |
| | | US 2009258824 A1 | 15-10-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5830849 A
- US 6008019 A
- WO 9324635 A
- EP 352119 A
- EP 382174 A
- US 5202238 A
- US 5204244 A


### Non-patent literature cited in the description


- **Günzler et al.** *Hoppe-Seyler's Z. Physiol. Chem.,* 1982, vol. 363, 1155
- **Pennica.** *Nature,* 1983, vol. 301, 214
- **Walz et al.** *Proc. Nat'l. Acad. Sci. USA,* 1977, vol. 74, 1969
- **Sottrup-Jensen et al.** Progress in Chemical Fibrinolysis and Thrombolysis. 1978, vol. 3, 191
- **Morrison, S.L. et al.** *Proc. Natl. Acad. Sci. USA,* 1984, vol. 81, 6851-6855
- **Riechmann, L. et al.** *Nature,* 1988, vol. 332, 323-327
- **Neuberger, M.S. et al.** *Nature,* 1985, vol. 314, 268-270
- **van Dijk, M.A. ; van de Winkel, J. G.** *Curr. Opin. Pharmacol.,* 2001, vol. 5, 368-374
- **Kellermann, S. A. et al.** *Curr Opin Biotechnol.,* 2002, vol. 13, 593-597
- **Benchenane et al.** *Circulation,* 2005, vol. 111 (17), 2241-2249
- **Benchenane et al.** *Stroke,* 2005, vol. 36 (5), 1065-1070
- **Benchenane et al.** *J. Cell Science,* 2007, vol. 120, 578-585
- **Fernandez-Monreal et al.** *J. Biol. Chem.,* 2004, vol. 279 (49), 50850-50856
- **Franklin ; Paxinos.** The mouse brain in stereotactic coordinates. Academis Press, 1997
- **Günzler et al.** *Hoppe-Seyler's, Z. Physiol. Chem.,* 1982, vol. 363 (10), 1155-1165
- **Kellermann et al.** *Curr. Opin. Biotechnol.,* 2002, vol. 13, 593-597
- **Liot et al.** *J. Cereb. Blood Flow Metab.,* 2004, vol. 24 (10), 1153-1159
- **Liot et al.** *J. Neurochem,* 2006, vol. 98 (5), 1458-1464
- **Morrison et al.** *Proc. Natl. Acad. Sci. USA,* 1984, vol. 81, 6851-6855
- **Neuberger et al.** *Nature,* 1985, vol. 314, 268-270
- **Orset et al.** *Stroke,* 2007, vol. 38 (10), 2771-2778
- **Pennica et al.** *Nature,* 1983, vol. 301 (5897), 214-221
- **Riechmann et al.** *Nature,* 1988, vol. 332, 323-327
- **Sottrup-Jensen et al.** Progress in Chemical Fibrinolysis and Thrombolysis. 1978, vol. 3, 191ff
- **van Dijk ; van de Winkel.** *Curr. Opin. Pharmacol.,* 2001, vol. 5, 368-374
- **Walz et al.** *Proc. Nat'l. Acad. Sci. USA,* 1977, vol. 74, 1969-1972
- **Yepes et al.** *Trends Neurosci,* 2009, vol. 32 (1), 48-55

SEQUENCE LISTING

<110> PAION Deutschland GmbH
Petersen, Karl-Uwe
Vivien, Denis

<120> Treatment of neurological or neurodegenerative disorders

<130> 47 924 K

<160> 7

<170> PatentIn version 3.3

<210> 1
<211> 82
<212> PRT
<213> Homo sapiens

<220>
<221> Kringle DOMAIN
<222> (1)..(82)
<223> t-PA_kringle 1

<400> 1

Cys Tyr Glu Asp Gln Gly Ile Ser Tyr Arg Gly Thr Trp Ser Thr Ala
1 5 10 15

Glu Ser Gly Ala Glu Cys Thr Asn Trp Asn Ser Ser Ala Leu Ala Gln
20 25 30

Lys Pro Tyr Ser Gly Arg Arg Pro Asp Ala Ile Arg Leu Gly Leu Gly
35 40 45

Asn His Asn Tyr Cys Arg Asn Pro Asp Arg Asp Ser Lys Pro Trp Cys
50 55 60

Tyr Val Phe Lys Ala Gly Lys Tyr Ser Ser Glu Phe Cys Ser Thr Pro
65 70 75 80

Ala Cys

<210> 2
<211> 82
<212> PRT
<213> homo sapiens

<220>
<221> Kringle DOMAIN
<222> (1)..(82)
<223> t-PA_kringle 2

<400> 2

Cys Tyr Phe Gly Asn Gly Ser Ala Tyr Arg Gly Thr His Ser Leu Thr
1 5 10 15

```
Glu Ser Gly Ala Ser Cys Leu Pro Trp Asn Ser Met Ile Leu Ile Gly
            20                  25                  30

Lys Val Tyr Thr Ala Gln Asn Pro Ser Ala Gln Ala Leu Gly Leu Gly
        35                  40                  45

Lys His Asn Tyr Cys Arg Asn Pro Asp Gly Asp Ala Lys Pro Trp Cys
    50                  55                  60

His Val Leu Lys Asn Arg Arg Leu Thr Trp Glu Tyr Cys Asp Val Pro
65                  70                  75                  80

Ser Cys


<210>  3
<211>  82
<212>  PRT
<213>  Desmodus rotundus


<220>
<221>  Kringle DOMAIN
<222>  (1)..(82)
<223>  kringle domain of DSPA alpha 1

<400>  3

Cys Tyr Glu Gly Gln Gly Val Thr Tyr Arg Gly Thr Trp Ser Thr Ala
1               5                   10                  15

Glu Ser Arg Val Glu Cys Ile Asn Trp Asn Ser Ser Leu Leu Thr Arg
            20                  25                  30

Arg Thr Tyr Asn Gly Arg Met Pro Asp Ala Phe Asn Leu Gly Leu Gly
        35                  40                  45

Asn His Asn Tyr Cys Arg Asn Pro Asn Gly Ala Pro Lys Pro Trp Cys
    50                  55                  60

Tyr Val Ile Lys Ala Gly Lys Phe Thr Ser Glu Ser Cys Ser Val Pro
65                  70                  75                  80

Val Cys


<210>  4
<211>  353
<212>  PRT
<213>  Rattus norvegicus


<220>
<221>  N-terminal sequence without signal peptide
<222>  (1)..(353)
<223>  as 19 to 371 of rat NR1-1a (NP_058706.1)
```

<400> 4

Arg Ala Ala Cys Asp Pro Lys Ile Val Asn Ile Gly Ala Val Leu Ser
1               5                   10                  15

Thr Arg Lys His Glu Gln Met Phe Arg Glu Ala Val Asn Gln Ala Asn
            20                  25                  30

Lys Arg His Gly Ser Trp Lys Ile Gln Leu Asn Ala Thr Ser Val Thr
        35                  40                  45

His Lys Pro Asn Ala Ile Gln Met Ala Leu Ser Val Cys Glu Asp Leu
    50                  55                  60

Ile Ser Ser Gln Val Tyr Ala Ile Leu Val Ser His Pro Pro Thr Pro
65                  70                  75                  80

Asn Asp His Phe Thr Pro Thr Pro Val Ser Tyr Thr Ala Gly Phe Tyr
                85                  90                  95

Arg Ile Pro Val Leu Gly Leu Thr Thr Arg Met Ser Ile Tyr Ser Asp
            100                 105                 110

Lys Ser Ile His Leu Ser Phe Leu Arg Thr Val Pro Pro Tyr Ser His
        115                 120                 125

Gln Ser Ser Val Trp Phe Glu Met Met Arg Val Tyr Asn Trp Asn His
    130                 135                 140

Ile Ile Leu Leu Val Ser Asp Asp His Glu Gly Arg Ala Ala Gln Lys
145                 150                 155                 160

Arg Leu Glu Thr Leu Leu Glu Glu Arg Glu Ser Lys Ala Glu Lys Val
                165                 170                 175

Leu Gln Phe Asp Pro Gly Thr Lys Asn Val Thr Ala Leu Leu Met Glu
            180                 185                 190

Ala Arg Glu Leu Glu Ala Arg Val Ile Ile Leu Ser Ala Ser Glu Asp
        195                 200                 205

Asp Ala Ala Thr Val Tyr Arg Ala Ala Ala Met Leu Asn Met Thr Gly
    210                 215                 220

Ser Gly Tyr Val Trp Leu Val Gly Glu Arg Glu Ile Ser Gly Asn Ala
225                 230                 235                 240

Leu Arg Tyr Ala Pro Asp Gly Ile Ile Gly Leu Gln Leu Ile Asn Gly
                245                 250                 255

Lys Asn Glu Ser Ala His Ile Ser Asp Ala Val Gly Val Val Ala Gln
            260                 265                 270

```
Ala Val His Glu Leu Leu Glu Lys Glu Asn Ile Thr Asp Pro Pro Arg
        275                 280                 285

Gly Cys Val Gly Asn Thr Asn Ile Trp Lys Thr Gly Pro Leu Phe Lys
    290                 295                 300

Arg Val Leu Met Ser Ser Lys Tyr Ala Asp Gly Val Thr Gly Arg Val
305                 310                 315                 320

Glu Phe Asn Glu Asp Gly Asp Arg Lys Phe Ala Asn Tyr Ser Ile Met
                325                 330                 335

Asn Leu Gln Asn Arg Lys Leu Val Gln Val Gly Ile Tyr Asn Gly Thr
            340                 345                 350

His
```

<210> 5
<211> 353
<212> PRT
<213> homo sapiens

<220>
<221> N-terminal sequence without sequence peptide
<222> (1)..(353)
<223> as 19 to 371 of human NR1-1 (NP_000823.4)

<400> 5

```
Arg Ala Ala Cys Asp Pro Lys Ile Val Asn Ile Gly Ala Val Leu Ser
1               5                   10                  15

Thr Arg Lys His Glu Gln Met Phe Arg Glu Ala Val Asn Gln Ala Asn
            20                  25                  30

Lys Arg His Gly Ser Trp Lys Ile Gln Leu Asn Ala Thr Ser Val Thr
        35                  40                  45

His Lys Pro Asn Ala Ile Gln Met Ala Leu Ser Val Cys Glu Asp Leu
    50                  55                  60

Ile Ser Ser Gln Val Tyr Ala Ile Leu Val Ser His Pro Pro Thr Pro
65                  70                  75                  80

Asn Asp His Phe Thr Pro Thr Pro Val Ser Tyr Thr Ala Gly Phe Tyr
                85                  90                  95

Arg Ile Pro Val Leu Gly Leu Thr Thr Arg Met Ser Ile Tyr Ser Asp
            100                 105                 110

Lys Ser Ile His Leu Ser Phe Leu Arg Thr Val Pro Pro Tyr Ser His
```

115 120 125

Gln Ser Ser Val Trp Phe Glu Met Met Arg Val Tyr Ser Trp Asn His
130 135 140

Ile Ile Leu Leu Val Ser Asp Asp His Glu Gly Arg Ala Ala Gln Lys
145 150 155 160

Arg Leu Glu Thr Leu Leu Glu Glu Arg Glu Ser Lys Ala Glu Lys Val
165 170 175

Leu Gln Phe Asp Pro Gly Thr Lys Asn Val Thr Ala Leu Leu Met Glu
180 185 190

Ala Arg Glu Leu Glu Ala Arg Val Ile Ile Leu Ser Ala Ser Glu Asp
195 200 205

Asp Ala Ala Thr Val Tyr Arg Ala Ala Ala Met Leu Asn Met Thr Gly
210 215 220

Ser Gly Tyr Val Trp Leu Val Gly Glu Arg Glu Ile Ser Gly Asn Ala
225 230 235 240

Leu Arg Tyr Ala Pro Asp Gly Ile Ile Gly Leu Gln Leu Ile Asn Gly
245 250 255

Lys Asn Glu Ser Ala His Ile Ser Asp Ala Val Gly Val Val Ala Gln
260 265 270

Ala Val His Glu Leu Leu Glu Lys Glu Asn Ile Thr Asp Pro Pro Arg
275 280 285

Gly Cys Val Gly Asn Thr Asn Ile Trp Lys Thr Gly Pro Leu Phe Lys
290 295 300

Arg Val Leu Met Ser Ser Lys Tyr Ala Asp Gly Val Thr Gly Arg Val
305 310 315 320

Glu Phe Asn Glu Asp Gly Asp Arg Lys Phe Ala Asn Tyr Ser Ile Met
325 330 335

Asn Leu Gln Asn Arg Lys Leu Val Gln Val Gly Ile Tyr Asn Gly Thr
340 345 350

His

<210> 6
<211> 1059
<212> DNA
<213> rattus norvegicus

<220>
<221> N-terminal_domain_without_signal_sequence
<222> (1)..(1059)
<223> nt 320 - 1378 of NM_017010.1| Rattus norvegicus glutamate receptor, ionotropic, N-methyl D-aspartate 1 (Grin1), mRNA

<400> 6
cgcgccgcct gcgaccccaa gatcgtcaac atcggcgcgg tgctgagcac gcgcaagcat 60

gaacagatgt tccgcgaggc agtaaaccag gccaataagc gacacggctc ttggaagata 120

cagctcaacg ccacttctgt cacccacaag cccaacgcca tacagatggc cctgtcagtg 180

tgtgaggacc tcatctctag ccaggtctac gctatcctag ttagccaccc gcctactccc 240

aacgaccact tcactcccac ccctgtctcc tacacagctg gcttctacag aatccctgtc 300

ctgggactga ctacccgaat gtccatctac tctgacaaga gtatccacct gagtttcctt 360

cgcacggtgc cgccctactc ccaccagtcc agcgtctggt ttgagatgat gcgagtctac 420

aactggaacc acatcatcct gctggtcagc gacgaccacg agggacgggc agcgcagaag 480

cgcttggaga cgttgctgga ggaacgggag tccaaggcag agaaggtgct gcagtttgac 540

ccaggaacca agaatgtgac ggctctgctg atggaggccc gggaactgga ggcccgggtc 600

atcatccttt ctgcaagcga ggacgacgct gccacagtgt accgcgcagc cgcaatgctg 660

aacatgacgg gctctgggta cgtgtggctg gtcggggaac gcgagatctc tgggaacgcc 720

ctgcgctacg ctcctgatgg catcatcgga cttcagctca tcaatggcaa gaatgagtca 780

gcccacatca gtgacgccgt gggcgtggtg gcacaggcag ttcacgaact cctagagaag 840

gagaatatca ctgacccacc gcggggttgc gtgggcaaca ccaacatctg gaagacagga 900

ccattgttca agagggtgct gatgtcttct aagtatgcgg acggagtgac tggccgtgtg 960

gaattcaatg aggatgggga ccggaagttt gccaactata gtatcatgaa cctgcagaac 1020

cgcaagctgg tgcaagtggg catctacaat ggtacccat 1059

<210> 7
<211> 1059
<212> DNA
<213> homo sapiens

<220>
<221> N-terminal_sequence_without_signal_peptide
<222> (1)..(1059)
<223> nt 388 - 1442 of NM_000832.5, Homo sapiens glutamate receptor, ionotropic, N-methyl D-aspartate 1 (GRIN1), transcript variant NR1-1, mRNA

<400> 7
cgtgccgcgt gcgaccccaa gatcgtcaac attggcgcgg tgctgagcac gcggaagcac 60

gagcagatgt tccgcgaggc cgtgaaccag gccaacaagc ggcacggctc ctggaagatt 120

cagctcaatg ccacctccgt cacgcacaag cccaacgcca tccagatggc tctgtcggtg 180

tgcgaggacc tcatctccag ccaggtctac gccatcctag ttagccatcc acctaccccc 240

aacgaccact tcactcccac ccctgtctcc tacacagccg gcttctaccg catacccgtg 300

```
ctggggctga ccacccgcat gtccatctac tcggacaaga gcatccacct gagcttcctg    360
cgcaccgtgc cgccctactc ccaccagtcc agcgtgtggt ttgagatgat gcgtgtctac    420
agctggaacc acatcatcct gctggtcagc gacgaccacg agggccgggc ggctcagaaa    480
cgcctggaga cgctgctgga ggagcgtgag tccaaggcag agaaggtgct gcagtttgac    540
ccagggacca agaacgtgac ggccctgctg atggaggcga aagagctgga ggcccgggtc    600
atcatccttt ctgccagcga ggacgatgct gccactgtat accgcgcagc cgcgatgctg    660
aacatgacgg gctccgggta cgtgtggctg gtcggcgagc gcgagatctc ggggaacgcc    720
ctgcgctacg ccccagacgg catcctcggg ctgcagctca tcaacggcaa gaacgagtcg    780
gcccacatca gcgacgccgt gggcgtggtg gcccaggccg tgcacgagct cctcgagaag    840
gagaacatca ccgacccgcc gcggggctgc gtgggcaaca ccaacatctg gaagaccggg    900
ccgctcttca agagagtgct gatgtcttcc aagtatgcgg atggggtgac tggtcgcgtg    960
gagttcaatg aggatgggga ccggaagttc gccaactaca gcatcatgaa cctgcagaac   1020
cgcaagctgg tgcaagtggg catctacaat ggcacccac                          1059
```